# EUROPÄISCHE PATENTSCHRIFT

(11) **EP 4 162 980 B1**
(45) Veröffentlichungstag und Bekanntmachung des Hinweises auf die Patenterteilung: **04.11.2026**
(21) Anmeldenummer: 22193844.2
(22) Anmeldetag: 05.09.2022
(51) Int. Cl.: A61N 5/06

(54) **SYSTEM ZUR DURCHFÜHRUNG EINER TRANSKUTANEN FOTODYNAMISCHEN THERAPIE (PDT) IN EINEM ORGAN ODER ORGANSEGMENT EINES ORGANISCHEN KÖRPERS**
SYSTEM FOR PERFORMING TRANSCUTANEOUS PHOTODYNAMIC THERAPY (PDT) IN AN ORGAN OR ORGAN SEGMENT OF AN ORGANIC BODY
SYSTÈME DE MISE EN UVRE D'UNE THÉRAPIE PHOTODYNAMIQUE TRANSCUTANEE (PDT) DANS UN ORGANE OU UN SEGMENT D'ORGANE D'UN CORPS ORGANIQUE

(30) Priorität: 07.10.2021 DE 102021211328
(43) Veröffentlichungstag der Anmeldung: 12.04.2023
(73) Patentinhaber: Richard Wolf GmbH, 75438 Knittlingen (DE)
(72) Erfinder: Weber, Bernd Claus, 76228 Karlsruhe (DE); Sieber, Stephan, 75438 Knittlingen-Freudenstein (DE); Lambertz, Matthias, 75015 Bretten (DE)
(74) Vertreter: Patentanwälte Hemmer Lindfeld Frese Partnerschaft mbB

(56) Entgegenhaltungen:
- EP-A1- 3 777 974
- US-A1- 2010 329 524
- FAMULARI GABRIEL ET AL: "A novel 169 Yb-based dynamic-shield intensity modulated brachytherapy delivery system for prostate cancer", vol. 47, no. 3, 30 December 2019 (2019-12-30), US, pages 859 - 868, XP093024936, ISSN: 0094-2405, Retrieved from the Internet <URL:https://onlinelibrary.wiley.com/doi/full-xml/10.1002/mp.13959> [retrieved on 20230220], DOI: 10.1002/mp.13959
- FAMULARI GABRIEL ET AL: "A novel 169 Yb-based dynamic-shield intensity modulated brachytherapy delivery system for prostate cancer - fig.S3", MEDICAL PHYSISCS, vol. 47, 30 December 2019 (2019-12-30), pages 859 - 868, XP093025028

## Beschreibung

Die vorliegende Offenbarung betrifft ein System zur Durchführung einer transkutanen photodynamischen Therapie (PDT) in einem Organ oder Organsegment eines organischen Körpers.

Die PDT ist eine bekannte medizinische Therapie von pathologischem Gewebe eines Patienten. Dem Patienten wird dazu ein Photosensibilisator bzw. Markerstoff verabreicht, der sich selektiv an dem zu therapierenden pathologischen Gewebe anreichert. Bei der PDT wird dann Licht mittels eines Lichtapplikators oder mehrerer Lichtapplikatoren unmittelbar auf oder sogar in das pathologische Gewebe appliziert, um lichtinduziert die Bildung von Sauerstoffradikalen mittels des örtlich begrenzt angereicherten Photosensibilisators bzw. Markerstoffs zu fördern und dadurch das pathologische Gewebe, wie etwa einen Tumor, zu zerstören. Typischerweise wird dazu Laserlicht in einen Lichtleiter eingekoppelt und zum Gewebe geleitet. Wenn das pathologische Gewebe flächig auf einer äußeren Oberfläche des Körpers, z.B. der Haut, oder einer inneren Oberfläche, z.B. der Speiseröhreninnenfläche oder Darmwandungen, angeordnet ist, dann kann das Therapielicht relativ einfach ausgekoppelt und auf die pathologische Gewebefläche gestrahlt werden. Erstreckt sich das pathologische Gewebe allerdings über ein Volumen in einem inneren Organ oder Organsegment, so kann wegen der begrenzten Eindringtiefe des Lichts in das Gewebe nicht immer ein Tumor von "außen" effektiv bestrahlt werden. In diesem Fall ist die PDT besonders effektiv, wenn das Licht vom Inneren des pathologischen Gewebevolumens bestrahlt wird. Dazu muss der Lichtapplikator oder die Lichtapplikatoren in das pathologische Gewebe eingestochen werden. Dies wird auch als interstitielle (durch innere Oberflächen) und/oder transkutane (durch die Haut) PDT bezeichnet. Die US 6,048,359 beschreibt beispielsweise ein System zur Durchführung einer transkutanen PDT, um ein inneres Volumen im Körper eines Patienten zu bestrahlen. Dabei wird eine Mehrzahl von nadelartigen Lichtapplikatoren parallel durch die Haut in den Körper gestochen, wobei jeder Lichtapplikator über seine Länge verteilt laterale Lichtauskopplungen aufweist, um so ein Volumen im Körper zu bestrahlen.

Ein System zur photodynamischen Therapie wird auch in EP 3 777 974 A1 beschrieben.

Nachteilig an der bekannten Lösung ist, dass die Lichtapplikatoren erstens relativ dick ausgestaltet werden müssen und zweitens relativ komplex und teuer sind, sodass sie erstens, insbesondere in der Summe, relativ patientenbelastend sind und zweitens nicht als Einwegartikel zum einmaligen Gebrauch realisierbar sind.

Daraus ergibt sich die Aufgabe, ein kostengünstigeres System zur Durchführung einer transkutanen PDT mit dünneren und damit patientenschonenderen Lichtapplikatoren bereitzustellen, wobei das System eine lückenlose Bestrahlung des Organs oder Organsegments erlauben soll.

Gemäß einem ersten Aspekt der vorliegenden Offenbarung wird zur Lösung dieses Problems ein System zur Durchführung einer transkutanen PDT in einem Organ oder Organsegment eines organischen Körpers bereitgestellt, bei dem das System eine Mehrzahl von individuell manuell platzierbaren Lichtapplikatoren, eine Versorgungseinheit zum Versorgen der Lichtapplikatoren mit Licht und/oder Strom, und eine in einer definierten Position zum organischen Körper platzierbare Platzierungsschablone zum definierten Ausrichten, Positionieren und Fixieren der einzelnen Lichtapplikatoren in bzw. an der Platzierungsschablone und damit schließlich zur definierten Platzierung der lichtemittierenden Applikatorspitzen im Organ oder Organsegment aufweist. Dafür weisen die einzeln platzierbaren Lichtapplikatoren jeweils einen nadelartigen Einführabschnitt zum transkutanen Einstechen entlang einer Einstichachse in das Organ oder Organsegment, am distalen Ende des Einführabschnitts eine lichtemittierende Applikatorspitze und mindestens einen definierten Fixierpunkt in einem proximalen Abstand d von der lichtemittierenden Applikatorspitze auf. Zudem definiert die Platzierungsschablone eine Mehrzahl an Fixierpunktaufnahmen, mittels welcher die einzelnen Lichtapplikatoren mit ihrem mindestens einen definierten Fixierpunkt fixierbar sind, wobei die Fixierpunktaufnahmen entsprechend einer dreidimensionalen Fixierpunktgitterstruktur in oder an der Platzierungsschablone angeordnet sind, wobei die Fixierpunktgitterstruktur einer virtuellen organspezifischen Sollpunktgitterstruktur für die lichtemittierenden Applikatorspitzen im Organ oder Organsegment entspricht und um den Abstand d entlang der Einstichachse parallelverschoben zur Sollpunktgitterstruktur angeordnet ist. Die Platzierungsschablone kann dabei eine Mehrzahl an Führungen definieren, wobei durch jede der Führungen ein Einführabschnitt jeweils eines der Lichtapplikatoren geführt transkutan in das Organ oder Organsegment entlang der Einstichachse einstechbar ist. Die Fixierpunktaufnahmen können an oder in den Führungen ausgebildet sein.

Die prinzipielle Vorgehensweise und die dabei zu Grunde liegende Idee mit dem vorausgehend beschriebenen Aufbau bestehen darin, mit der Realisierung und Positionierung der Platzierungsschablone außerhalb des Körpers in einem ersten Schritt die virtuelle dreidimensionale Sollpunktgitterstruktur mittels einer Parallelverschiebung bzw. Translation um die Strecke d auf eine dreidimensionale Fixierpunktgitterstruktur abzubilden. Die virtuelle dreidimensionale Sollpunktgitterstruktur repräsentiert einerseits die definierte Soll-Anordnung der lichtemittierenden Applikatorspitzen im Organ oder Organsegment im Hinblick auf eine lückenlose Bestrahlung des Organs oder Organsegments. Die Bedienperson soll also die Sollpunkte der Sollpunktgitterstruktur nach und nach mit den einzelnen lichtemittierenden Applikatorspitzen besetzen. Allerdings sind die Sollpunkte der Sollpunktgitterstruktur im Patientenkörper für die Bedienperson nicht sichtbar und deshalb zunächst nicht gezielt und direkt mit den lichtemittierenden Applikatorspitzen ansteuerbar. Die Fixierpunktgitterstruktur, welche von den Fixierpunktaufnahmen in bzw. an der Platzierungsschablone außerhalb des Patientenkörpers repräsentiert wird, ist im Gegensatz zu den innerhalb des Körpers liegenden virtuellen Sollpunkten der virtuellen Sollpunktgitterstruktur für die Bedienperson sichtbar und mit dem mindestens einen Fixierpunkt eines jeweiligen Lichtapplikators gezielt und direkt ansteuerbar. Nach dem ersten Schritt der Parallelverschiebung bzw. Translation werden also in einem zweiten Schritt die einzelnen lichtemittierenden Applikatorspitzen mittels einer in Bezug auf die oben beschriebene Parallelverschiebung inversen Parallelverschiebung an den virtuellen und für die Bedienperson unsichtbaren Sollpunkten der virtuellen Sollpunktgitterstruktur im Organ oder Organsegment in definierter, eindeutiger und damit sicherer Weise platziert.

Im ersten Schritt wird also die virtuelle, nicht sichtbare und deshalb nicht direkt ansteuerbare, organspezifische Sollpunktgitterstruktur innerhalb des Körpers mittels einer Parallelverschiebung bzw. Translation in bzw. auf die Fixierpunktgitterstruktur, repräsentiert durch die real existierenden, sichtbaren und deshalb direkt zugänglichen Fixierpunktaufnahmen der Platzierungsschablone, außerhalb des Körpers transformiert bzw. abgebildet.

Nach diesem ersten Schritt folgt als zweiter Schritt die Durchführung der eigentlichen Therapievorbereitung durch die Bedienperson, welche in der ordnungsgemäßen Verteilung und Platzierung der einzelnen Lichtapplikatoren bzw. der lichtemittierenden Applikatorspitzen im Patienten im Hinblick auf eine lückenlose Bestrahlung des Organs oder Organsegments besteht. Bei diesem zweiten Schritt werden die lichtemittierenden Applikatorspitzen - ausgehend von den sichtbaren und direkt ansteuerbaren Fixierpunktaufnahmen der Platzierungsschablone außerhalb des Körpers - mittels der Parallelverschiebung, welche zur Parallelverschiebung des ersten Schrittes invers ist, in definierter, eindeutiger und damit sicherer Weise zu den unsichtbaren Sollpunkten innerhalb des Körpers transformiert bzw. bewegt. Die Definiertheit und Eindeutigkeit bei der Platzierung der einzelnen Applikatorspitzen in den Sollpunkten wird in erfindungsgemäßer Weise dadurch erreicht, dass erstens die Platzierungsschablone mit parallel zueinander ausgerichteten Führungen für eine definierte, eindeutige, parallele Ausrichtung der einzelnen Lichtapplikatoren versehen ist und dass zweitens einerseits die Platzierungsschablone mit Fixierpunktaufnahmen und andererseits die Lichtapplikatoren mit mindestens einem Fixierpunkt versehen sind, mit welchem die Lichtapplikatoren in bzw. an den Fixierpunktaufnahmen der Platzierungsschablone an einer definierten und eindeutigen Stelle fixiert werden können, wodurch eine definierte und eindeutige Eindringtiefe der Lichtapplikatoren in das Organ bzw. Organsegment erreicht wird.

Die einzelnen Lichtapplikatoren können dabei besonders dünn und starr als Metallschaft und/oder -rohr ausgestaltet werden, da nur die Applikatorspitze lichtemittierend ausgestaltet werden muss. An der Applikatorspitze kann beispielsweise eine lichtemittierende Komponente in Form einer Lichtleiterauskopplung oder einer LED angeordnet sein. Die Applikatorspitze kann als lichttransparenter Streukörper distalseitig von der lichtemittierenden Komponente angeordnet sein. Das System zur Durchführung einer transkutanen PDT ist damit wesentlich gewebeschonender und weniger invasiv als aus dem Stand der Technik bekannte Systeme. Durch die dreidimensionale Fixierpunktgitterstruktur, welche durch die oben erläuterte Parallelverschiebung um die Länge d entlang der Einstichachse aus der Sollpunktgitterstruktur hervorgeht, mit einerseits Fixierpunktaufnahmen, die entsprechend dieser dreidimensionalen Fixierpunktgitterstruktur angeordnet sind, und mit andererseits Führungen, die beide zusammen (Fixierpunktaufnahme und Führung) in der Kombination mit dem mindestens einen Lichtapplikator-Fixierpunkt eine Bewegung und finale Platzierung der Lichtapplikatoren und der lichtemittierenden Applikatorspitzen im Körper und im Organ ausschließlich gemäß einer der oben erläuterten Parallelverschiebung inversen Parallelverschiebung zulassen, und in diesem Sinne die diesbezüglichen Freiheitsgrade der Bedienperson auf ein Minimum reduzieren, wird sichergestellt, dass die lichtemittierenden Applikatorspitzen entsprechend der Sollpunktgitterstruktur im Organ oder Organsegment so angeordnet sind, dass das Organ oder Organsegment lückenlos über das gesamte Volumen der Sollpunktgitterstruktur bestrahlt wird. Jeder Gitterpunkt der Sollpunktgitterstruktur wird dabei vorzugsweise von einer Applikatorspitze besetzt. Dies muss allerdings nicht unbedingt zeitgleich erfolgen. Eine besonders schnelle Durchführung der transkutanen PDT wird dadurch erreicht, dass sämtliche Gitterpunkte der Sollpunktgitterstruktur nach und nach jeweils mit einer Applikatorspitze besetzt werden und dann zeitgleich das gesamte Organ oder Organsegment bestrahlt wird. Alternativ dazu kann die transkutane PDT sukzessive für unterschiedliche Gitterpunkte oder Gitterpunktgruppen der Sollpunktgitterstruktur durchgeführt werden. Dadurch kann, unter Inkaufnahme einer längeren Behandlungszeit, die Anzahl der benötigten Lichtapplikatoren und/oder Einstiche reduziert werden, wodurch die Patientenbelastung und die Kosten für die Behandlung ggf. reduziert werden können.

Beispielsweise kann die Sollpunktgitterstruktur mehrere Sollpunktgitterflächen aufweisen, die entlang der Einstichachse einen Gitterflächenabstand k aufweisen. Die Sollpunktgitterflächen können sich flach als Sollpunktgitterebenen oder gekrümmt als Teil der Sollpunktgitterstruktur erstrecken. Die perkutane PDT kann dann sukzessive für eine Sollpunktgitterfläche nach der anderen durchgeführt werden. Beispielsweise kann eine Bedienperson mit einer am tiefsten gelegenen Sollpunktgitterfläche beginnen und sich dann durch entsprechendes proximalwärtiges Herausziehen der Lichtapplikatoren um jeweils einen Gitterflächenabstand k Sollpunktgitterfläche für Sollpunktgitterfläche vorarbeiten, bis die gesamte Sollpunktgitterstruktur bestrahlt wurde und damit das Organ oder Organsegment lückenlos bestrahlt wurde.

Die virtuelle organspezifische Sollpunktgitterstruktur liegt im Inneren des organischen Körpers über das Organ oder Organsegment verteilt und ist damit für die Bedienperson nicht sichtbar. Die dreidimensionale Fixierpunktgitterstruktur hingegen, die vorzugsweise durch die Platzierungsschablone mit ihren Fixierpunktaufnahmen und die Lichtapplikatoren mit ihren Fixierpunkten in Kombination miteinander definiert wird, liegt außerhalb des Körpers für die Bedienperson einsehbar und stellt sicher, dass die lichtemittierenden Applikatorspitzen sich dann an den entsprechenden Gitterpunkten der Sollpunktgitterstruktur befinden, wenn sich der Fixierpunkt des jeweiligen Lichtapplikators am zugehörigen Gitterpunkt der Fixierpunktgitterstruktur, d.h. in der entsprechenden Fixierpunktaufnahme der Platzierungsschablone, befindet.

Eine Bedienperson kann beispielsweise durch eine Führung, die sich erstens zentral in der Platzierungsschablone befindet und deren zugehörige Fixierpunktaufnahme zweitens weitgehend distal in der Platzierungsschablone angeordnet ist, einen ersten Lichtapplikator in das Organ oder Organsegment einstechen. Die Bedienperson kann dann mittels eines bildgebenden Verfahrens, beispielsweise mittels Ultraschall-Sonographie, die Applikatorspitze dieses ersten Lichtapplikators an einem dieser Fixierpunktaufnahme entsprechenden Gitterpunkt der Sollpunktgitterstruktur im Organ oder Organsegment, d.h. gleichfalls weitgehend distal und innerhalb dieser ausgewählten, distalen Sollpunktgitterebene gleichfalls zentral angeordnet, platzieren. Der Körper des Patienten ist dabei vorzugsweise relativ zu einer festen Referenzfläche, beispielsweise einem Behandlungstisch, fixiert. Die Platzierungsschablone kann dann bei korrekt positioniertem ersten Lichtapplikator bzw. dessen lichtemittierender Applikatorspitze und unter Aufrechterhaltung dieser ausgewählten Lichtapplikator-Position relativ zu diesem entlang der Einstichachse verschoben und so positioniert werden, dass sich der Fixierpunkt des ersten Lichtapplikators am entsprechenden Gitterpunkt der Fixierpunktgitterstruktur befindet. In dieser Position kann sowohl der Fixierpunkt des Lichtapplikators an der Fixierpunktaufnahme der Platzierungsschablone als auch die Platzierungsschablone an der äußeren Referenzfläche, beispielsweise einem Behandlungstisch, fixiert werden.

Mit diesem Vorgang wird die dreidimensionale Fixierpunktgitterstruktur in Bezug auf den Körper des Patienten räumlich festgelegt. Zudem wird mit diesem Vorgang, durch welchen also der definierte Abstand d zwischen virtueller Sollpunktgitterstruktur und Fixierpunktgitterstruktur hergestellt wird, auch der erste Schritt der erfindungsgemäßen Vorgehensweise, nämlich die erste Abbildung, d.h. die Parallelverschiebung bzw. Translation der virtuellen Sollpunktgitterstruktur im Organ bzw. Organsegment innerhalb des menschlichen Körpers in einen Bereich außerhalb des menschlichen Körpers abgeschlossen.

Die Platzierungen der weiteren einzelnen Lichtapplikatoren ist nun besonders einfach, fehlerfrei und schnell durchführbar, da sie jeweils nur durch die entsprechenden Führungen der Platzierungsschablone eingestochen werden müssen und so weit vorgeschoben werden müssen, bis sich ihr Fixierpunkt im entsprechenden Gitterpunkt der Fixierpunktgitterstruktur, d.h. in der Fixierpunktaufnahme der Platzierungsschablone befindet. Automatisch befindet sich dann die Applikatorspitze am zugehörigen Gitterpunkt der Sollpunktgitterstruktur. Die virtuelle organspezifische Sollpunktgitterstruktur stellt sicher, dass zum einen die Applikatorspitzen nicht zu weit voneinander entfernt sind, sodass keine Bestrahlungslücken im Organ oder Organsegment entstehen, und zum anderen nicht zu nah zueinander angeordnet sind, um den Patienten nicht mit unnötig vielen Einstichen belasten zu müssen. Die Sollpunktgitterstruktur ist organspezifisch, da die äußere Form und Größe der Sollpunktgitterstruktur vom Organ oder Organsegment abhängt. Zudem hängt die Dichte der Sollpunkte der Sollpunktgitterstruktur davon ab, wie groß die Eindringtiefe des Lichts in das Gewebe des jeweiligen Organs oder Organsegments ist. Je geringer die Eindringtiefe des Bestrahlungslichts ist, umso dichter muss die Sollpunktgitterstruktur gewählt werden, um eine lückenlose Bestrahlung des Organs oder Organsegments sicherzustellen.

Optional kann die Versorgungseinheit dazu eingerichtet sein, die Lichtapplikatoren mit Strom zu versorgen, wobei jeder Lichtapplikator am distalen Ende des Einführabschnitts eine mit dem Strom betreibbare LED und/oder eine andere lichtemittierende Komponente aufweist. Gegenüber einer Lichtleiterauskopplung hat eine LED und/oder eine andere entsprechende lichtemittierende Komponente am distalen Ende des Einführabschnitts den großen Vorteil, dass kein teurer Laser benötigt wird, dessen Licht über die Versorgungseinheit in den Lichtleiter eingekoppelt werden muss. Da die Versorgungseinheit die Lichtapplikatoren lediglich mit Strom versorgen muss, kann sie besonders einfach und günstig ausgestaltet sein. Die LED und/oder die andere lichtemittierende Komponente weist vorzugsweise ein auf den Photosensibilisator bzw. Markerstoff abgestimmtes Leuchtspektrum auf. Alternativ oder zusätzlich kann dazu ein Lichtfilter eingesetzt werden.

Optional sind die Fixierpunktaufnahmen jeweils in oder an einer zugehörigen Führung angeordnet oder werden von dieser gebildet.

Optional können die Lichtapplikatoren jeweils mindestens einen Fixierpunkt aufweisen, der durch einen Anschlag ausgebildet ist und entsprechend der dreidimensionalen Fixierpunktgitterstruktur an einer Fixierpunktaufnahme der Platzierungsschablone arretierbar ist. Durch den Anschlag und die Arretierung erhält eine Bedienperson ein haptisches, akustisches und/oder optisches Feedback über die korrekte Platzierung des Fixierpunkts an einem Gitterpunkt der Fixierpunktgitterstruktur bzw. der entsprechenden Fixierpunktaufnahme.

Optional kann die virtuelle organspezifische Sollpunktgitterstruktur eine Mehrzahl von räumlich auf Sollpunktgitterflächen verteilt angeordneten Sollpunkten aufweisen, wobei die Sollpunktgitterflächen entlang der Einstichachse einen Abstand k zueinander haben. Vorzugsweise erstrecken sich die Sollpunktgitterflächen quer zur Einstichachse.

Optional können die Lichtapplikatoren jeweils mindestens zwei Fixierpunkte aufweisen, welche zueinander entlang der Einstichachse den Abstand k haben, den auch die Sollpunktgitterflächen entlang der Einstichachse zueinander haben. Dadurch hat die Bedienperson die Möglichkeit, einen der Fixierpunkte auszuwählen und am entsprechenden Gitterpunkt der Fixierpunktgitterstruktur bzw. der zugehörigen Fixierpunktaufnahme der Platzierungsschablone zu fixieren und dadurch die Sollpunktgitterfläche festzulegen, in der die Applikatorspitze liegen soll.

Optional kann die Platzierungsschablone eine Mehrzahl von räumlich auf Fixierpunktaufnahmegitterflächen verteilt angeordneten Fixierpunktaufnahmen aufweisen, wobei die Fixierpunktaufnahmegitterflächen entlang der Einstichachse den Abstand k zueinander haben, den auch die entsprechenden Sollpunktgitterflächen entlang der Einstichachse zueinander haben. Eine Bedienperson hat dabei die Möglichkeit, durch Auswahl der Fixierpunktaufnahme, in der der Fixierpunkt arretiert werden soll, die Sollpunktgitterfläche festzulegen, in welcher die Applikatorspitze liegen soll.

Optional kann die virtuelle organspezifische Sollpunktgitterstruktur eine Mehrzahl von räumlich auf Sollpunktgitterflächen verteilt angeordneten Sollpunkten aufweisen, wobei mindestens acht der Sollpunkte Eckpunkte einer Gitterelementarzelle der Sollpunktgitterstruktur in Form eines Parallelepipeds bilden. Vorzugsweise weist dabei die Gitterelementarzelle drei Gitterelementarzellenkanten und vier Gitterelementarzellendiagonalen auf, von denen eine oder keine entlang der Einstichachse verläuft. Die Variante, bei der keine der drei Gitterelementarzellenkanten oder vier Gitterelementarzellendiagonalen entlang der Einstichachse verläuft, hat den Vorteil, dass mehrere Sollpunktgitterflächen gleichzeitig mit Applikatorspitzen besetzt werden können und somit die PDT schneller durchgeführt werden kann. Die Variante, bei der eine der drei Gitterelementarzellenkanten oder der vier Gitterelementarzellendiagonalen entlang der Einstichachse verläuft, hat dagegen den Vorteil, dass ein Einstich für mehrere Sollpunktgitterflächen verwendet werden kann und somit zum einen weniger Einstiche benötigt werden, was für den Patienten gewebeschonender ist, und zum anderen weniger Lichtapplikatoren benötigt werden, wodurch das System kostengünstiger wird.

Gemäß einem zweiten Aspekt der vorliegenden Offenbarung, der vorzugsweise mit dem ersten Aspekt kombinierbar oder unabhängig davon ist, wird ein System zur Durchführung einer transkutanen PDT in einem Organ oder Organsegment eines organischen Körpers bereitgestellt, wobei das System eine Mehrzahl von Lichtapplikatoren, eine Versorgungseinheit zum Versorgen der Lichtapplikatoren mit Licht und/oder Strom und eine in einer definierten Position zum organischen Körper platzierbare Platzierungsschablone zum definierten Ausrichten, Platzieren und Fixieren der Lichtapplikatoren aufweist. Die Lichtapplikatoren weisen dabei jeweils einen nadelartigen Einführabschnitt zum transkutanen Einstechen entlang einer Einstichachse in das Organ oder Organsegment und am distalen Ende des Einführabschnitts eine lichtemittierende Applikatorspitze auf, sowie mindestens einen definierten Fixierpunkt in einem proximalen Abstand d von der Applikatorspitze. Zudem definiert die Platzierungsschablone eine Mehrzahl an Fixierpunktaufnahmen, mittels welcher die Lichtapplikatoren mit ihrem mindestens einen definierten Fixierpunkt fixierbar sind. Die Platzierungsschablone kann dabei eine Mehrzahl an Führungen definieren, wobei durch jede der Führungen ein Einführabschnitt eines der Lichtapplikatoren geführt transkutan in das Organ oder Organsegment entlang der Einstichachse einstechbar ist. Die Fixierpunktaufnahmen können an oder in den Führungen ausgebildet sein.

Die Platzierungsschablone weist gemäß dem zweiten Aspekt mindestens einen ersten Schablonenteil auf, der eine erste Untergruppe der Fixierpunktaufnahmen definiert, und mindestens einen zweiten Schablonenteil, der eine zweite Untergruppe der Fixierpunktaufnahmen definiert, wobei der erste Schablonenteil relativ zum zweiten Schablonenteil entlang der Einstichachse geführt verschiebbar ist.

Durch die Schablonenteile können verschiedene Untergruppen von Lichtapplikatoren, mittels Fixierung in entsprechenden Untergruppen von Fixierpunktaufnahmen, gemeinsam konzertiert wahlweise bewegt oder festgehalten werden. Dadurch wird es möglich, mit einer Untergruppe von Lichtapplikatoren das Organ oder Organsegment festzuhalten bzw. seine Form aufrecht zu erhalten, während die andere Untergruppe bewegt wird.

Ein solches System und eine solche Vorgehensweise können aus nachfolgend genanntem Grund vorteilhaft sein. Werden ein in das Organ oder Organsegment eingeführter Lichtapplikator bewegt bzw. verschoben, dann übt dieser auf das Organ oder Organsegment bzw. auf das Organgewebe, welches am Lichtapplikator anliegt, eine Haftreibungskraft aus. Werden die Lichtapplikatoren alle gemeinsam bzw. gleichzeitig in eine Richtung bewegt, dann ist auch die Summe dieser Haftreibungskräfte auf das Organ oder Organsegment in diese Richtung entsprechend groß. Abhängig von den unterschiedlichen, nachfolgend erläuterten Randbedingungen können dann zwei prinzipiell unterschiedliche, relevante Fälle eintreten, die auch in kombinierter Form auftreten können.

In einem ersten Fall, welcher auf der Randbedingung basiert, dass die beim gemeinsamen bzw. gleichzeitigen Bewegen der Lichtapplikatoren in eine Richtung auf das Organ bzw. Organsegment wirksame Gesamthaftreibungskraft größer ist als die Bindungskräfte, die innerhalb des umgebenden, mit dem zu therapierenden Organ oder Organsegment direkt verbundenen Gewebe wirken und wiederum kleiner sind als die innerhalb des Organs oder Organsegments selbst wirksamen Bindungskräfte, kann das Organ oder Organsegment von den gemeinsam bzw. gleichzeitig in eine gemeinsame Richtung bewegten Lichtapplikatoren als Ganzes und in seiner Form unverändert "mitgenommen" werden, d.h. das Organ oder Organsegment hält beim gemeinsamen bzw. gleichzeitigen Verschieben der Lichtapplikatoren in eine Richtung unter den vorausgehend genannten Randbedingungen zwar seine Form aufrecht, verändert aber seine Position im Körper. In diesem ersten Fall würde dann zwar die Sollpunktgitterstruktur in ihrer Form unverändert bleiben und dadurch weiterhin der Form der Fixierpunktgitterstruktur entsprechen, allerdings würde sich die Lage der Sollpunktgitterstruktur gegenüber der weiterhin unveränderten Lage der Fixierpunktgitterstruktur ändern, d.h. die Fixierpunktgitterstruktur wäre in unerwünschter Weise nicht mehr länger um die ursprüngliche, die Parallelverschiebung kennzeichnende Länge d entlang der Einstichachse, welche dem Abstand d zwischen Applikatorspitze und Fixierpunkt entspricht, parallelverschoben.

In einem zweiten Fall, welcher auf der Randbedingung basiert, dass die beim gemeinsamen bzw. gleichzeitigen Bewegen der Lichtapplikatoren in eine Richtung auf das Organ oder Organsegment wirksame Gesamthaftreibungskraft größer ist als die innerhalb des Organs oder Organsegments selbst wirksamen Bindungskräfte, welche wiederum kleiner sind als die Bindungskräfte, die innerhalb des umgebenden, mit dem zu therapierenden Organ oder Organsegment direkt verbundenen Gewebe wirken, kann das Organ oder Organsegment in Bewegungsrichtung der Lichtapplikatoren gedehnt bzw. gestreckt werden, d.h. das Organ oder Organsegment hält beim gemeinsamen bzw. gleichzeitigen Verschieben der Lichtapplikatoren in eine Richtung unter den vorausgehend genannten Randbedingungen zwar seine Position im Körper in erster Näherung aufrecht, verändert aber seine Form. In diesem zweiten Fall wäre dann zwar die Lage der Sollpunktgitterstruktur gegenüber der Lage der Fixierpunktgitterstruktur in erster Näherung unverändert, d.h. der Abstand zwischen den beiden Gitterstrukturen entspräche nach dem gemeinsamen bzw. gleichzeitigen Verschieben der Lichtapplikatoren in erster Näherung noch der die Parallelverschiebung kennzeichnenden Länge d, allerdings würde sich mit der Form des Organs in gleicher Weise die Form der Sollpunktgitterstruktur ändern, die wiederum dann aber nicht mehr länger der Form der Fixierpunktgitterstruktur entspräche.

In beiden Fällen, oder einer Kombination daraus, wäre ein solcher "Aufgabeleffekt" negativ, da, mathematisch-geometrisch betrachtet, in beiden Fällen die ursprüngliche Parallelverschiebung um die Länge d, durch welche die Fixierpunktgitterstruktur aus der Sollpunktgitterstruktur hervorging und welche die Basis der hier dargestellten Vorgehensweise für eine vollständige und vor allen Dingen punktgenaue Behandlung des Organs oder Organsegments bildet, beeinträchtigt bzw. modifiziert würde, d.h. kennzeichnende Parameter der Parallelverschiebung (Objekt-Form, Verschiebungslänge d) verändert würden und deshalb eine gleichmäßige und vor allen Dingen lückenlose Bestrahlung nicht mehr automatisch gewährleistet wäre.

Beide erläuterten Fälle des "Aufgabeleffekts" bzw. deren Auswirkungen gewinnen mit zunehmender Anzahl von Lichtapplikatoren an Bedeutung, weil jeder zusätzliche Lichtapplikator an seiner Kontaktfläche mit dem Organ oder Organsegment, welche im Wesentlichen durch den Lichtapplikator-Schaft gebildet wird, eine zusätzliche Haftreibungskraft auf das Organ oder Organsegment ausübt, welche die Gesamthaftreibungskraft weiter erhöht. Damit steigt mit zunehmender Anzahl von Lichtapplikatoren die Wahrscheinlichkeit, dass die Gesamthaftreibungskraft entweder die Bindungskräfte innerhalb des umgebenden, mit dem Organ direkt verbundenen Gewebes übersteigt (erster oben beschriebener Fall) und/oder die Bindungskräfte innerhalb des Organs oder Organsegments selbst übersteigt (zweiter oben beschriebener Fall) und daraus entweder eine Verschiebung des Organs oder Organsegments in proximale Richtung (erster oben beschriebener Fall), und/oder eine Verformung des Organs oder Organsegments (zweiter oben beschriebener Fall) resultiert.

Der "Aufgabeleffekt" kann dadurch vermieden werden, dass eine Untergruppe von Lichtapplikatoren festgehalten wird, während die andere bewegt bzw. verschoben bzw. herausgezogen wird. Die jeweiligen Untergruppen können abwechselnd bewegt werden, während die andere festgehalten wird. Die gemeinsame Bewegung und das gemeinsame Festhalten der Lichtapplikatoren kann durch entsprechende Bewegung bzw. Festhalten der Schablonenteile bewirkt werden.

Optional kann die erste Untergruppe der Fixierpunktaufnahmen ein erstes Fixierpunktaufnahmeteilgitter und die zweite Untergruppe der Fixierpunktaufnahmen ein zweites Fixierpunktaufnahmeteilgitter definieren, wobei das zweite Fixierpunktaufnahmeteilgitter quer zur Einstichachse um einen lateralen Gitterversatz versetzt zum ersten Fixierpunktaufnahmeteilgitter ist. Vorzugsweise hat dadurch jeder Lichtapplikator mindestens einen anderen Lichtapplikator als lateral nächstliegenden Nachbar, der in der jeweils anderen Untergruppe der Führungen steckt. Dadurch kann der "Aufgabeleffekt" besonders gut vermieden werden, weil sich die resultierenden Haftreibungskräfte benachbarter Lichtapplikatoren paarweise in erster Näherung aufheben und dadurch auch die Gesamthaftreibungskraft Null ergibt. Außerdem können dadurch auch lokale Verschiebungen und/ Verformungen des Organs oder Organsegments vermieden werden. Vorzugsweise weist das erste Fixierpunktaufnahmeteilgitter in etwa die gleiche Anzahl an Fixierpunktaufnahmen auf wie das zweite Fixierpunktaufnahmeteilgitter.

Optional kann der laterale Gitterversatz kleiner sein als eine Seitenlänge einer Gitterelementarzelle des ersten Fixierpunktaufnahmeteilgitters und/oder des zweiten Fixierpunktaufnahmeteilgitters. Vorzugsweise ist die Seitenlänge einer Gitterelementarzelle des ersten Fixierpunktaufnahmeteilgitters und des zweiten Fixierpunktaufnahmeteilgitters identisch und der laterale Gitterversatz beträgt die Hälfte der Seitenlänge einer Gitterelementarzelle. Der Gitterversatz kann vorzugsweise in zwei orthogonalen seitlichen Richtungen vorgesehen sein, sodass eine Fixierpunktaufnahme des ersten Fixierpunktaufnahmeteilgitters den Mittelpunkt einer Gitterzelle des zweiten Fixierpunktaufnahmeteilgitters bildet und umgekehrt.

Optional kann das erste Fixierpunktaufnahmeteilgitter und das zweite Fixierpunktaufnahmeteilgitter in einer Fixierpunktaufnahmegitterfläche liegen. Dadurch ergänzen sich die Fixierpunktaufnahmeteilgitter zu einem gesamten Fixierpunktaufnahmegitter in einer Fixierpunktaufnahmegitterfläche. Eine solche Anordnung hat - im Vergleich mit der nachfolgend beschriebenen Anordnung - den Vorteil, dass sich beim Bewegen bzw. Verschieben eines Schablonenteils, welcher eines der beiden Fixierpunktaufnahmeteilgitter repräsentiert, die in entgegengesetzte Richtungen zeigenden Reibungskräfte noch besser aufheben, weil die die Reibungskräfte bestimmenden Schaftlängen innerhalb des Organs oder Organsegments gleich oder ähnlich sind, weil sie zumindest zeitweise paarweise gleich weit in das Organ oder Organsegment hineinragen. Bei der angestrebten paarweisen Aufhebung der Reibungskräfte wird zunächst vereinfachend zu Grunde gelegt, dass beide Fixierpunktaufnahmegitterflächen mit der gleichen Anzahl von Applikatoren besetzt sind. Lägen die Fixierpunktaufnahmeteilgitter jedoch nicht in derselben Fixierpunktaufnahmegitterfläche, kann es sein, dass sich die Reibungskräfte nicht mehr vollständig paarweise kompensieren, da sich die jeweiligen Schaftlängen unterscheiden, welche sich innerhalb des Organs oder Organsegments befinden. Dies kann ganz oder teilweise dadurch korrigiert werden, dass das Fixierpunktaufnahmeteilgitter bzw. das entsprechende Schablonenteil, welches zuerst in proximale Richtung bewegt wird, mit einer höheren Anzahl von Lichtapplikatoren belegt wird.

Optional können die erste Untergruppe der Führungen und Fixierpunktaufnahmen, die zweite Untergruppe der Führungen und Fixierpunktaufnahmen sowie die Lichtapplikatoren zusammen eine dreidimensionale Fixierpunktgitterstruktur definieren, wobei die Fixierpunktgitterstruktur einer virtuellen organspezifischen Sollpunktgitterstruktur für die lichtemittierenden Applikatorspitzen im Organ oder Organsegment entspricht und um eine Länge d entlang der Einstichachse parallelverschoben zur Sollpunktgitterstruktur ist, wobei jeder Lichtapplikator mindestens einen definierten Fixierpunkt im Abstand d von der Applikatorspitze aufweist.

Optional kann die Versorgungseinheit dazu eingerichtet sein, die Lichtapplikatoren mit Strom zu versorgen und jeder Lichtapplikator kann am distalen Ende des Einführabschnitts eine mit dem Strom betreibbare LED oder eine andere lichtemittierende Komponente aufweisen. Gegenüber einer Lichtleiterauskopplung hat eine LED am distalen Ende des Einführabschnitts den großen Vorteil, dass kein teurer Laser benötigt wird, dessen Licht über die Versorgungseinheit in den Lichtleiter eingekoppelt werden muss. Da die Versorgungseinheit die Lichtapplikatoren lediglich mit Strom versorgen muss, kann sie besonders einfach und günstig ausgestaltet sein. Die LED weist vorzugsweise ein auf den Photosensibilisator bzw. Markerstoff abgestimmtes Leuchtspektrum auf. Alternativ oder zusätzlich kann dazu ein Lichtfilter eingesetzt werden.

Optional können die Lichtapplikatoren jeweils mindestens einen Fixierpunkt aufweisen, der durch einen Anschlag ausgebildet und arretierbar ist. Durch den Anschlag und die Arretierung erhält eine Bedienperson ein haptisches, akustisches und/oder optisches Feedback über die korrekte Platzierung des Fixierpunkts an einem Gitterpunkt der Fixierpunktgitterstruktur bzw. der entsprechenden Fixierpunktaufnahme der Platzierungsschablone bzw. des Schablonenteils.

Optional kann die virtuelle organspezifische Sollpunktgitterstruktur eine Mehrzahl von räumlich auf Sollpunktgitterflächen verteilt angeordneten Sollpunkten aufweisen, wobei die Sollpunktgitterflächen entlang der Einstichachse einen Abstand k zueinander haben. Vorzugsweise erstrecken sich die Sollpunktgitterflächen orthogonal zur Einstichachse.

Optional können mindestens acht der Sollpunkte Eckpunkte einer Gitterelementarzelle der Sollpunktgitterstruktur in Form eines Parallelepipeds bilden. Optional kann dabei die Gitterelementarzelle drei Gitterelementarzellenkanten und vier Gitterelementarzellendiagonalen aufweisen, von denen eine entlang der Einstichachse verläuft. Dadurch ist es besonders einfach, alle im ersten Schablonenteil geführten Lichtapplikatoren durch Verschieben des ersten Schablonenteils zeitgleich gemeinsam so zu positionieren, dass die jeweiligen lichtemittierenden Applikatorspitzen von einer Sollpunktgitterfläche zur nächsten geführt werden. Währenddessen können die durch das zweite Schablonenteil geführten Lichtapplikatoren ortsfest gehalten werden und somit das Organ oder Organsegment festhalten und/oder seine Verformung unterdrücken, während die im ersten Schablonenteil geführten Lichtapplikatoren gemeinsam bewegt werden. Sobald die im ersten Schablonenteil geführten Lichtapplikatoren platziert sind, können diese ortsfest gehalten werden, während die im zweiten Schablonenteil geführten Lichtapplikatoren mit ihren lichtemittierenden Applikatorspitzen zur nächsten Sollpunktgitterfläche bewegt werden. Vorzugsweise beginnt dieser Prozess mit der distalwärtig am tiefsten im Körper gelegenen Sollpunktgitterfläche des jeweiligen Schablonenteils und man arbeitet sich dann durch Zurückziehen des jeweiligen Schablonenteils zur nächsten Sollpunktgitterfläche proximalwärts vor. Die PDT kann jeweils dann durchgeführt werden, wenn die Applikatorspitzen an ihrem Sollpunkt im Sollpunktgitter positioniert sind.

Optional kann eine mit der ersten Untergruppe der Führungen bzw. Fixierpunktaufnahmen korrespondierende erste Untergruppe der Sollpunkte ein erstes Sollpunktteilgitter und eine mit der zweiten Untergruppe der Führungen bzw. Fixierpunktaufnahmen korrespondierende zweite Untergruppe der Sollpunkte ein zweites Sollpunktteilgitter definieren, sodass sich das erste Sollpunktteilgitter und das zweite Sollpunktteilgitter zu einer der Sollpunktgitterflächen ergänzen. Auf diese Weise kann jede Sollpunktgitterfläche dadurch vollständig mit lichtemittierenden Applikatorspitzen besetzt werden, dass zunächst ein Sollpunktteilgitter mit lichtemittierenden Applikatorspitzen besetzt wird und sodann das andere Sollpunktteilgitter besetzt wird. Dies kann auf einfache Weise durch entsprechendes Verschieben der Schablonenteile relativ zueinander erfolgen.

Optional können die Lichtapplikatoren jeweils mindestens zwei Fixierpunkte aufweisen, welche zueinander entlang der Einstichachse den Abstand k haben, den auch die Sollpunktgitterflächen zueinander haben. Damit kann die Bedienperson durch einen der Fixierpunkte, der im Gitterpunkt der Fixierpunktgitterstruktur bzw. in der entsprechenden Fixierpunktaufnahme des jeweiligen Schablonenteils arretiert werden soll, bestimmen, in welcher Sollpunktgitterfläche die lichtemittierende Applikatorspitze liegen soll.

Optional kann die Platzierungsschablone eine Mehrzahl von räumlich auf Fixierpunktaufnahmegitterflächen verteilt angeordneten Fixierpunktaufnahmen aufweisen, wobei die Fixierpunktaufnahmegitterflächen entlang der Einstichachse den Abstand k zueinander haben, den auch die Sollpunktgitterflächen zueinander haben. Damit kann eine Bedienperson durch Auswahl einer der Fixierpunktaufnahmen, in der ein Fixierpunkt arretiert werden soll, bestimmen, in welcher Sollpunktgitterfläche die Applikatorspitze liegen soll.

Optional kann der erste Schablonenteil eine erste Untergruppe der Fixierpunktaufnahmen einer ersten Fixierpunktaufnahmegitterfläche und der zweite Schablonenteil eine zweite Untergruppe der Fixierpunktaufnahmen einer zweiten Fixierpunktaufnahmegitterfläche aufweisen. Die beiden Untergruppen von Fixierpunktaufnahmen können sich daher über zwei Fixierpunktgitterflächen der Fixierpunktgitterstruktur ergänzen.

Optional kann der zweite Schablonenteil in proximale Richtung relativ zum ersten Schablonenteil nur bis zur Anlage an den ersten Schablonenteil verschiebbar sein, wobei bei Anlage des zweiten Schablonenteils am ersten Schablonenteil die vom ersten Schablonenteil definierte erste Untergruppe der Fixierpunktaufnahmen distal von der vom zweiten Schablonenteil definierten zweiten Untergruppe der Fixierpunktaufnahmen liegt.

Im Folgenden wird das hierin offenbarte System näher anhand der beiliegenden Figuren erläutert. Es zeigen:
- Fig. 1: eine schematische Darstellung der prinzipiellen Funktionsweise einer perkutanen PDT mit einem Lichtapplikator;
- Fig. 2: eine schematische Darstellung der prinzipiellen Funktionsweise einer perkutanen PDT mit mehreren Lichtapplikatoren mit relativ hoher Eindringtiefe des Lichts;
- Fig. 3: eine schematische Darstellung der prinzipiellen Funktionsweise einer perkutanen PDT mit mehreren Lichtapplikatoren mit relativ niedriger Eindringtiefe des Lichts;
- Fig. 4: eine schematische Darstellung einer prinzipiell gewünschten Verteilung von mehreren Lichtapplikatoren bei einer perkutanen PDT;
- Fig. 5: eine schematische Darstellung einer virtuellen organspezifischen Sollpunktgitterstruktur für die Applikatorspitzen im Organ oder Organsegment;
- Fig. 6: eine schematische Darstellung einer zur virtuellen organspezifischen Sollpunktgitterstruktur aus Fig. 5 parallelverschobenen Fixierpunktgitterstruktur;
- Fig. 7: eine schematische Darstellung eines Ausführungsbeispiels eines hierin offenbarten Systems zur Durchführung einer transkutanen PDT;
- Fig. 8: eine schematische Darstellung des in Fig. 7 gezeigten Systems mit sämtlichen Lichtapplikatoren in eingestochener Position für die PDT;
- Fig. 9: eine schematische Darstellung eines weiteren Ausführungsbeispiels eines hierin offenbarten Systems zur Durchführung einer transkutanen PDT sukzessive Sollpunktgitterfläche für Sollpunktgitterfläche;
- Fig. 10: eine schematische Darstellung des in Fig. 9 gezeigten Systems mit Lichtapplikatoren für die PDT;
- Fig. 11: eine schematische Darstellung eines weiteren Ausführungsbeispiels eines hierin offenbarten Systems zur Durchführung einer transkutanen PDT mit Applikatorspitzen in einer ersten Sollpunktgitterfläche;
- Fig. 12: eine schematische Darstellung des in Fig. 11 gezeigten Systems mit Applikatorspitzen in einer zweiten Sollpunktgitterfläche;
- Fig. 13: eine schematische Darstellung des in Fig. 11 und 12 gezeigten Systems mit Applikatorspitzen in einer dritten Sollpunktgitterfläche;
- Fig. 14: eine schematische Darstellung eines weiteren Ausführungsbeispiels eines hierin offenbarten Systems zur Durchführung einer transkutanen PDT mit Applikatorspitzen in einer ersten Sollpunktgitterfläche;
- Fig. 15: eine schematische Darstellung des in Fig. 14 gezeigten Systems mit Applikatorspitzen in einer zweiten Sollpunktgitterfläche;
- Fig. 16: eine schematische Darstellung des in Fig. 14 und 15 gezeigten Systems mit Applikatorspitzen in einer dritten Sollpunktgitterfläche;
- Fig. 17: eine schematische Darstellung eines weiteren Ausführungsbeispiels eines hierin offenbarten Systems zur Durchführung einer transkutanen PDT mit sämtlichen Lichtapplikatoren in eingestochener Position;
- Fig. 18: eine schematische Darstellung eines weiteren Ausführungsbeispiels eines hierin offenbarten Systems zur Durchführung einer transkutanen PDT mit sämtlichen Lichtapplikatoren in eingestochener Position;
- Fig. 19: eine schematische Darstellung eines weiteren Ausführungsbeispiels eines hierin offenbarten Systems zur Durchführung einer transkutanen PDT sukzessive Sollpunktgitterfläche für Sollpunktgitterfläche;
- Fig. 20: eine schematische Darstellung des in Fig. 19 gezeigten Systems mit Applikatorspitzen in einer ersten Sollpunktgitterfläche;
- Fig. 21: eine schematische Darstellung des in Fig. 20 gezeigten Systems mit Applikatorspitzen in einer dritten Sollpunktgitterfläche;
- Fig. 22: eine schematische Darstellung eines weiteren Ausführungsbeispiels eines hierin offenbarten Systems zur Durchführung einer transkutanen PDT in zwei Sollpunktgitterflächen gleichzeitig;
- Fig. 23: eine schematische Darstellung des in Fig. 22 gezeigten Systems mit Applikatorspitzen in einer ersten und zweiten Sollpunktgitterfläche;
- Fig. 24: eine schematische Darstellung des in Fig. 22 und 23 gezeigten Systems mit Applikatorspitzen in einer fünften und sechsten Sollpunktgitterfläche;
- Fig. 25a,b: schematische Darstellungen des prinzipiell zu vermeidenden Aufgabeleffekts gemäß erster Variante;
- Fig. 25c,d: schematische Darstellungen des prinzipiell zu vermeidenden Aufgabeleffekts gemäß zweiter Variante;
- Fig. 26: eine schematische Darstellung eines Ausführungsbeispiels eines Systems gemäß dem zweiten Aspekt der vorliegenden Offenbarung zur Vermeidung des Aufgabeleffekts bei einer transkutanen PDT mit Applikatorspitzen in einer ersten Sollpunktgitterfläche;
- Fig. 27: eine schematische Darstellung des in Fig. 26 gezeigten Systems mit einer ersten Untergruppe von Applikatoren in einem ersten Schablonenteil, deren Applikatorspitzen zu einer zweiten Sollpunktgitterfläche gezogen werden, während eine zweite Untergruppe von Applikatoren in einem zweiten Schablonenteil festgehalten wird;
- Fig. 28: eine schematische Darstellung des in Fig. 26 und 27 gezeigten Systems, wobei die Applikatorspitzen der ersten Untergruppe von Applikatoren in der zweiten Sollpunktgitterfläche festgehalten werden, während die Applikatorspitzen der zweiten Untergruppe von Applikatoren zu der zweiten Sollpunktgitterfläche gezogen werden;
- Fig. 29: eine schematische Darstellung eines Ausführungsbeispiels eines Systems gemäß dem zweiten Aspekt der vorliegenden Offenbarung zur Vermeidung des Aufgabeleffekts bei einer transkutanen PDT;
- Fig. 30: eine schematische Darstellung des in Fig. 29 gezeigten Systems mit Applikatorspitzen in einer ersten und zweiten Sollpunktgitterfläche;
- Fig. 31: eine schematische Darstellung des in Fig. 29 und 30 gezeigten Systems mit einer ersten Untergruppe von Applikatoren in einem ersten Schablonenteil, deren Applikatorspitzen zu einer vierten Sollpunktgitterfläche gezogen werden, während eine zweite Untergruppe von Applikatoren in einem zweiten Schablonenteil festgehalten wird;
- Fig. 32: eine schematische Darstellung des in Fig. 31 gezeigten Systems, wobei die Applikatorspitzen der ersten Untergruppe von Applikatoren in der vierten Sollpunktgitterfläche festgehalten werden, während die Applikatorspitzen der zweiten Untergruppe von Applikatoren zu einer dritten Sollpunktgitterfläche gezogen werden;
- Fig. 33: eine schematische Darstellung eines verbesserten Ausführungsbeispiels eines Systems gemäß dem zweiten Aspekt der vorliegenden Offenbarung zur Vermeidung des Aufgabeleffekts bei einer transkutanen PDT;
- Fig. 34: eine schematische Darstellung des in Fig. 33 gezeigten Systems mit Applikatorspitzen in einer ersten und zweiten Sollpunktgitterfläche;
- Fig. 35: eine schematische Darstellung des in Fig. 33 und 34 gezeigten Systems mit einer ersten Untergruppe von Applikatoren in einem ersten Schablonenteil, deren Applikatorspitzen zu einer dritten Sollpunktgitterfläche gezogen werden, während eine zweite Untergruppe von Applikatoren in einem zweiten Schablonenteil festgehalten wird; und
- Fig. 36: eine schematische Darstellung des in Fig. 35 gezeigten Systems, wobei die Applikatorspitzen der ersten Untergruppe von Applikatoren in der dritten Sollpunktgitterfläche festgehalten werden, während die Applikatorspitzen der zweiten Untergruppe von Applikatoren zu einer vierten Sollpunktgitterfläche gezogen werden.

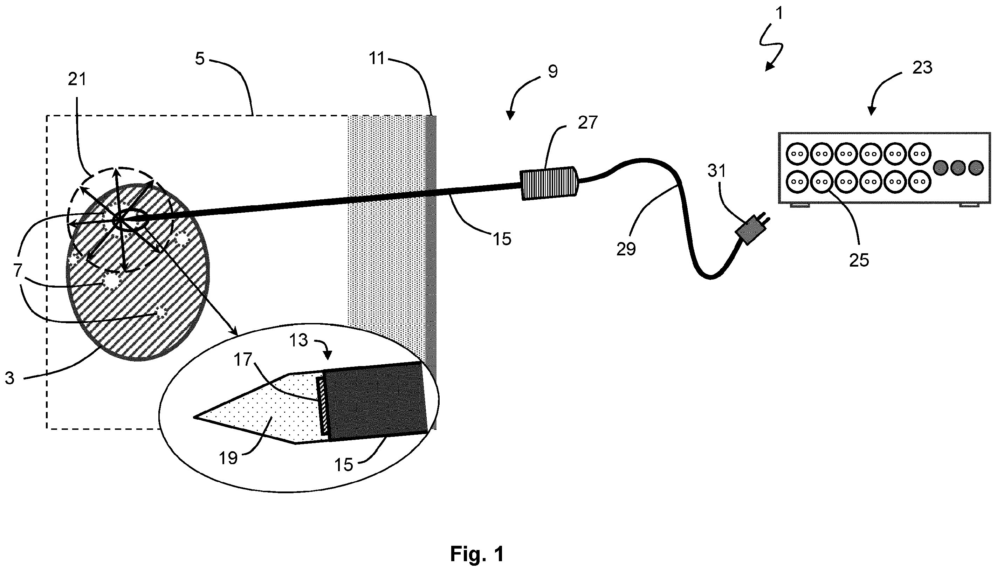

Fig. 1 zeigt ein System 1 zur Durchführung einer transkutanen photodynamischen Therapie (PDT) in einem Organ 3, beispielsweise der Prostata, eines organischen Körpers 5. Das Organ 3 weist hier mehrere pathologische Gewebebereiche 7 (in den Figuren allesamt durch eine gepunktete Umrandung gekennzeichnet) auf, in denen sich ein verabreichter Photosensibilisator bzw. Markerstoff angereichert hat. Allerdings können, beispielsweise aufgrund ihrer geringen Größe und/oder ihres unzureichenden Kontrastes, nicht alle pathologische Gewebebereiche 7 visualisiert werden und sind dementsprechend, also aufgrund ihrer teilweisen Unsichtbarkeit für die Bedienperson, auch nicht allesamt gezielt, d.h. fokal bzw. gewebespezifisch einzeln behandelbar. Man weiß aber aus der Erfahrung, beispielsweise durch Entnahme von Zufallsbiopsien aus dem Organ 3, dass es solche pathologischen Gewebebereiche 7 dennoch fast immer gibt. Sämtliche der sichtbaren und unsichtbaren pathologischen Gewebebereiche 7 sollen nun mit Licht bestrahlt werden, um eine entsprechende therapeutische Wirkung bei allen pathologischen Gewebebereichen 7 zu erzielen. Werden bestimmte pathologischen Gewebebereiche 7, beispielsweise ein Tumor, nicht bestrahlt, so bleiben diese untherapiert und können sich vergrößern oder anderweitig medizinisch schadhaft bleiben oder werden.

In Fig. 1 ist gezeigt, dass das System 1 einen nadelartigen Lichtapplikator9 aufweist, der durch die Haut 11 des Körpers 5, also transkutan, in das Organ 3 gestochen wird. Der Lichtapplikator 9 ist möglichst dünn, um mit dem Einstich möglichst wenig gesundes Gewebe des Körpers 5 zu schädigen, d.h. um einen minimalinvasiven Eingriff zu ermöglichen. Am distalen Ende 13 eines dünnen schaftförmigen Einführabschnitts 15 des Lichtapplikators 9 ist ein lichtemittierendes Element 17 in Form einer LED angeordnet. Distalseitig von der LED 17 ist am distalen Ende 13 des Einführabschnitts 15 des Lichtapplikators 9 eine lichttransparente und lichtstreuende Applikatorspitze 19 angeordnet. Durch die Applikatorspitze 19 wird das Licht der LED 17 möglichst isotrop in einen Raumwinkel von über 3π abgestrahlt. Die Lichtabstrahlung von der Applikatorspitze 19 ist also annähernd kugelförmig, was in Fig. 1 durch eine gestrichelte Lichtkugel 21 bzw. in der vorliegenden Schnittbilddarstellung durch einen gestrichelten Kreis angedeutet ist. Die Größe der Lichtkugel 21 als Orientierung bzw. Maß dafür, in welchem Gewebebereich bzw. Gewebevolumen eine im Hinblick auf die gewünschte therapeutische Wirkung noch ausreichend hohe Lichtmenge ankommt, hängt von der Eindringtiefe des Lichts in das Gewebe des Organs 3 ab und ist daher organspezifisch.

Das System 1 weist ferner eine Versorgungseinheit 23 auf, mit welcher der Lichtapplikator 9 mit Strom versorgt wird, mit welchem die LED 17 betrieben wird. Die Versorgungseinheit 23 weist vorzugsweise eine Mehrzahl von Anschlüssen 25 für eine Mehrzahl von einzelnen Lichtapplikatoren 9 auf, die gleichzeitig bei der PDT zum Einsatz kommen können. Der Lichtapplikator 9 weist am proximalen Ende des Einführabschnitts 15 ein Griffelement 27 auf, mit dem eine Bedienperson den Lichtapplikator 9 manuell greifen und positionieren kann. Über ein Kabel 29 mit einem Stecker 31, der in einen der Anschlüsse 25 der Versorgungseinheit 23 passt, kann der Lichtapplikator 9 jeweils angeschlossen und mit Strom versorgt werden. Der Einführabschnitts 15 des Lichtapplikators 9 weist vorzugsweise einen Kern und einen vom Kern elektrisch isolierten Mantel auf, sodass Kern und Mantel als Hin- und Rückleiterpaar fungieren können, um die LED 17 mit Strom zu versorgen. Alternativ oder zusätzlich dazu kann ein extra Stromleiter im Einführabschnitts 15 des Lichtapplikators 9 vorgesehen sein.

In Fig. 1 wird anhand der Lichtkugel 21 bzw. deren Größe bereits deutlich, dass die Eindringtiefe des Lichts nicht ausreicht, um das ganze Organ 3 mit einem Einstich zu bestrahlen. Ziel ist aber eine lückenlose Bestrahlung des ganzen Organs 3, da einerseits bei der hier vorgestellten Vorgehensweise die Bestrahlung mit Licht für gesundes Gewebe unschädlich ist, weil aufgrund der vergleichsweise geringen applizierten Lichtmenge bei der PDT eine zerstörerische Wirkung nur dort stattfinden kann, wo sich der Photosensibilisator angereichert hat, was wegen der Tumorselektivität des ausgewählten Photosensibilisators nur im malignen Gewebe passiert, und andererseits sichergestellt werden soll, dass kein pathologischer Gewebebereich 7 und insbesondere auch kein für die Bedienperson unsichtbarer pathologischer Gewebebereich 7 im Organ 3 untherapiert bleibt. Eine Bedienperson müsste also nach einer Bestrahlung den Lichtapplikator 9 an anderen Stellen neu einstechen, um mit den entsprechenden Lichtkugeln 21 nach und nach, d.h. sequentiell das gesamte Volumen des Organs 3 abzudecken.

Solch ein Vorgehen erfordert allerdings sehr gute Erfahrung der Bedienperson und ist mit vielen Risiken und Nachteilen verbunden. Zum einen kann nicht sichergestellt werden, dass am Ende das gesamte Volumen des Organs 3 lückenlos bestrahlt wurde, weil einerseits die Bedienperson viel Freiräume bei der Platzierung der Lichtapplikatoren 9 im Organ 3 hat und weil andererseits derjenige Bereich, der therapeutisch wirksam erfasst wurde, bzw. dessen Grenzen, die in Fig. 1 durch die Oberfläche der Lichtkugel 21 bzw. in der dortigen Schnittbilddarstellung durch den Umfang des Kreises dargestellt sind, nicht unmittelbar visualisiert und damit der Bedienperson zur Kontrolle nicht unmittelbar sichtbar gemacht werden können, sodass keine Gewissheit besteht, ob der Lichtapplikator 9 bzw. dessen lichtemittierende Applikatorspitze 19 immer an der richtigen Stelle platziert wurde. Zum anderen kann es zu einem "Herumstochern" kommen, bei dem zu viele Einstiche benötigt werden, die den Patienten belasten. Schließlich kann die PDT in dieser Form sehr lange dauern.

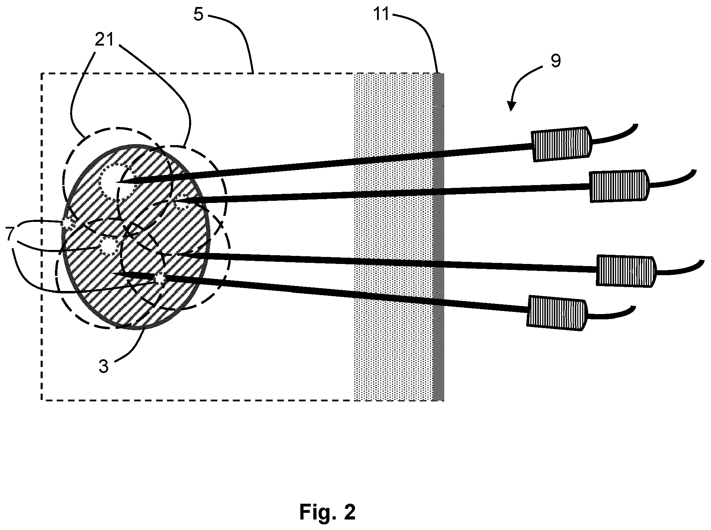

In Fig. 2 ist gezeigt, wie die PDT beschleunigt werden kann, wenn mehrere einzelne Lichtapplikatoren 9 gleichzeitig zum Einsatz kommen. Dies verteuert zwar das System 1, spart aber Zeit. Auch hier ist allerdings große Erfahrung der Bedienperson erforderlich, denn auch in diesem Fall erkennt man, wie vorausgehend schon bei der sequentiellen Platzierung eines einzelnen Lichtapplikators 9 beschrieben wurde, dass trotz der vermeintlich lückenlosen Bestrahlung des Organs 3 ein pathologischer Gewebebereich 7 im Organ 3 unbemerkt untherapiert geblieben ist (in Fig. 2 ganz links), weil auch hier einerseits die beschriebene Vorgehensweise der Bedienperson bei der Platzierung der einzelnen Lichtapplikatoren 9 sehr viel Freiräume lässt, aber andererseits derjenige Bereich, der therapeutisch wirksam erfasst wird/wurde, der Bedienperson zur Kontrolle nicht sichtbar gemacht werden kann, was dann, wie im vorliegenden Beispiel der Fig. 2, in einer unzureichenden Platzierung im Sinne einer lückenhaften bzw. unvollständigen Bestrahlung resultieren kann. Günstig wirkt sich hier aus, wie im Beispiel der Fig. 1 und 2 gezeigt, dass die Eindringtiefe des Lichts relativ groß ist, d.h. die Lichtkugeln 21 sind relativ groß, sodass hier nur relativ wenige Lichtapplikatoren 9 benötigt werden, um das Organ 3 vollständig zu bestrahlen.

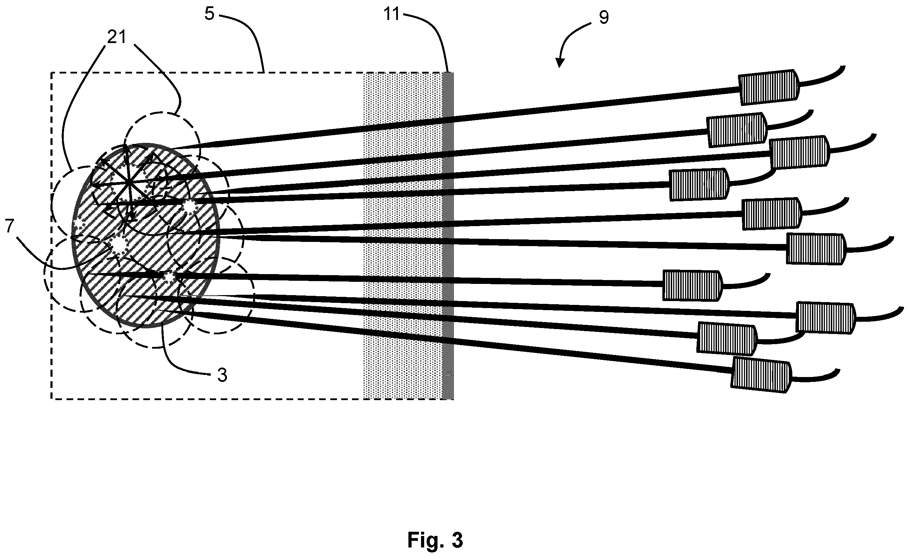

Im Gegensatz zu den vorausgehenden Beispielen ist jedoch in Fig. 3 die Eindringtiefe des Lichts relativ klein, d.h. die Lichtkugeln 21 sind relativ klein, sodass hier relativ viele Lichtapplikatoren 9 benötigt werden, um das Organ 3 vollständig zu bestrahlen. Dadurch wird die Lage in Bezug auf eine korrekte Platzierung der Lichtapplikatoren 9 bzw. deren Applikatorspitzen 19 im Vergleich mit derjenigen in Fig. 1 und Fig. 2 für die Bedienperson entsprechend komplexer, was schließlich dazu führt, dass die Wahrscheinlichkeit, dass bestimmte Gewebebereiche des Organs 3 unbestrahlt und deshalb ein pathologischer Gewebebereich 7 untherapiert bleibt (z.B. in Fig. 3 mittig-links), noch weiter zunimmt.

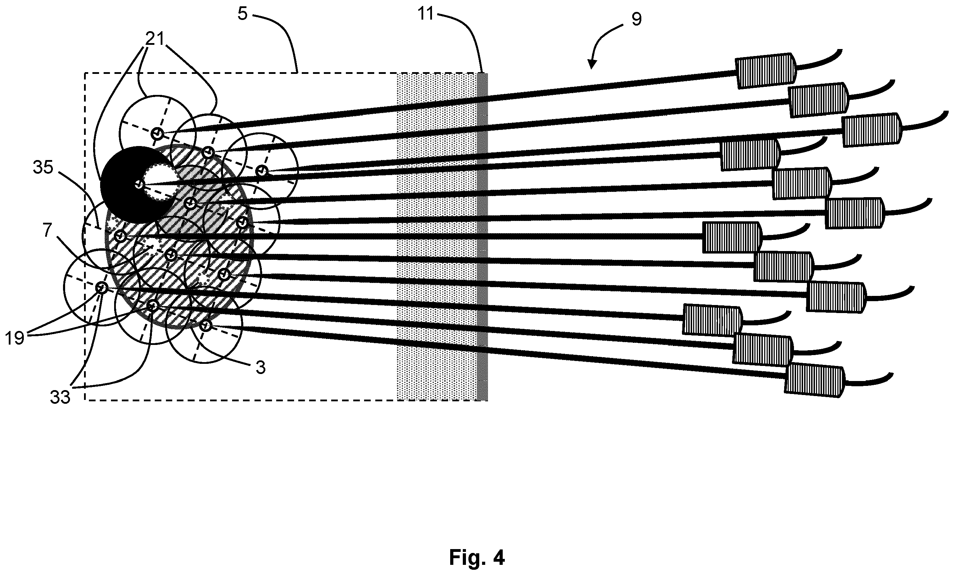

In Fig. 4 ist gezeigt, dass es deshalb, d.h. basierend auf den vorausgehend gewonnenen Erkenntnissen, sinnvoll wäre, die Applikatorspitzen 19 an sämtliche virtuelle Sollpunkte 33 einer virtuellen dreidimensionalen Sollpunktgitterstruktur 35 zu platzieren. Die Sollpunktgitterstruktur 35 ist an die Form und Lage des Organs 3 sowie die Eindringtiefe des Lichts im Organ 3 angepasst. Die Sollpunktgitterstruktur 35 ist also organspezifisch. Die Abstände der virtuellen Sollpunkte 33 sind so an das Organ 3 angepasst, dass sich einerseits die Lichtkugeln 21 lückenlos überlappen und das gesamte Organ 3 lückenlos bestrahlt wird, dass aber andererseits diese Überlappung im Sinne einer patientenschonenden Vorgehensweise vorzugsweise nur so weit wie nötig vorangetrieben wird, d.h. dass unnötig kleine Abstände der lichtemittierenden Applikatorspitzen 19 vermieden werden, um dadurch die Anzahl der Lichtapplikatoren 9 bzw. der Lichtapplikatoreinstiche zu minimieren. Aus diesen beiden gegensätzlichen Anforderungen ergibt sich eine Sollpunktgitterstruktur 35, bei der die Lage und vor allen Dingen die Abstände der virtuellen Sollpunkte 33 zueinander recht genau definiert und vergleichsweise eng toleriert sind.

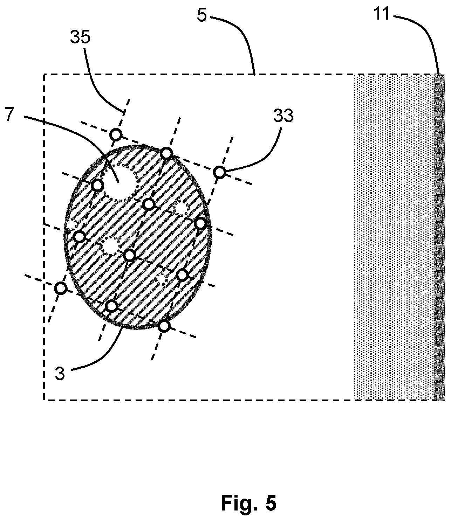

Die Schwierigkeit besteht nun darin, die Applikatorspitzen 19 genau an diesen virtuellen Sollpunkten 33 zu platzieren, da die virtuelle dreidimensionale Sollpunktgitterstruktur 35 für die Bedienperson nicht sichtbar ist und damit die virtuellen Sollpunkte 33 auch nicht gezielt und direkt von der Bedienperson mit den lichtemittierenden Applikatorspitzen 19 angesteuert werden können. Der Übersichtlichkeit wegen ist diese virtuelle Sollpunktgitterstruktur 35 mit ihren virtuellen Sollpunkten 33 und ihrer Lage in Bezug auf das zu behandelnde Organ 3 in Fig. 5 nochmals separat, d.h. ohne die Applikatoren 9 und ohne die von deren Applikatorspitzen 19 erzeugten Lichtkugeln 21, dargestellt.

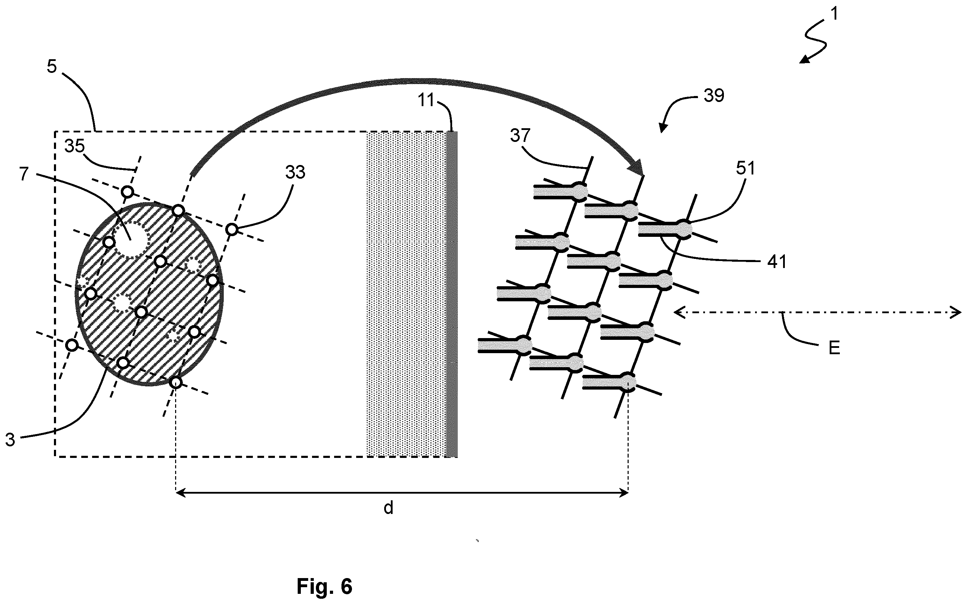

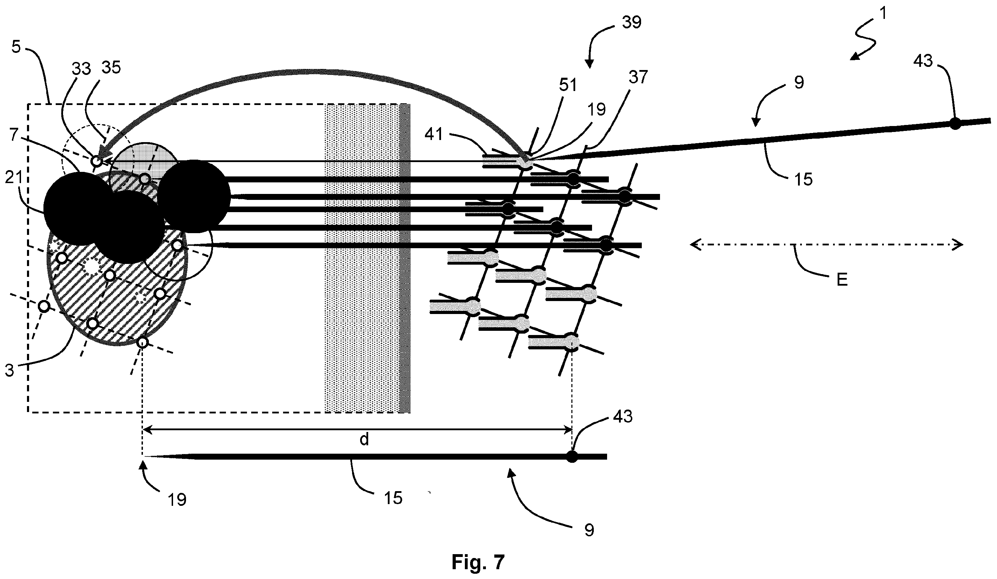

Die Fig. 6 und 7 verdeutlichen nun die erfindungsgemäße Vorgehensweise zur Lösung für das vorausgehend geschilderte Problem, d.h. die zielgenaue Platzierung der lichtemittierenden Applikatorspitzen 19 in den virtuellen und unsichtbaren Sollpunkten 33, die aus zwei Schritten besteht.

In einem ersten Schritt, der in Fig. 6 gezeigt ist, wird die virtuelle und unsichtbare Sollpunktgitterstruktur 35 mit ihren virtuellen und unsichtbaren Sollpunkten 33 aus dem Inneren des Körpers 5 auf eine Fixierpunktgitterstruktur 37 außerhalb des Körpers 5 abgebildet, welche durch eine real existierende und sichtbare Platzierungsschablone 39 mit real existierenden und sichtbaren und damit auch von der Bedienperson mit den lichtemittierenden Applikatorspitzen 19 gezielt und direkt ansteuerbaren Fixierpunktaufnahmen 51 gebildet wird. Bei dieser Abbildung der Sollpunktgitterstruktur 35 auf die Fixierpunktgitterstruktur 37 handelt es sich um eine Parallelverschiebung bzw. Translation um eine definierte Länge d, die durch den Abstand des Organs 3 zum Raum außerhalb des Körpers, wo die Platzierungsschablone 39 positioniert werden soll, definiert ist, parallel zu einer Achse E, deren Richtung durch die Einstichachse der Applikatoren 9 definiert ist (siehe dazu auch Fig. 7). Diese spezifische Form der hier durchgeführten Abbildung, d.h. die Parallelverschiebung der Sollpunktgitterstruktur 35 bzw. der Sollpunkte 33 mit ihren beiden kennzeichnenden Parametern, Verschiebungslänge d und Verschiebungsrichtung E, soll in der Fig. 6 nochmals explizit durch den dicken bogenförmigen Pfeil nach rechts in schematischer Weise verdeutlicht werden. Die Achse E verläuft vorzugsweise aber nicht zwangsweise orthogonal zur Haut 11.

Erst in einem zweiten Schritt, der in Fig. 7 gezeigt ist, erfolgt dann die eigentliche, therapievorbereitende Applikatorplatzierung, indem die lichtemittierenden Applikatorspitzen 19 zunächst zu den real existierenden und sichtbaren und damit gezielt und direkt ansteuerbaren Fixierpunktaufnahmen 51 der Platzierungsschablone 39 geführt werden, um von dort in definierter, eindeutiger und damit sicherer Weise in bzw. zu den virtuellen und für die Bedienperson unsichtbaren Sollpunkten 33 "abgebildet" bzw. bewegt zu werden. Diese im Rahmen der zweistufigen Vorgehensweise durchzuführende zweite Abbildung, also die Parallelverschiebung der lichtemittierenden Applikatorspitzen 19 von außerhalb des Körpers 5 in den Körper 5 hinein, ist nun aber entsprechend der erfindungsgemäßen Vorgehensweise gerade dadurch gekennzeichnet, dass sie zur ersten, in Fig. 6 dargestellten Abbildung, der Parallelverschiebung der Sollpunktgitterstruktur 35 von innerhalb des Körpers 5 nach draußen, invers ist. Konkret heißt das, dass die Applikatorspitzen 19 - ausgehend von den Fixierpunktaufnahmen 51 und damit ausgehend von den Zielpunkten der ersten Abbildung bzw. Parallelverschiebung - um die gleiche definierte Länge d und parallel zur gleichen Achse E, aber genau entgegen der ersten Parallelverschiebung bewegt werden. Diese spezifische Form der zweiten hier durchgeführten Abbildung, d.h. die Parallelverschiebung mit ihren beiden kennzeichnenden Parametern, Verschiebungslänge d und Verschiebungsrichtung E, soll in der Fig. 7 nochmals explizit durch den dicken bogenförmigen Pfeil nach links in schematischer Weise verdeutlich werden.

Dabei wird die Definiertheit, Eindeutigkeit und Sicherheit hinsichtlich der Zielgenauigkeit bei der Platzierung der lichtemittierenden Applikatorspitzen 19 in den Sollpunkten 33 im Organ 3, also die hier entscheidenden Merkmale im Hinblick auf die geforderte lückenlose Bestrahlung des Organs 3, beim Einführen der lichtemittierenden Applikatorspitzen 19 von den Fixierpunktaufnahmen 51 außerhalb des Körpers 5 ausgehend zu den virtuellen Sollpunkten 33 innerhalb des Körpers 5 - trotz der Unsichtbarkeit der Sollpunkte 33 für die Bedienperson und der daraus resultierenden Unmöglichkeit, diese gezielt ansteuern zu können - dadurch erreicht, dass die Freiheitsgrade für die Bedienperson bei der Bewegung der Lichtapplikatoren 9 bzw. bei der Platzierung der lichtemittierenden Applikatorspitzen 19 im Körper 5 auf ein Minimum reduziert werden, d.h. dass es keine andere Bewegungsmöglichkeit gibt als die erforderliche, zur ersten Parallelverschiebung der Sollpunktgitterstruktur 35 exakt inverse Parallelverschiebung der lichtemittierenden Applikatorspitzen 19.

Das Führen der Bewegung der lichtemittierenden Applikatorspitzen 19 ausschließlich gemäß der inversen Parallelverschiebung in den Körper 5 hinein, geschieht erstens dadurch, dass die Platzierungsschablone 39 neben den schon genannten Fixierpunktaufnahmen 51 zusätzlich mit Führungen 41 versehen ist, welche eine definierte Ausrichtung haben, nämlich eine parallele Ausrichtung zur Achse E, wodurch nur eine einzige, nämlich die für die gewünschte inverse Parallelverschiebung definierte Bewegungsrichtung der Lichtapplikatoren 9 mit ihren Applikatorspitzen 19, parallel zur Achse E, zugelassen bzw. möglich wird, und zweitens dadurch, dass die Lichtapplikatoren 9 allesamt mit mindestens einem Fixierpunkt 43 versehen sind, welcher einen definierten Abstand d zur lichtemittierenden Applikatorspitze 19 hat, der nämlich genau der Verschiebungslänge d der Sollpunktgitterstruktur 35 bei der ersten Abbildung bzw. Parallelverschiebung entspricht, und welcher in bzw. an den Fixierpunktaufnahmen 51 der Platzierungsschablone 39 fixierbar ist, wodurch nur eine einzige, nämlich die für die gewünschte inverse Parallelverschiebung definierte Eindringtiefe der zugehörigen lichtemittierenden Applikatorspitze 19 in den Körper 5 und in das Organ 3 zugelassen bzw. möglich wird.

Beides zusammen führt also dazu, dass bei einer Bewegung der Lichtapplikatoren 9 in distale Richtung bis auf Anschlag in der Platzierungsschablone 39 - ausgehend von den sichtbaren und deshalb direkt ansteuerbaren Fixierpunktaufnahmen 51 - die lichtemittierenden Applikatorspitzen 19 auch allesamt automatisch in den Sollpunkten 33 ankommen und damit auch automatisch die gewünschte lückenlose Bestrahlung des Organs 3 gewährleistet ist, vorausgesetzt, dass auch alle Fixierpunktaufnahmen 51 der Schablone 39 mit Lichtapplikatoren 9 belegt und aktiviert sind.

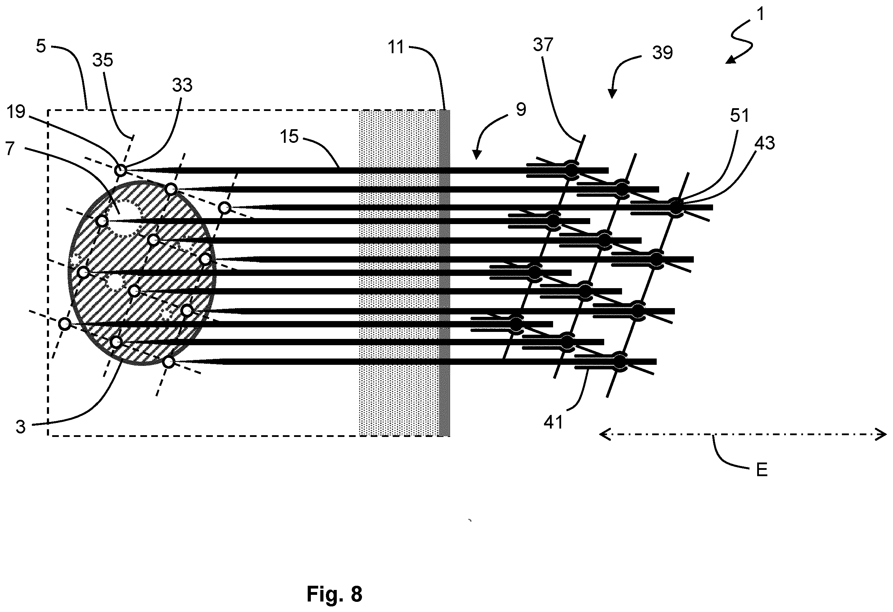

Die Bedienperson muss also nur die Lichtapplikatoren 9 an die Fixierpunktaufnahmen 51 heranführen, dann durch die Führungen 41 hindurchführen und transkutan in das Organ 3 einstechen bis sich der Fixierpunkt 43 am Gitterpunkt der Fixierpunktgitterstruktur 37, d.h. an der Fixierpunktaufnahme 51, befindet. Dann ist auch die Applikatorspitze 19 automatisch am zugehörigen Sollpunkt 33 platziert. Die Bedienperson hat dadurch - im Gegensatz zur Vorgehensweise wie anhand der Fig. 2 und 3 geschildert - keine oder nur wenige Freiheitsgrade und benötigt keine große Erfahrung. Außerdem ist die Platzierung schnell durchführbar mit gerade so vielen Einstichen wie nötig, aber mit so wenig Einstichen wie möglich. Die PDT kann zeitgleich mit sämtlichen platzierten Lichtapplikatoren 9 durchgeführt werden, was eine kurze Behandlungsdauer ermöglicht. Dies ist in Fig. 8 gezeigt. Der besseren Übersichtlichkeit wegen sind dort die Lichtkugeln 21 nicht eingezeichnet, die Fig. 8 zeigt also die Situation unmittelbar vor oder unmittelbar nach der Bestrahlung.

Die erfinderische Vorgehensweise basiert also erstens auf der Durchführung zweier Abbildungen, nämlich einerseits der Abbildung der Sollpunkte 33 auf die Fixierpunktaufnahmen 51 der Platzierungsschablone 39 und andererseits der Abbildung der lichtemittierenden Applikatorspitzen 19 von den Fixierpunktaufnahmen 51 ausgehend zurück auf die Sollpunkte 33, wobei die zweite Abbildung dementsprechend dadurch gekennzeichnet ist, dass sie zur ersten Abbildung invers ist, und zweitens darauf, dass mechanisch-konstruktiv sichergestellt ist, dass die zweite Abbildung, welche der eigentlichen Applikatorplatzierung durch die Bedienperson entspricht, alternativlos ist, d.h. eine von der zweiten Abbildung abweichende Bewegung der Applikatoren 9 bzw. der Applikatorspitzen 19 - von den Fixierpunkten bzw. Fixierpunktaufnahmen 51 ausgehend in den Körper 5 und in das Organ 3 hinein - gar nicht möglich ist.

Mit zunehmender Größe des Organs 3 wächst die Anzahl der erforderlichen Lichtapplikatoren 9 sehr stark an. Beispielsweise bedeutet die Verdopplung des Durchmessers eines kugelförmigen Organs 3, dass achtmal so viele Lichtapplikatoren 9 benötigt werden. Wenn außerdem einerseits die Eindringtiefe des Lichtes vergleichsweise gering ist und andererseits die Ausdehnung des Organs 3 entlang der Einstichachse E vergleichsweise groß ist, dann ist die Dichte der Lichtapplikatoren 9 in den Ebenen senkrecht zur Einstichachse E entsprechend hoch und es kann passieren, dass die Lichtapplikatoren 9 nicht mehr genügend Platz nebeneinander finden. Dies wird bei den Fig. 7 und 8 deutlich, wo die vorausgehend beschriebene Vorgehensweise diesbezüglich schon fast an ihre Grenzen stößt, insbesondere dann, wenn man bei den Lichtapplikatoren 9 auch noch einen handlichen Handgriff berücksichtigt, der in den Fig. 7 und 8 gar nicht eingezeichnet ist.

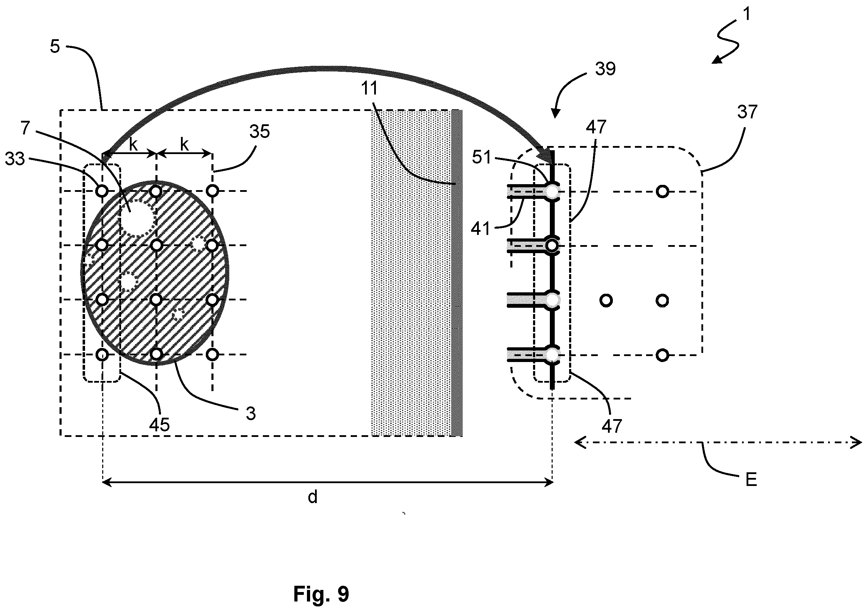

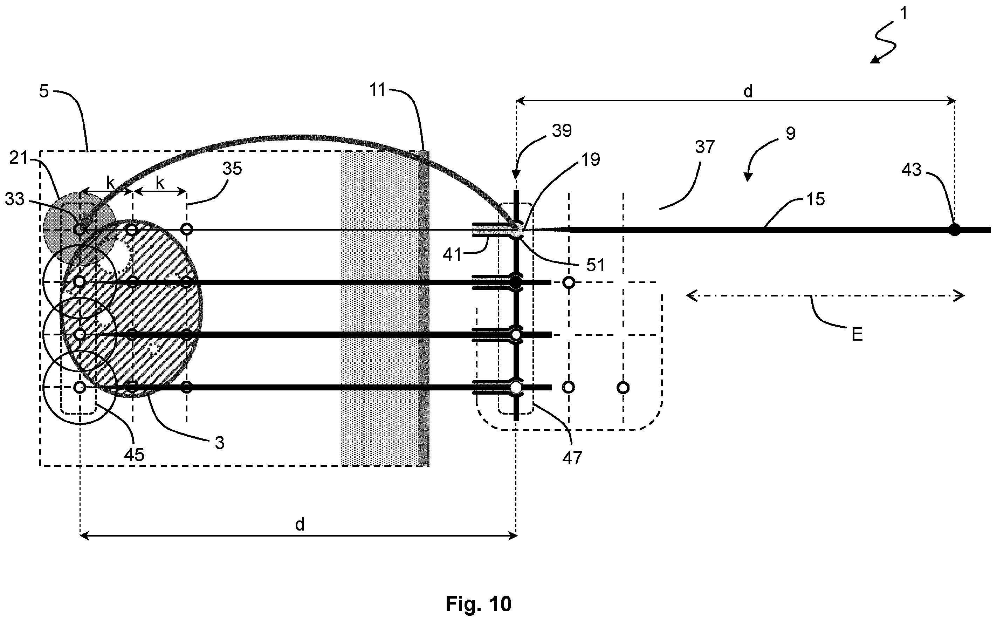

Diesem Problem kann mit der Durchführung einer schrittweisen PDT begegnet werden. In Fig. 9 und 10 ist gezeigt wie die PDT schrittweise in Sollpunktgitterflächen 45 der Sollpunktgitterstruktur 35 durchgeführt werden kann. Dazu werden die Fixierpunkte 43 der Lichtapplikatoren 9 an den Gitterpunkten bzw. den Fixierpunktaufnahmen 51 einer zugehörigen Fixierpunktgitterfläche 47 der Fixierpunktgitterstruktur 37 platziert bzw. fixiert, sodass die Applikatorspitzen 19 an den zugehörigen Sollpunkten 33 der Sollpunktgitterfläche 45 platziert sind. Die PDT wird erst für die erste Sollpunktgitterfläche 45 durchgeführt, die vorzugsweise die am tiefsten unter der Haut liegende Sollpunktgitterfläche 45 ist, also am weitesten distalwärts liegt. Dann können die Lichtapplikatoren 9 um einen Abstand k der Sollpunktgitterflächen 45 proximalwärts zurückgezogen werden, um die PDT für die zweite Sollpunktgitterfläche 45 durchzuführen. Dies wird schrittweise für alle Sollpunktgitterflächen 45 durchgeführt bis das gesamte Volumen des Organs 3 mit Licht bestrahlt wurde. Vorteilhaft bei dieser Vorgehensweise ist, dass weniger Lichtapplikatoren 9 und weniger Einstiche benötigt werden als bei dem in Fig. 8 gezeigten Ausführungsbeispiel. Außerdem können so in der Regel auch alle Lichtapplikatoren 9 eingeführt werden und sie sind sich dabei nicht im Weg. Allerdings ist die Behandlungsdauer länger, da die PDT schrittweise bzw. nacheinander an jeder Sollpunktgitterfläche 45 durchgeführt wird und nicht in einem Schritt bzw, nicht gleichzeitig für das ganze Organ 3.

Außerdem wird in Fig. 9 und 10 noch einmal explizit die erfindungsgemäße zweistufige Vorgehensweise zur Lösung des Problems gezeigt, dass die Bedienperson für eine lückenlose Bestrahlung des Organs 3 einerseits die lichtemittierenden Applikatorspitzen 19 zielgenau in den Sollpunkten 33 platzieren muss, aber andererseits diese Sollpunkte 33 gar nicht direkt zielgerichtet ansteuern kann, weil es sich dabei lediglich um virtuelle und dementsprechend unsichtbare Punkte innerhalb des ohnehin gar nicht einsehbaren Körper- und Organinneren handelt.

In einem ersten Schritt der zweistufigen Vorgehensweise, der in Fig. 9 gezeigt ist, wird eine virtuelle und unsichtbare Sollpunktgitterfläche 45 der virtuellen und unsichtbaren Sollpunktgitterstruktur 35 mit ihren Sollpunkten 33 aus dem Inneren des Körpers 5 auf eine entsprechende Fixierpunktgitterfläche 47 der Fixierpunktgitterstruktur 37 außerhalb des Körpers 5 abgebildet, welche durch eine real existierende und sichtbare Platzierungsschablone 39 mit real existierenden und sichtbaren und damit auch von der Bedienperson mit den lichtemittierenden Applikatorspitzen 19 gezielt und direkt ansteuerbaren Fixierpunktaufnahmen 51 gebildet wird. Bei dieser Abbildung der Sollpunktgitterfläche 45 auf die Fixierpunktgitterfläche 47 handelt es sich, wie schon bei der in Fig. 6 erläuterten Vorgehensweise, um eine Parallelverschiebung bzw. Translation um eine definierte Länge d, die durch den Abstand des Organs 3 zum Raum außerhalb des Körpers 5, wo die Platzierungsschablone 39 positioniert werden soll, definiert ist, parallel zu einer Achse E, deren Richtung durch die Einstichachse der Applikatoren 9 definiert ist (siehe dazu auch Fig. 10). Diese spezifische Form der hier durchgeführten Abbildung, d.h. die Parallelverschiebung der Sollpunktgitterfläche 45 mit ihren beiden kennzeichnenden Parametern, Verschiebungslänge d und Verschiebungsrichtung E, soll in der Fig. 9 nochmals explizit durch den dicken bogenförmigen Pfeil nach rechts in schematischer Weise verdeutlicht werden.

Im zweiten Schritt der zweistufigen Vorgehensweise, der in Fig. 10 gezeigt ist, erfolgt dann die eigentliche, therapievorbereitende Applikatorplatzierung, indem die lichtemittierenden Applikatorspitzen 19 zunächst zu den real existierenden und sichtbaren und damit gezielt und direkt ansteuerbaren Fixierpunktaufnahmen 51 der Fixierpunktgitterfläche 47 der Platzierungsschablone 39 geführt werden, um von dort in definierter, eindeutiger und damit sicherer Weise in bzw. zu den virtuellen und für die Bedienperson unsichtbaren Sollpunkten 33 "abgebildet" bzw. bewegt zu werden. Diese im Rahmen der zweistufigen Vorgehensweise durchzuführende zweite mathematische Abbildung, also die Parallelverschiebung der lichtemittierenden Applikatorspitzen 19 von außerhalb des Körpers 5 in den Körper 5 hinein, ist nun aber entsprechend der erfindungsgemäßen Vorgehensweise gerade dadurch gekennzeichnet, dass sie zur ersten, in Fig. 9 dargestellten mathematischen Abbildung in Form einer Parallelverschiebung der Sollpunktgitterfläche 45 von innerhalb des Körpers 5 nach draußen invers ist. Konkret heißt das, dass die Applikatorspitzen 19 - ausgehend von den Fixierpunktaufnahmen 51 und damit ausgehend von den Zielpunkten der ersten Parallelverschiebung - um die gleiche definierte Länge d und parallel zur gleichen Achse E, aber genau entgegen der ersten Parallelverschiebung bewegt werden. Diese spezifische Form der zweiten hier durchgeführten Abbildung, d.h. die Parallelverschiebung mit ihren beiden kennzeichnenden Parametern, Verschiebungslänge d und Verschiebungsrichtung E, soll in der Fig. 10 nochmals explizit durch den dicken, bogenförmigen Pfeil nach links in schematischer Weise verdeutlich werden.

Dabei wird die Definiertheit, Eindeutigkeit und Sicherheit hinsichtlich der Zielgenauigkeit bei der Platzierung der lichtemittierenden Applikatorspitzen 19 in den Sollpunkten 33 im Organ 3, also die hier entscheidenden Merkmale im Hinblick auf die geforderte lückenlose Bestrahlung des Organs 3, beim Einführen der lichtemittierenden Applikatorspitzen 19 von den Fixierpunktaufnahmen 51 außerhalb des Körpers 5 ausgehend zu den virtuellen Sollpunkten 33 innerhalb des Körpers 5 - trotz der Unsichtbarkeit der Sollpunkte 33 für die Bedienperson und der daraus resultierenden Unmöglichkeit, diese gezielt ansteuern zu können - dadurch erreicht, dass die Freiheitsgrade für die Bedienperson bei der Bewegung der Lichtapplikatoren 9 bzw. bei der Platzierung der lichtemittierenden Applikatorspitzen 19 im Körper 5 auf ein Minimum reduziert werden, d.h. dass die erforderliche, zur ersten Parallelverschiebung der Sollpunktgitterfläche 45 inverse Parallelverschiebung der lichtemittierenden Applikatorspitzen 19 alternativlos gestaltet wird.

Letzteres geschieht erstens dadurch, dass die Platzierungsschablone 39 neben den schon genannten Fixierpunktaufnahmen 51 zusätzlich mit Führungen 41 versehen ist, welche eine definierte Ausrichtung haben, nämlich eine parallele Ausrichtung zur Achse E, wodurch nur eine einzige, nämlich die für die gewünschte inverse Parallelverschiebung definierte Bewegungsrichtung der Lichtapplikatoren 9 mit ihren Applikatorspitzen 19, parallel zur Achse E, zugelassen bzw. möglich wird, und zweitens dadurch, dass die Lichtapplikatoren 9 allesamt mit mindestens einem Fixierpunkt 43 versehen sind, welcher einen definierten Abstand d zur lichtemittierenden Applikatorspitze 19 hat, der nämlich genau der Verschiebungslänge d der Sollpunktgitterfläche 45 bei der ersten Abbildung bzw. Parallelverschiebung entspricht, und welcher in bzw. an den Fixierpunktaufnahmen 51 der Platzierungsschablone 39 fixierbar ist, wodurch nur eine einzige, nämlich die für die gewünschte inverse Parallelverschiebung definierte Eindringtiefe der zugehörigen lichtemittierenden Applikatorspitze 19 in den Körper 5 und in das Organ 3 zugelassen bzw. möglich wird.

Beides zusammen führt also dazu, dass bei einer Bewegung der Lichtapplikatoren 9 in distale Richtung bis auf Anschlag in der Platzierungsschablone 39 - ausgehend von den sichtbaren und deshalb direkt ansteuerbaren Fixierpunktaufnahmen 51 - die lichtemittierenden Applikatorspitzen 19 allesamt automatisch in den Sollpunkten 33 ankommen und damit auch automatisch die gewünschte lückenlose Bestrahlung des Organs 3 gewährleistet ist, vorausgesetzt, dass auch alle Fixierpunktaufnahmen 51 der Schablone 39 mit Lichtapplikatoren 9 belegt und aktiviert sind.

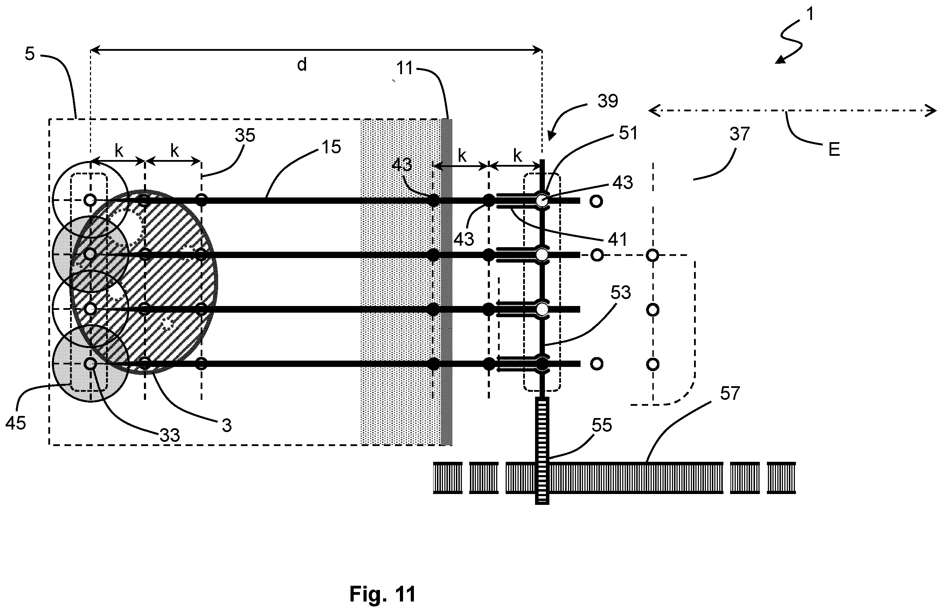

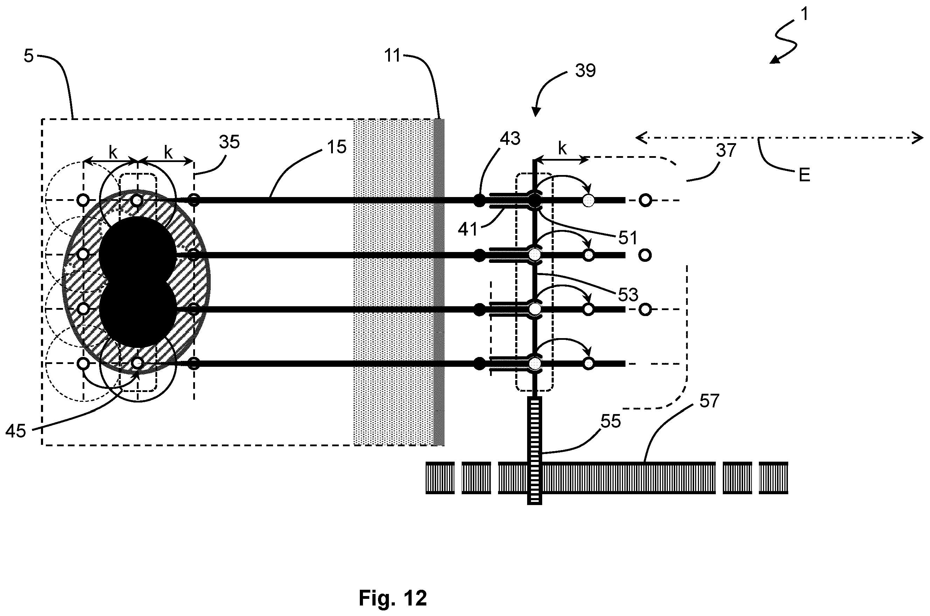

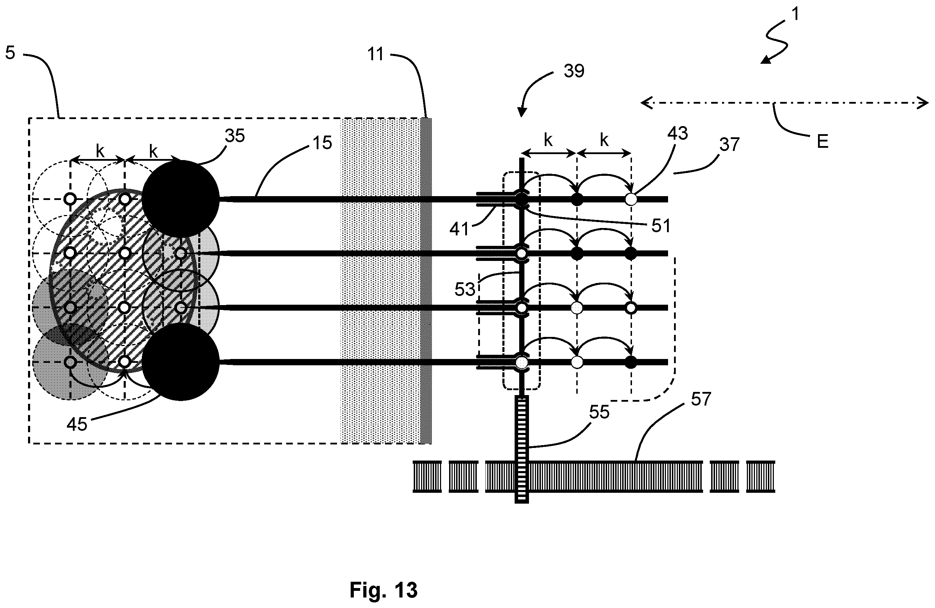

Fig. 11 bis 16 zeigen verschiedene Umsetzungsmöglichkeiten für die schrittweise PDT in Sollpunktgitterflächen 45. In Fig. 11 bis 13 weisen die Lichtapplikatoren 9 jeweils mehrere im Abstand k angeordnete Fixierpunkte 43 auf, sodass die Bedienperson den Fixierpunkt 43 wählen kann, der am entsprechenden Gitterpunkt der Fixierpunktgitterstruktur 37 bzw. an der entsprechenden Fixierpunktaufnahme 51 der Platzierungsschablone 39 platziert wird, um die Sollpunktgitterflächen 45 zu bestimmen, in der die Applikatorspitze 19 liegen soll. Die dreidimensionale Fixierpunktgitterstruktur 37 wird hier also zum einen von ortsfesten Fixierpunktaufnahmen 51 definiert, die auf einer Fixierpunktaufnahmegitterfläche 53 liegen, und zum anderen von den im Abstand k angeordneten Fixierpunkten 43 der Lichtapplikatoren 9, wobei diese dreidimensionale Fixierpunktgitterstruktur 37 bei der hier gezeigten Vorgehensweise schrittweise aufgebaut wird und ihre endgültige Form und Größe erst dann erreicht, nachdem alle Fixerpunkte 43 eines jeden Lichtapplikators 9 die jeweils zugehörige Fixierpunktaufnahme 53 der Platzierungsschablone 39 schrittweise durchlaufen haben, was schließlich im Zustand der Fig. 13 erreicht ist. Die Fixierpunktaufnahmen 51 der Fixierpunktaufnahmegitterfläche 53 sind hier in oder an den Führungen 41 der Platzierungsschablone 39 angeordnet. Die Platzierungsschablone 39 weist eine Befestigungsvorrichtung 55 auf, mit welcher die Platzierungsschablone 39 gegenüber einer festen Referenzfläche 57, beispielsweise einem Behandlungstisch, in einer von der Bedienperson eingestellten Position und/oder Ausrichtung fixierbar ist. Der Körper 5 des Patienten ist dabei ebenfalls relativ zu der festen Referenzfläche 57, beispielsweise einem Behandlungstisch, fixiert. Nach der PDT mit den Applikatorspitzen 19 in der ersten Sollpunktgitterfläche 45 gemäß Fig. 11 wird in Fig. 12 das Organ 3 mit den Applikatorspitzen 19 in der zweiten Sollpunktgitterfläche 45 bestrahlt. In Fig. 13 wird sodann das Organ 3 mit den Applikatorspitzen 19 in der dritten und letzten Sollpunktgitterfläche 45 bestrahlt.

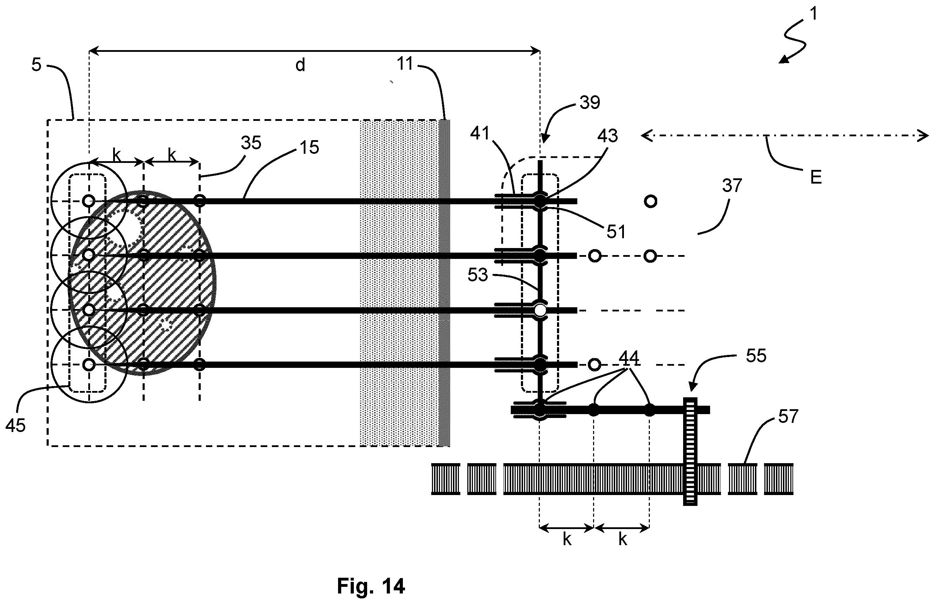

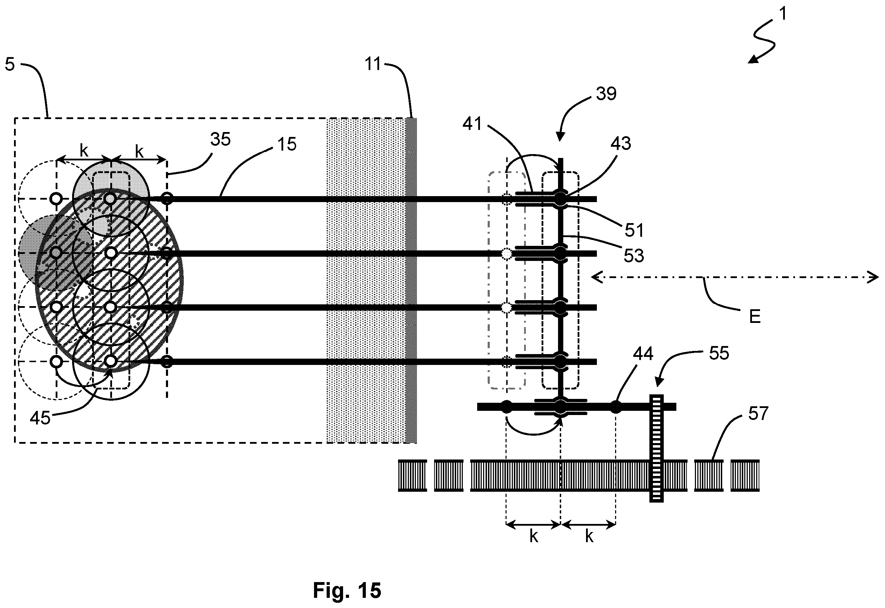

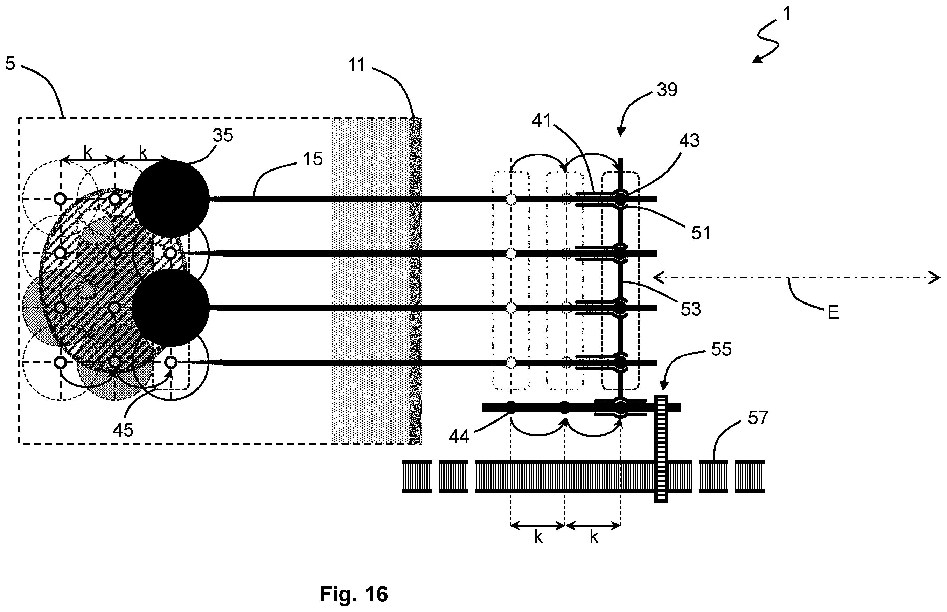

In Fig. 14 weisen die Lichtapplikatoren 9 jeweils nicht mehrere im Abstand k angeordnete Fixierpunkte 43 auf, sondern die Platzierungsschablone 39 ist als ganze gegenüber der festen Referenzfläche 57 um den Abstand k definiert verschiebbar. Dazu definiert die Platzierungsschablone 39 mittels der Befestigungsvorrichtung 55 mehrere im Abstand k angeordnete Fixierpunkte 44, um die Fixierpunktaufnahmegitterfläche 53 als ganze um den Abstand k definiert verschieben zu können. Dies hat den Vorteil, dass die Lichtapplikatoren 9 beim Wechsel zur nächsten Sollpunktgitterfläche 45 nicht einzeln bewegt werden müssen, sondern zusammen konzertiert bewegt werden. Dies beschleunigt und vereinfacht den Behandlungsprozess. Nach der PDT mit den Applikatorspitzen 19 in der ersten Sollpunktgitterfläche 45 gemäß Fig. 14 wird in Fig. 15 das Organ 3 mit den Applikatorspitzen 19 in der zweiten Sollpunktgitterfläche 45 bestrahlt. In Fig. 16 wird sodann das Organ 3 mit den Applikatorspitzen 19 in der dritten und letzten Sollpunktgitterfläche 45 bestrahlt. Der Übersichtlichkeit wegen ist in den Fig. 15 und 16 die Fixierpunktgitterstruktur 37 nicht mehr eingezeichnet.

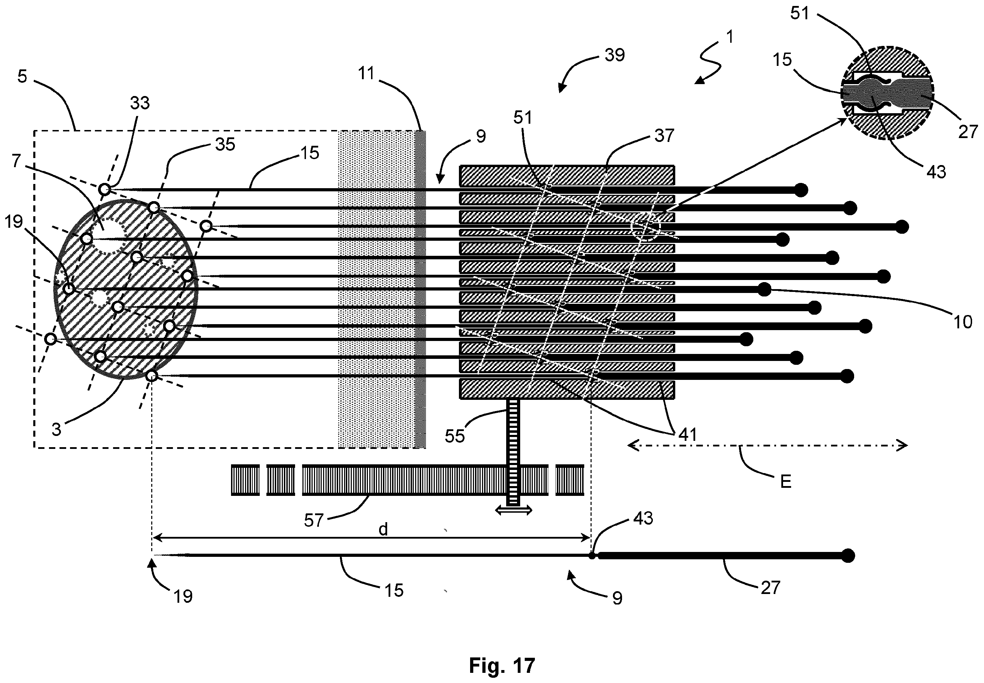

Fig. 17 zeigt ein Ausführungsbeispiel für das in Fig. 8 gezeigte Prinzip. Die Platzierungsschablone 39 ist hier ein Block oder anders geformter Körper mit einer gewissen Dicke entlang der Einstichachse E, durch welchen eine Vielzahl von parallel zueinander angeordneten Führungen 41 entlang der Einstichachse E verläuft. Jede Führung 41 weist eine Fixierpunktaufnahme 51 auf, welche einen Gitterpunkt der Fixierpunktgitterstruktur 37 bildet. Die so gebildete dreidimensionale Fixierpunktgitterstruktur 37 ist identisch mit der organspezifischen virtuellen Sollpunktgitterstruktur 35 für die Applikatorspitzen 19 und geht aus jener durch eine Parallelverschiebung bzw. Translation um die Länge d hervor. Jeder Lichtapplikator 9 weist einen dünnen Einführabschnitt 15 auf und einen dickeren proximalen Abschnitt, der als Griffelement 27 sowie der Führung in den Führungen 41 dient. Der Fixierpunkt 43 wird hier durch einen kugelförmigen Anschlag gebildet, der am proximalen Ende des Einführabschnitts 15 angeordnet ist. Die Führungen 41 sind entsprechend dem Durchmesser des Einführabschnitts 15 und des dickeren proximalen Abschnitts der Lichtapplikatoren 9 angepasst dimensioniert. Die Lichtapplikatoren 9 sind vorzugsweise alle identisch, um das Risiko einer fehlerhaften Auswahl eines "falschen" Lichtapplikators 9 und daraus resultierend eine Fehlpositionierung der lichtemittierenden Applikatorspitze 19 im Organ 3 zu vermeiden. Die Bedienperson hat in diesem Ausführungsbeispiel keine Freiheitsgrade, sondern kann die Lichtapplikatoren 9 nur auf eine Art und Weise einstechen bis die Fixierpunkte 43 in den Fixierpunktaufnahmen 51 einrasten. Dazu weisen die Fixierpunktaufnahmen 51 hier Maulklammern oder ein anderweitiges Klemmmittel auf. Dies gibt der Bedienperson ein haptisches Feedback zur korrekten Platzierung und verhindert ein ungewolltes proximalwärtiges Verrutschen der Lichtapplikatoren 9. Die Platzierungsschablone 39 ist entlang der Einstichachse E mittels ihrer Befestigungsvorrichtung 55 geführt bewegbar und relativ zur Referenzfläche 57 fixierbar. Die diesbezügliche Position der Platzierungsschablone 39 kann vorzugsweise wie folgt bestimmt werden. Die Platzierungsschablone 39 wird mit der Befestigungsvorrichtung 55 an der Referenzfläche 57 zunächst ganz nahe am Körper 5 bzw. an der Haut 11 platziert und fixiert. Anschließend wird vorzugsweise ein bzgl. einer Ebene senkrecht zur Einstichachse E zentral und bzgl. der Einstichachse E selbst distal zu positionierender, erster Lichtapplikator 10 eingestochen und - mit einem bildgebenden Verfahren (beispielsweise Ultraschall-Sonografie) kontrolliert - so weit vorgeschoben, bis seine Applikatorspitze 19 den distalen Bereich des Organs 3 erreicht hat, wie z.B. in Fig. 17 dargestellt. Dann wird - unter Aufrechterhaltung dieser erreichten Position des ersten Lichtapplikators 10 bzw. dessen Applikatorspitze 19 - die Platzierungsschablone 39 mittels der Befestigungsvorrichtung 55 an der Referenzfläche 57 axial, d.h. entlang der Einstichachse E, soweit in proximale Richtung von der Haut 11 weg zurückgezogen, bis der Fixierpunkt 43 in der Fixierpunktaufnahme 51 fixiert werden kann, d.h. im hier gezeigten Fall in der Maulklammer einrastet. In dieser Position wird dann die Platzierungsschablone 39 mittels der Befestigungsvorrichtung 55 an der Referenzfläche 57 fixiert.

Mit diesem Vorgang wird der definierte Abstand d zwischen der virtuellen Sollpunktgitterstruktur 35 und Fixierpunktgitterstruktur 37 hergestellt. Dieser Vorgang vollendet damit den ersten Schritt der erfindungsgemäßen Vorgehensweise, nämlich die erste Abbildung, d.h. die Parallelverschiebung bzw. Translation der virtuellen Sollpunktgitterstruktur 35 im Organ 3 innerhalb des Körpers 5 in einen Bereich außerhalb des Körpers 5, und schafft so die Voraussetzung für die die eigentliche Therapievorbereitung, nämlich die korrekte Platzierung der restlichen Lichtapplikatoren 9 bzw. deren lichtemittierender Applikatorspitzen 19 im Organ 3.

Die restlichen Lichtapplikatoren 9 müssen dann nur noch durch die verbleibenden Führungen 41 der Platzierungsschablone 39 hindurchgeführt und mit ihren Fixierpunkten 43 an der jeweiligen Fixierpunktaufnahme 51 fixiert werden. Die Freiheitsgrade der Bedienperson sind dabei minimiert, was wiederum bedeutet, dass die Positionen der restlichen Lichtapplikatoren 9 und in der Folge die Positionen ihrer lichtemittierenden Applikatorspitzen 19 nach der Fixierung der Platzierungsschablone 39 eindeutig festgelegt sind und dadurch automatisch mit den Sollpunkten 33 übereinstimmen.

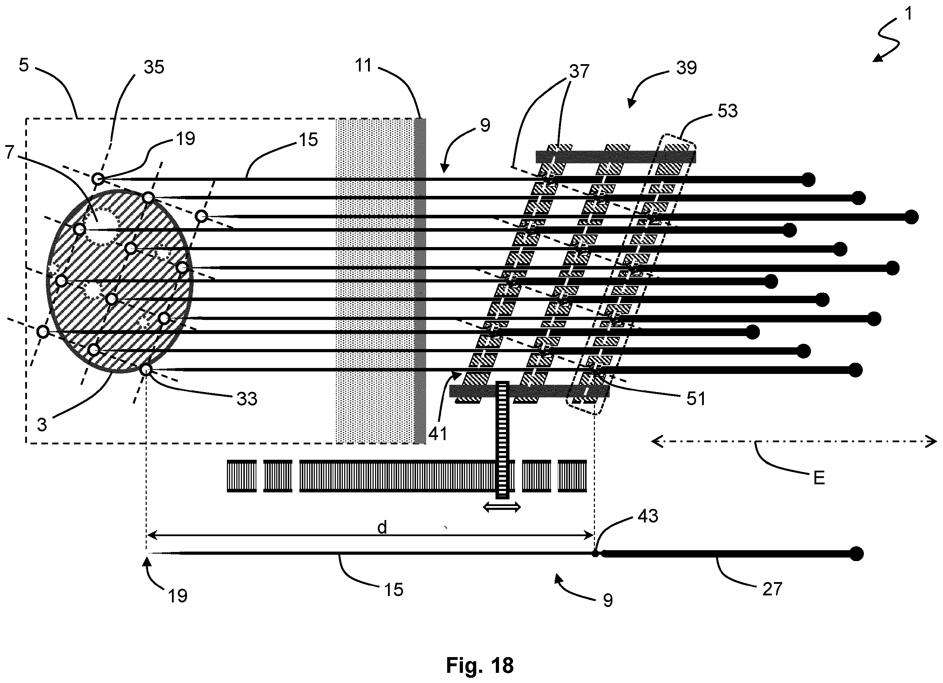

Fig. 18 zeigt eine alternative Ausführungsform, bei der die Platzierungsschablone 39 aus mehreren fest zueinander positionierten Platten besteht, die den Block aus Fig. 18 ersetzen. Dadurch wird die Platzierungsschablone 39 leichter, und es wird nicht benötigtes Material für die Platzierungsschablone 39 gespart. Die Platten weisen vorzugsweise jeweils die Fixierpunktaufnahmen 51 einer Fixierpunktaufnahmegitterfläche 53 auf. Die Platten sind vorzugsweise parallel zueinander angeordnet. Sie können orthogonal zur Einstichachse E verlaufen oder, wie in Fig. 18, in einem Winkel dazu. Auch hier entsprechen die Fixierpunktaufnahmen 51 wieder den Gitterpunkten der Fixierpunktgitterstruktur 37, welche hinsichtlich Form und Größe identisch ist mit der organspezifischen virtuellen Sollpunktgitterstruktur35 für die Applikatorspitzen 19 und welche aus jener durch eine Parallelverschiebung bzw. Translation um die Länge d hervorgeht.

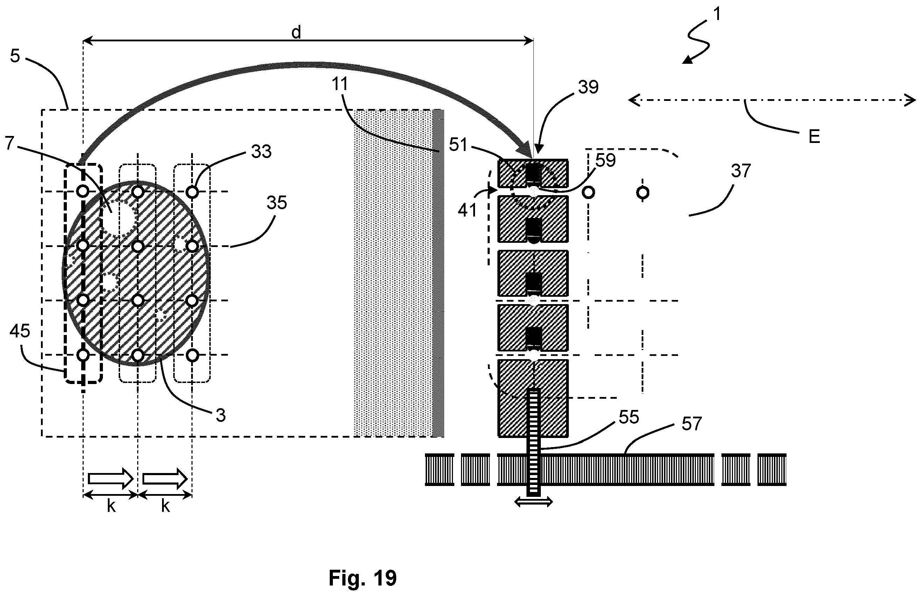

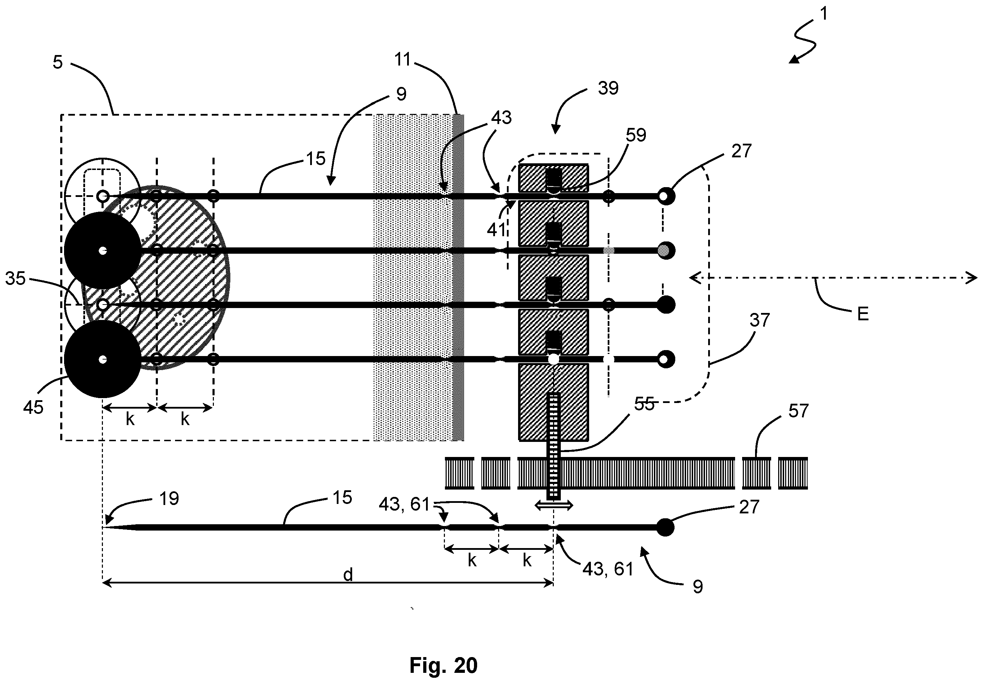

Fig. 19 bis 21 zeigen ein Ausführungsbeispiel für das in Fig. 11 bis 13 gezeigte Prinzip für die schrittweise PDT in Sollpunktgitterflächen 45, d.h. dass hier die Lichtapplikatoren 9 und mit ihnen die lichtemittierenden Applikatorspitzen 19 schrittweise entlang der Einstichachse E über mehrere im Abstand k angeordnete Fixierpunkte 43 verschoben werden, während die Platzierungsschablone 39 über die Befestigungsvorrichtung 55 in unveränderter Position gegenüber der Referenzfläche 57 fixiert bleibt. Die Fixierpunktaufnahmen 51 sind hier durch jeweils federnd in die Führungen 41 lateral eingreifende Eingriffselemente 59 gebildet. Die Fixierpunkte 43 sind entsprechend durch laterale Eingriffsaufnahmen 61 in Form von Verjüngungen am Lichtapplikator 9 gebildet. Die Fixierpunkte 43 haben entlang der Einstichachse E einen Abstand k, der dem Abstand k der Sollpunktgitterflächen 45 der Sollpunktgitterstruktur 35 entspricht.

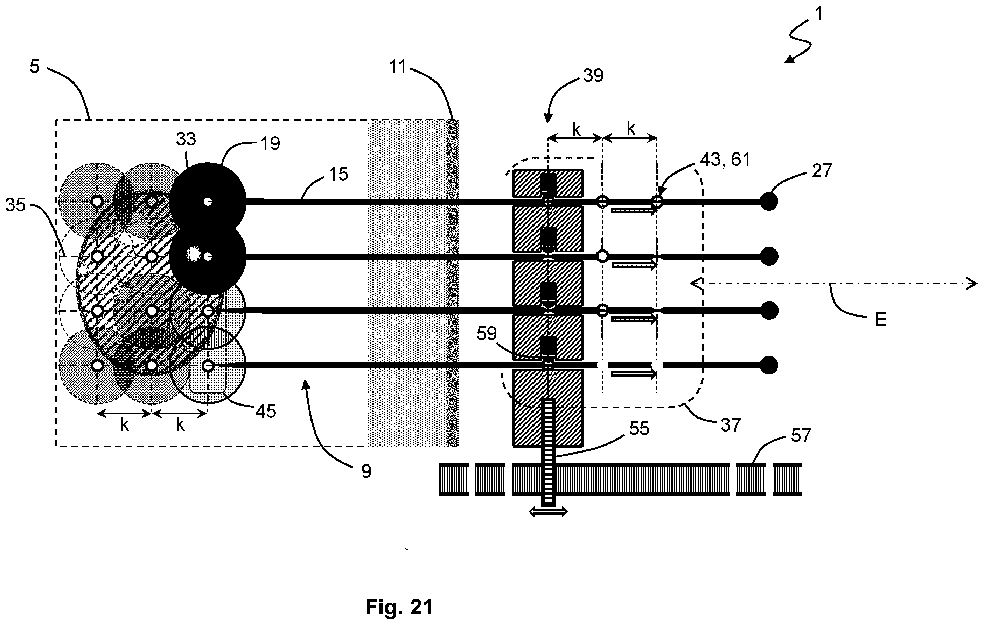

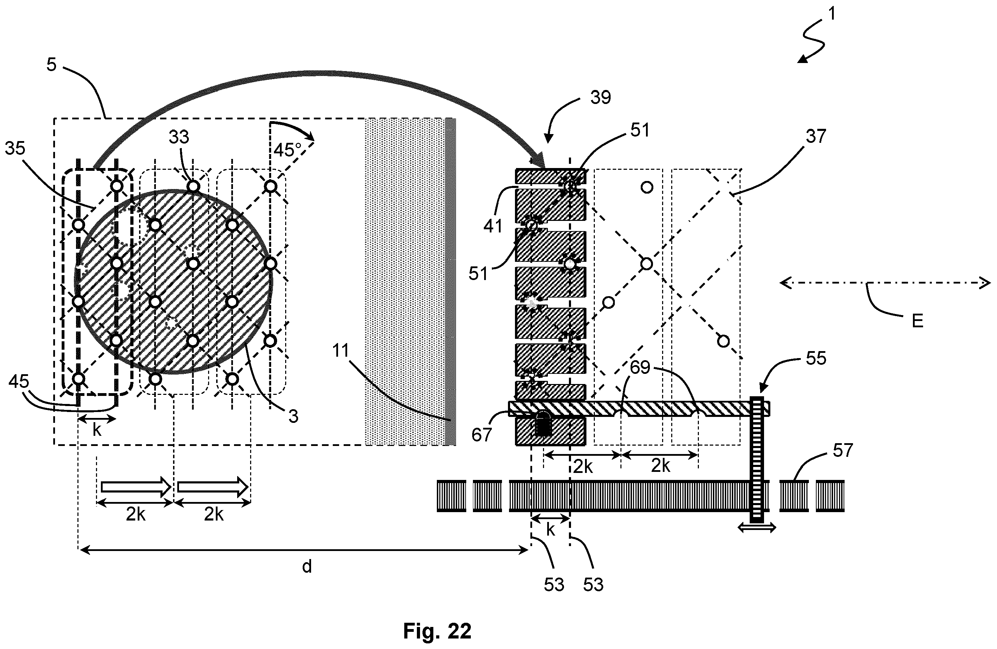

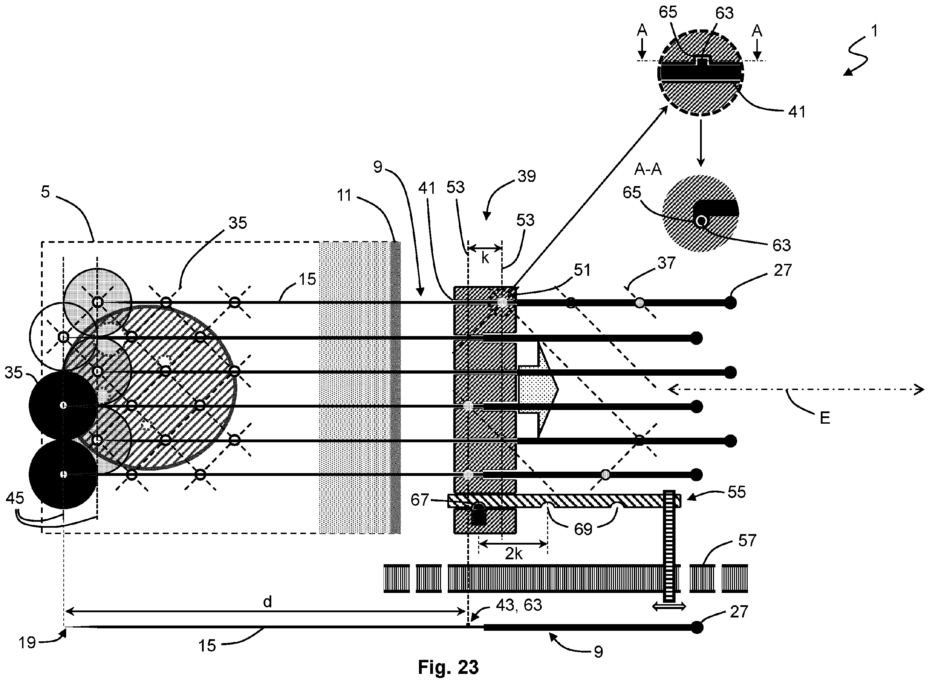

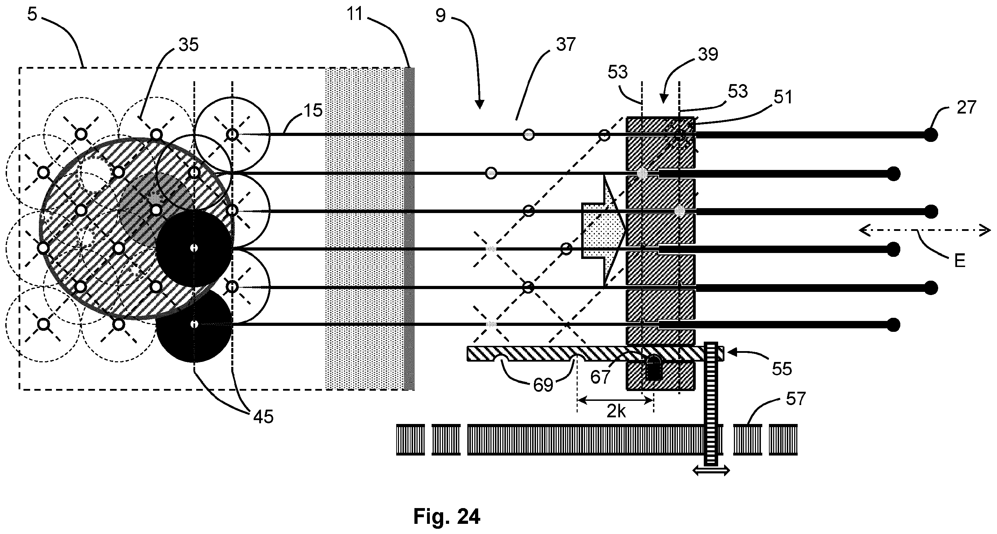

Fig. 22 bis 24 zeigen ein Ausführungsbeispiel, für das in Fig. 14 bis 16 gezeigte Prinzip für die schrittweise PDT in Sollpunktgitterflächen 45. Das heißt, dass hier die Platzierungsschablone 39 nicht wie im vorausgehenden Ausführungsbeispiel an unveränderter Position gegenüber der festen Referenzfläche 57 fixiert bleibt und lediglich die Lichtapplikatoren 9 schrittweise verschoben werden, sondern dass die Platzierungsschablone 39 als ganze gegenüber der festen Referenzfläche 57 um definierte Abstände schrittweise verschoben wird und dabei die eingeführten Lichtapplikatoren 9 mit ihren lichtemittierenden Applikatorspitzen 19 mitgenommen werden und damit im Gegensatz zum vorausgehend beschriebenen Fall alle Lichtapplikatoren 9 gemeinsam bzw. gleichzeitig durch eine einzige Aktion der Bedienperson bewegt werden. Gleichzeitig integriert dieses Ausführungsbeispiel das in Fig. 8 gezeigte Prinzip, dass nämlich in einer Bestrahlungseinheit alle Sollpunkte aus mehreren Sollpunktgitterflächen 45 gleichzeitig, in der vorliegenden Ausführungsform konkret aus zwei Sollpunktgitterflächen 45 gleichzeitig, abgedeckt werden. Dieses Prinzip, mehrere Sollpunktgitterflächen 45 gleichzeitig zu behandeln, gewinnt vor allen Dingen dann an Bedeutung, wenn die Ausdehnung des Organs 3 - vor allen Dingen auch entlang der Einstichachse E - vergleichsweise groß ist, weil dadurch in der gleichen Zeit bzw. mit der gleichen Anzahl an sequenziellen Bestrahlungseinheiten (in den vorliegenden Beispielen drei Bestrahlungseinheiten) ein deutlich größeres Volumen behandelt werden kann (vergleiche dazu Fig. 19 mit Fig. 22). Die Platzierungsschablone 39 weist dazu Fixierpunktaufnahmen 51 von zwei benachbarten Fixierpunktaufnahmegitterflächen 53 auf. Die Platzierungsschablone 39 ist dann im Vergleich mit der vorausgehend beschriebenen Ausführungsform um die doppelte Länge, nämlich um 2k verschiebbar, um die PDT für die folgenden zwei Sollpunktgitterflächen 45 durchzuführen. Mit dieser Vorgehensweise, d.h. dass in einer Bestrahlungseinheit alle Sollpunkte aus mehreren Sollpunktgitterflächen 45 gleichzeitig abgedeckt werden, können im Vergleich mit der vorausgehend beschriebenen Ausführungsform (Fig. 19 bis 21) in der gleichen Zeit, d.h. mit der gleichen Anzahl an Bestrahlungseinheiten, größere d.h. auch stärker entlang der Einstichachse E ausgedehnte Organe 3 behandelt werden (vergleiche dazu Fig. 21 mit Fig. 24). Die Fixierpunkte 43 und die Fixierpunktaufnahmen 51 bilden in der vorliegenden Ausführungsform eine Art Bajonettverschluss, bei dem ein lateraler Vorsprung 63 am Lichtapplikator 9 durch Drehung des Lichtapplikators 9 um die Einstichachse E in eine entsprechende laterale Aufnahmesicherung 65 in der Führung 41 drehbar ist. Die Befestigungsvorrichtung 55 der Platzierungsschablone 39 weist ein Eingriffselement 67 auf, das in fest bezüglich der Referenzfläche 57 jeweils im Abstand 2k entlang der Einstichachse E angeordnete Eingriffsaufnahmen 69 eingreift.

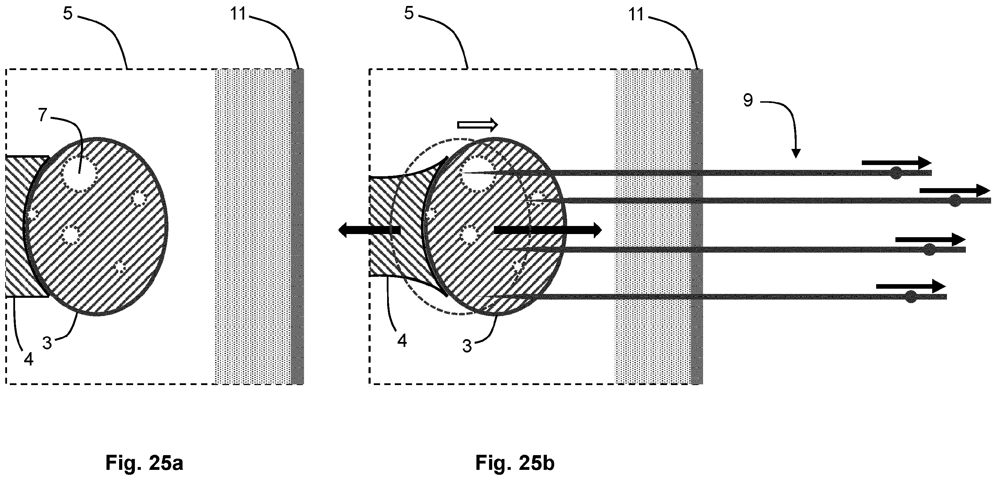

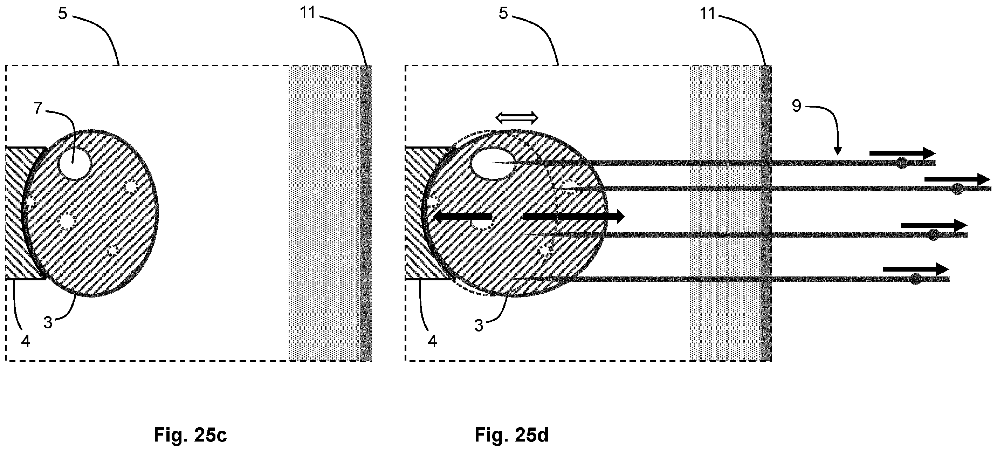

In Fig. 25a,b und 25c,d werden die beiden prinzipiellen Varianten des zu vermeidenden "Aufgabeleffekts" schematisch verdeutlicht, die jeweils für sich oder in Kombination bei der transkutanen PDT auftauchen können. Fig. 25a zeigt die normale Position und die ursprüngliche Form des Organs 3 im Körper 5 des Patienten ohne eingestochene Lichtapplikatoren 9. Werden die im Rahmen der Durchführung der Therapie dann eingestochenen Lichtapplikatoren 9 gemeinsam bzw. gleichzeitig proximalwärts bewegt bzw. zurückgezogen und ist dabei die von den Lichtapplikatoren 9 auf das Organ 3 ausgeübte und wirksame Gesamthaftreibungskraft größer als die Bindungskräfte, die innerhalb des umgebenden mit dem Organ 3 direkt verbundenen Gewebes 4 wirken und wiederum kleiner als die innerhalb des Organs 3 selbst wirksamen Bindungskräfte sind, dann kommt es zu einer "Aufgabelung" des Organs 3 durch die Lichtapplikatoren 9 in einer solchen Form, dass die Lichtapplikatoren 9 das Organ 3 als Ganzes und unter Aufrechterhaltung seiner Form innerhalb des Körpers 5 mitnehmen bzw. mitziehen. Das heißt, dass zwar die Form des Organs 3 erhalten bleibt, dass sich aber seine Position im Körper 5 verändert, wie das in Fig. 25b schematisch dargestellt ist. Hinsichtlich der ursprünglich definierten und geplanten Parallelverschiebung zwischen Sollpunktgitterstruktur und Fixierpunktgitterstruktur heißt dies aber, dass zwar die Form der Sollpunktgitterstruktur erhalten bleibt, sich aber ihre Lage bzw. ihr ursprünglich definierter Abstand d zur Fixierpunktgitterstruktur in undefinierter und damit unerwünschter Weise ändert und damit der die Parallelverschiebung kennzeichnende definierte Abstand d zwischen Sollpunktgitterstruktur und Fixierpunktgitterstruktur nicht mehr aufrechterhalten wird.

Fig. 25c zeigt noch einmal die normale Position und ursprüngliche Form des Organs 3 im Körper 5 des Patienten ohne eingestochene Lichtapplikatoren 9. Werden die im Rahmen der Therapie eingestochenen Lichtapplikatoren 9 gemeinsam bzw. gleichzeitig proximalwärts zurückgezogen und ist dabei die von den Lichtapplikatoren 9 auf das Organ 3 ausgeübte und wirksame Gesamthaftreibungskraft nun größer als die innerhalb des Organs 3 selbst wirksamen Bindungskräfte, die zudem kleiner als die Bindungskräfte sind, die innerhalb des umgebenden, mit dem Organ 3 direkt verbundenen Gewebes 4 wirken, wie in Fig. 25d gezeigt, dann kommt es zu einer "Aufgabelung" des Organs 3 durch die Lichtapplikatoren 9 in einer solchen Form, dass die Lichtapplikatoren 9 das Organ 3 auseinanderziehen bzw. strecken. Das heißt, dass zwar die Position des Organs 3 im Körper 5 weitgehend erhalten bleibt, dass sich aber seine Form verändert, wie das in Fig. 25d schematisch dargestellt ist. Hinsichtlich der ursprünglich definierten und geplanten Parallelverschiebung zwischen Sollpunktgitterstruktur und Fixierpunktgitterstruktur heißt dies aber, dass zwar die Lage der Sollpunktgitterstruktur bzw. ihr ursprünglich definierter Abstand d zur Fixierpunktgitterstruktur in erster Näherung erhalten bleibt, sich aber ihre Form in undefinierter und damit in unerwünschter Weise ändert.

Beide vorausgehend beschriebenen Formen der "Aufgabelung" bzw. deren Auswirkungen auf Form und Position bzw. Lage des Organs 3 im Körper 5 gewinnen mit zunehmender Anzahl der eingesetzten Lichtapplikatoren 9 an Bedeutung und sollten vorzugsweise deshalb bei der Therapieplanung in angemessener Weise berücksichtigt werden, da die auf das Organ 3 ausgeübte (Gesamt-)Haftreibung in entsprechender Weise zunimmt, und dadurch in gleicher Weise auch die Effekte der Positions- bzw. Lageveränderung des Organs 3 (siehe Fig. 25b) und/oder der Formveränderung des Organs 3 (siehe Fig. 25d) zunehmen.

Insbesondere bei der schrittweisen PDT in Sollpunktgitterflächen 45, bei der die Platzierungsschablone 39 als ganze gegenüber der festen Referenzfläche 57 um definierte Abstände schrittweise verschoben wird und dabei die eingeführten Lichtapplikatoren 9 mit ihren lichtemittierenden Applikatorspitzen 19 gleichzeitig bzw. gemeinsam bewegt werden, wie das anhand der Fig. 11 bis 13 in schematischer Form erläutert und anhand der Fig. 22 bis 24 an einer konkreten Ausführungsform beispielhaft gezeigt wurde, ist dies problematisch, da nicht klar ist, wo sich das Organ 3 befindet bzw. wie stark sich das Organ 3 verformt hat, wenn die Lichtapplikatoren 9 für die PDT in der nächsten Sollpunktgitterfläche 45 zurückgezogen werden. Wenn zum Beispiel im Aufbau der Fig. 22 die beiden Fixierpunktaufnahmegitterflächen 53 um den Abstand 2k von der distalen Position in die benachbarte, proximalwärts gelegene Position verschoben werden, was durch ein Verschieben der Platzierungsschablone 39 von der distalen Eingriffsaufnahme 69 in die direkt benachbarte, proximalwärts gelegene Eingriffsaufnahme 69 geschieht (in der Fig. 22 die mittig gelegene Eingriffsaufnahme 69), dann werden zwar auch die Applikatorspitzen 19 um den Abstand 2k in proximale Richtung verschoben. Weil aber, entgegen der idealisierten Darstellung in der Fig. 24, in Wirklichkeit das Organ 3 aufgrund des "Aufgabeleffekts" in unerwünschter Weise gleichfalls eine gewisse, vor allen Dingen aber undefinierte Strecke in proximale Richtung "mitgenommen" wurde, wie in Fig. 25a,b schematisch dargestellt und/oder um eine undefinierte Länge gestreckt bzw. gedehnt wurde, wie in Fig. 25c,d schematisch dargestellt, sind nun auch die organbezogenen Sollpunktgitterflächen 45 nicht mehr in ihrer ursprünglichen Position, wie in Fig. 24 in idealisierter Form eingezeichnet, sondern zusammen mit dem in Wirklichkeit verschobenen und/oder gestreckten Organ 3 in proximale Richtung verschoben und können dadurch von den "lediglich" um den Abstand 2k verschobenen Applikatorspitzen 19 nicht mehr wie ursprünglich vorgesehen erreicht werden. Das Ziel, durch ein sequenzielles Verschieben und Einrasten der Schablone 39 in die gegebenen Eingriffsaufnahmen 69 in für die Bedienperson einfacher und bequemer Weise eine effektive und vor allen Dingen homogene Bestrahlung des Organs 3 auf der Basis der ursprünglichen Sollpunktgitterstruktur 35 zu realisieren, kann unter diesen Umständen, dass also das Organ 3 in unerwünschter und vor allen Dingen undefinierter Weise durch die auf das Organ 3 wirksame Gesamthaftreibungskraft der gemeinsam bzw. gleichzeitig bewegten Lichtapplikatoren 9 mitbewegt und/oder gestreckt wird, nicht mehr erreicht werden.

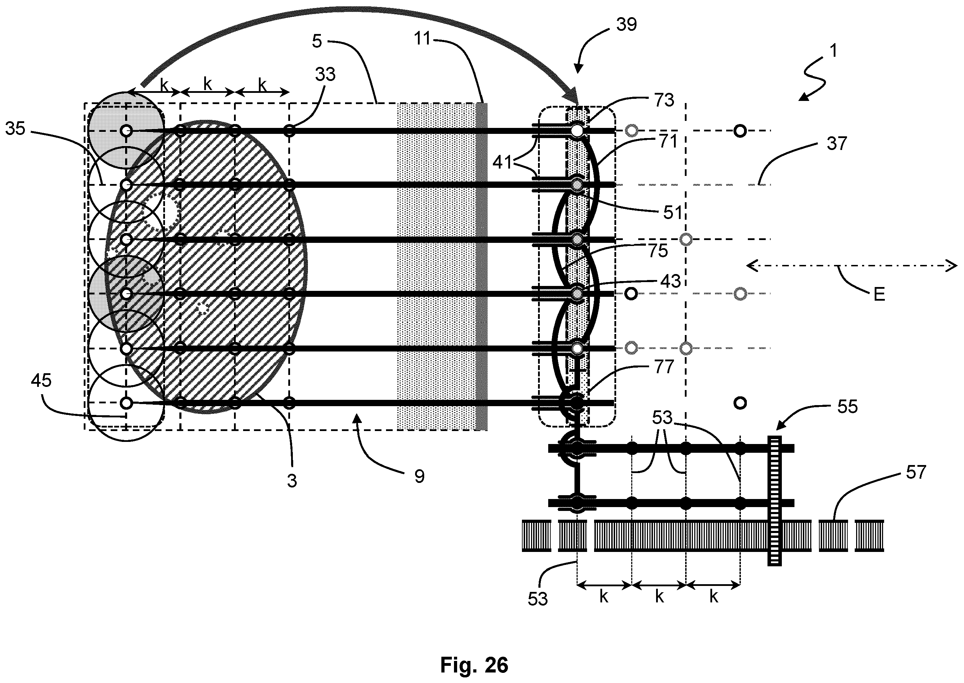

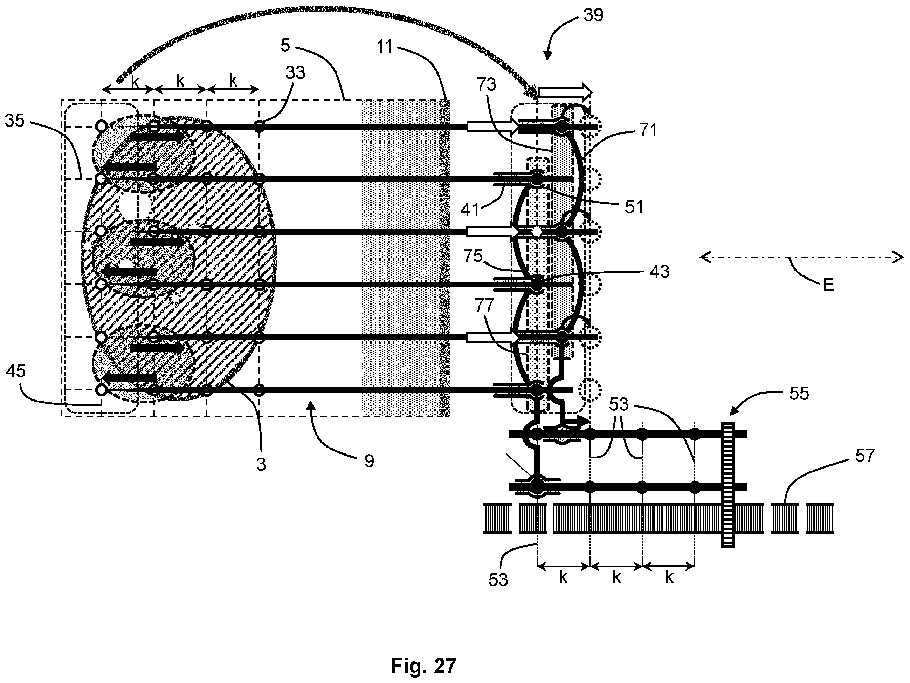

Fig. 26 bis 28 zeigen ein Lösungsprinzip für dieses Problem. Die Platzierungsschablone 39 weist hier nämlich mindestens zwei Teile auf, nämlich einen ersten Schablonenteil 71, der eine erste Untergruppe 73 der Führungen 41 mit Fixierpunktaufnahmen 51 definiert, und einen zweiten Schablonenteil 75, der eine zweite Untergruppe 77 der Führungen 41 mit Fixierpunktaufnahmen 51 definiert. Der erste Schablonenteil 71 ist dabei relativ zum zweiten Schablonenteil 75 entlang der Einstichachse E geführt verschiebbar. Mittels der Schablonenteile 71, 75 können verschiedene Untergruppen von Lichtapplikatoren 9 durch die entsprechenden Untergruppen 73, 77 von Führungen 41 mit Fixierpunktaufnahmen 51 gemeinsam konzertiert wahlweise bewegt oder festgehalten werden. Dies hat den Vorteil, dass mit einer Untergruppe von Lichtapplikatoren 9 das Organ 3 festgehalten werden kann, während die andere Untergruppe bewegt wird. Der "Aufgabeleffekt" kann also dadurch vermieden oder zumindest verringert werden, dass eine Untergruppe von Lichtapplikatoren 9 festgehalten wird, während die andere herausgezogen wird. Die jeweiligen Untergruppen können abwechselnd bewegt werden, während die andere festgehalten wird. Die gemeinsame Bewegung und das gemeinsame Festhalten der Lichtapplikatoren 9 kann durch entsprechende Bewegung bzw. Festhalten der Schablonenteile 71, 75 bewirkt werden. In Fig. 27 ist gezeigt, wie der erste Schablonenteil 71 mit der ersten Untergruppe von Lichtapplikatoren 9 bewegt wird während der zweite Schablonenteil 75 festgehalten wird. Die Summe der Haftreibungskräfte, welche sowohl die bewegten Lichtapplikatoren 9 der ersten Untergruppe 73 als auch die festgehaltenen Lichtapplikatoren 9 der zweiten Untergruppe 77 auf das Organ 3 ausüben, ergibt in erster Näherung Null, was in Fig. 27 in schematischer Weise durch die Paarung von Kraftpfeilen innerhalb der gestrichelten, grauschattierten Ellipsen im Organ 3, die gleichlang, aber in gegensätzliche Richtung zeigen, verdeutlicht ist. Dadurch wird es mit dieser Vorgehensweise möglich, eine Vielzahl von Lichtapplikatoren 9 gemeinsam bzw. gleichzeitig in eine gemeinsame Richtung, hier in proximale Richtung, zu bewegen, ohne dabei das Organ 3 mitzunehmen und/oder ohne das Organ 3 zu verformen.

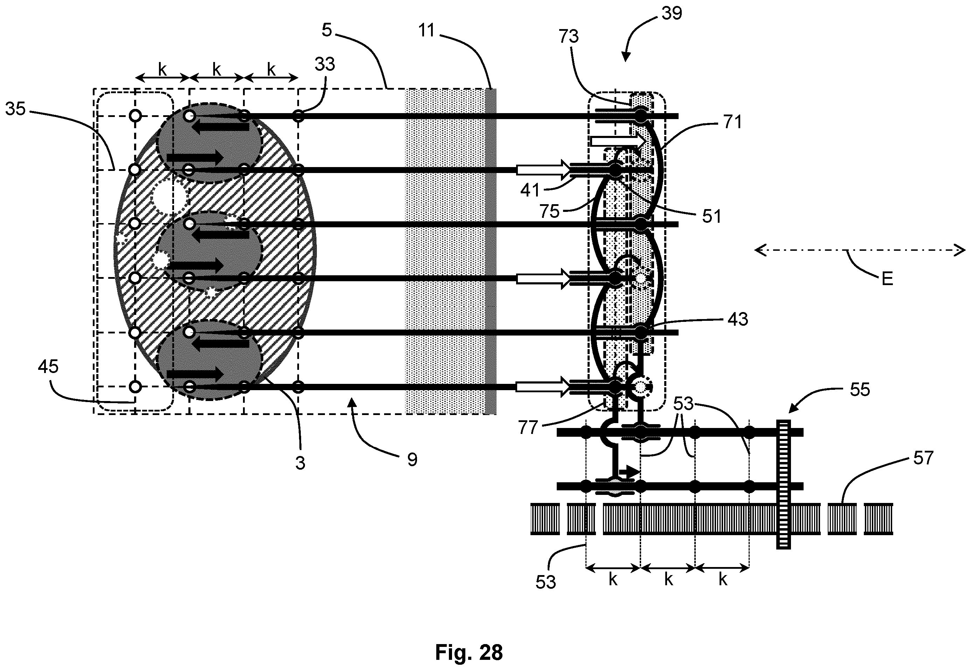

In Fig. 28 ist gezeigt, wie der zweite Schablonenteil 75 mit der zweiten Untergruppe 77 von Lichtapplikatoren 9 nachgezogen wird während der erste Schablonenteil 71 mit der ersten Untergruppe 73 von Lichtapplikatoren 9 festgehalten wird. Die Summe aller Haftreibungskräfte, welche von allen Lichtapplikatoren 9 gemeinsam auf das Organ 3 ausgeübt wird, ergibt auch hier wieder in erster Näherung Null was auch hier wieder durch die gepaarte Darstellung von gleichlangen, aber in entgegengesetzte Richtung zeigenden Kraftpfeilen innerhalb der gestrichelten, grauschattierten Ellipsen verdeutlicht ist, sodass das Organ 3 trotz der gleichzeitigen Bewegung einer Vielzahl von Lichtapplikatoren 9, nämlich derjenigen der zweiten Untergruppe 77, in eine gemeinsame Richtung, weder mitgezogen wird noch verformt wird.

Die vergleichsweise hohe Dichte der Führungen 41 und Fixierpunktaufnahmen 51 und daraus resultierend die entsprechend hohe potenzielle Dichte von Applikatoren 9 im Organ 3, sowie die Anordnung der Führungen 41 und Fixierpunktaufnahmen 51 unterschiedlicher Untergruppen 73 und 77 und entsprechend der Applikatoren 9 unterschiedlicher Untergruppen 73 und 77 zueinander, d.h. der regelmäßige Wechsel der Zugehörigkeit von Führungen 41 und Fixierpunktaufnahmen 51 zu den unterschiedlichen Untergruppen 73 und 77, führen dazu, dass auch lokale Verschiebungen und Verformungen innerhalb des Organs 3 weitgehend vermieden werden können.

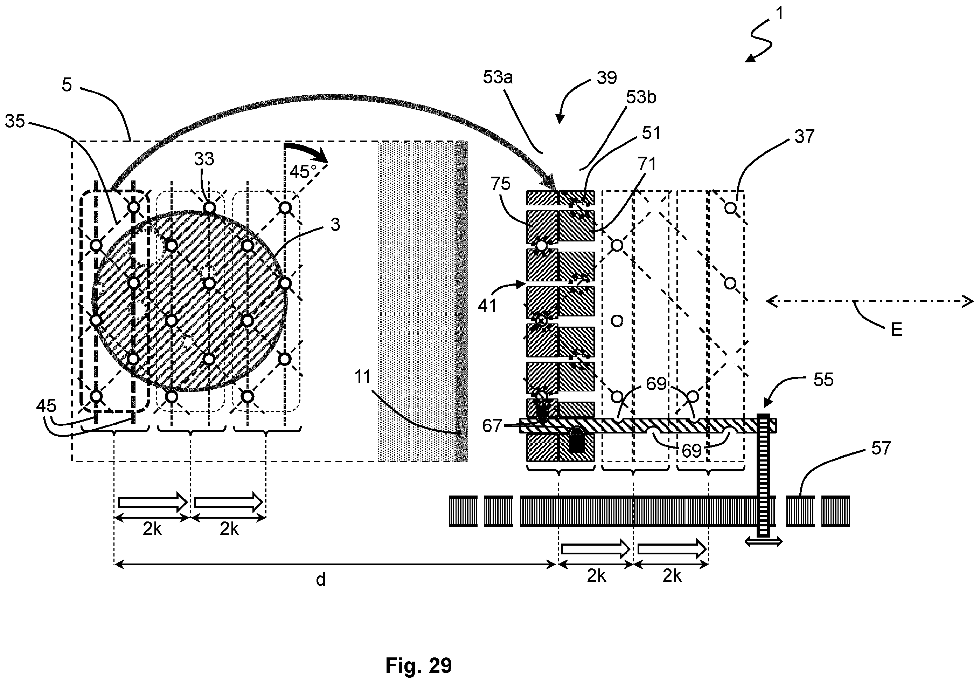

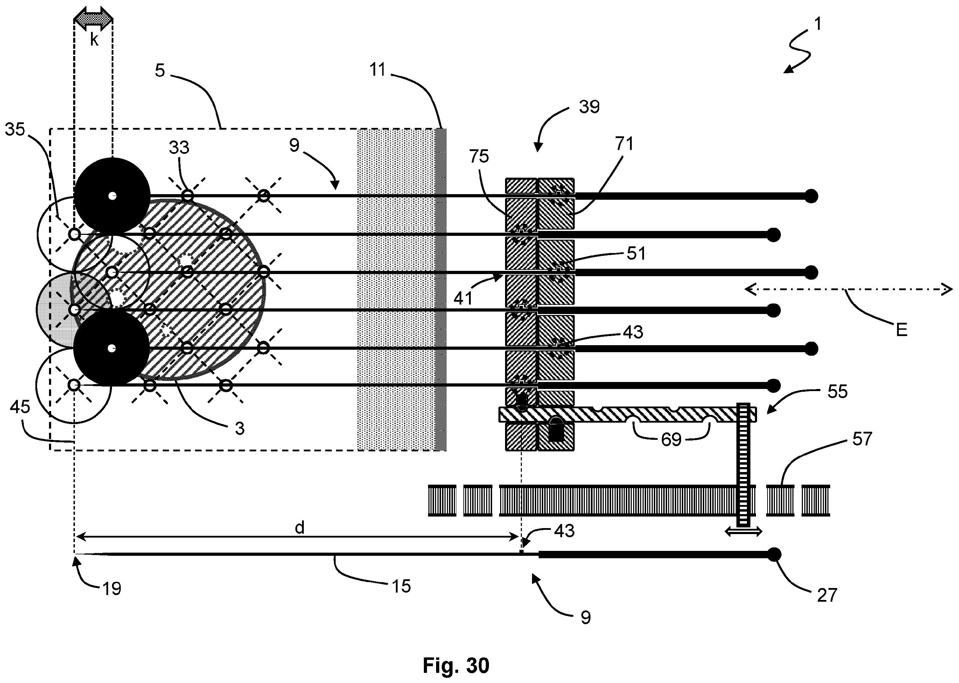

Fig. 29 bis 32 zeigen ein weiteres Ausführungsbeispiel zur Vermeidung des "Aufgabeleffekts", bei dem die PDT analog zum Ausführungsbeispiel gemäß Fig. 22 bis 24 in zwei Sollpunktgitterflächen 45 gleichzeitig durchgeführt wird. Wie bereits dort erläutert wurde, spielt diese Vorgehensweise dann eine wichtige Rolle, wenn das zu behandelnde Organ 3 größer und vor allen Dingen auch entlang der Einstichachse E ausgedehnter ist, weil damit innerhalb der gleichen Zeit bzw. mit der gleichen Anzahl an sequenziellen Bestrahlungseinheiten (im vorliegenden Fall drei sequenzielle Bestrahlungseinheiten) ein größeres Volumen behandelt werden kann (vergleiche dazu Fig. 29 mit Fig. 19). Die Platzierungsschablone 39 weist hier zwei parallele Platten 71, 75 auf, welche die Schablonenteile 71, 75 bilden und entlang der Einstichachse E geführt relativ zueinander beweglich sind. Der erste Schablonenteil 71 weist die Fixierpunktaufnahmen 51 von einer ersten Fixierpunktaufnahmegitterfläche 53b auf und der zweite Schablonenteil 75 weist die Fixierpunktaufnahmen 51 von einer zweiten Fixierpunktaufnahmegitterfläche 53a auf. In einer in Fig. 29 und 30 gezeigten ersten Relativposition, in der die Platten 71,75 aneinander liegen, unterscheidet sich die Platzierungsschablone 39 im Wesentlichen nicht von dem in Fig. 22 bis 24 gezeigten Ausführungsbeispiel und kann genauso verwendet werden, sofern kein "Aufgabeleffekt" zu befürchten ist.

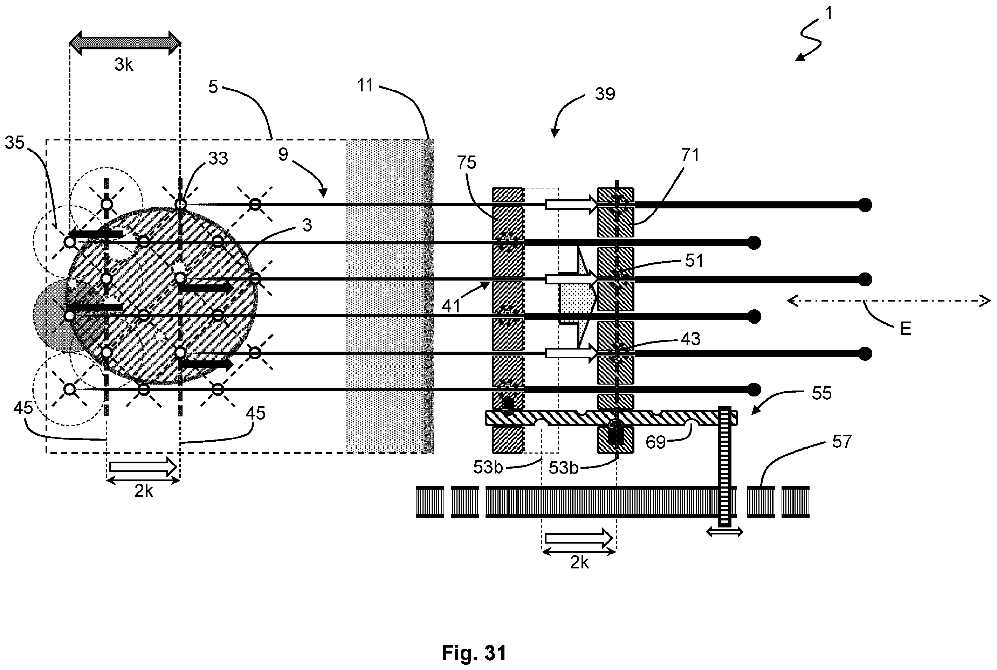

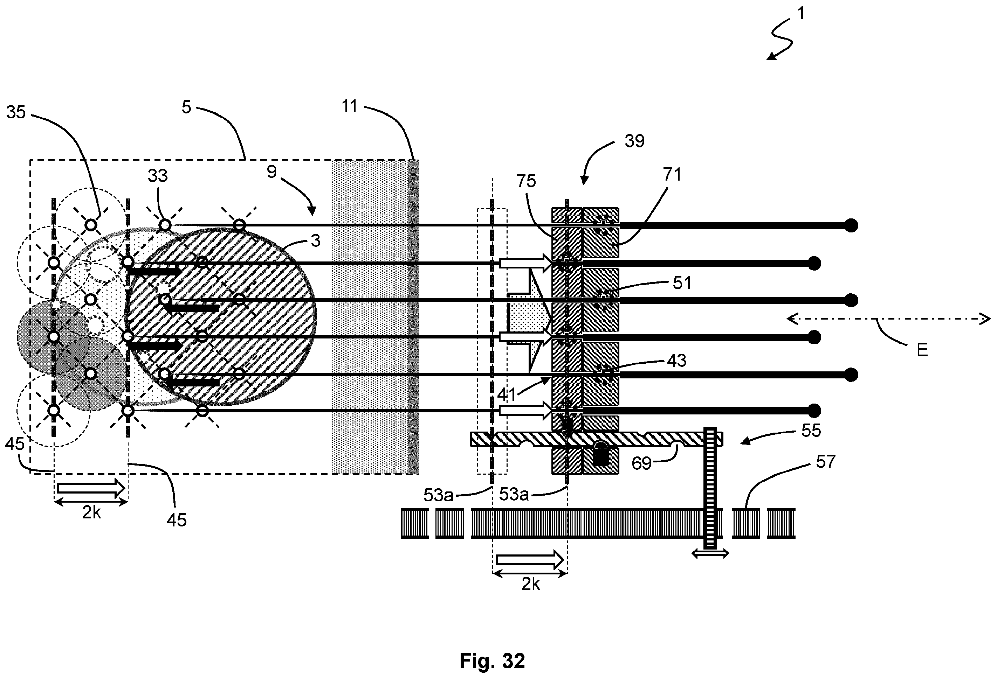

Ist jedoch ein "Aufgabeleffekt" zu befürchten, kann wie in Fig. 31 gezeigt zunächst der erste Schablonenteil 71 um 2k zurückgezogen werden, während der zweite Schablonenteil 75 festgehalten wird. Sobald der erste Schablonenteil 71 positioniert ist, kann wie in Fig. 32 gezeigt der zweite Schablonenteil 75 um 2k proximalwärts nachgezogen werden, während der erste Schablonenteil 71 festgehalten wird.

Allerdings kann es unter Umständen vorkommen, dass die Vermeidung des "Aufgabeleffektes" bei diesem Ausführungsbeispiel nur in eingeschränkter Weise funktioniert, was in Fig. 32 an der ungewollt veränderten Lage des Organs 3 deutlich wird und nachfolgend im Detail erläutert wird. Die Haftreibungskraft, die ein Lichtapplikator 9 auf das Organ 3 beim Herausziehen ausübt, steht in einem direkten Verhältnis zu der Lichtapplikatorschaft-Mantelfläche bzw. - bei gleichbleibendem Lichtapplikator-Durchmesser - zu der Lichtapplikatorschaft-Länge, die mit Gewebe des Organs 3 bedeckt ist. Dies führt dazu, dass der "Aufgabeleffekt" in bestimmten Situationen trotz der Aufteilung der Platzierungsschablone 39 in zwei Schablonenteile 71 und 75 und der sequenziellen Bewegung dieser Schablonenteile 71 und 75 nicht unterdrückt werden kann. Sehr deutlich wird dies am vorausgehend erläuterten Ausführungsbeispiel. Wenn, ausgehend von der Fig. 30, der erste Schablonenteil 71 um 2k in proximale Richtung bewegt wird, was schließlich in Fig. 31 abgeschlossen ist, dann ist die Untergruppe der Lichtapplikatoren 9, die im ersten Schablonenteil 71 fixiert ist und mit diesem bewegt wird, zunächst über eine ähnliche Schaftlänge mit Gewebe des Organs 3 bedeckt wie die Untergruppe der Lichtapplikatoren 9, die im zweiten Schablonenteil 75 fixiert ist und später mit jenem bewegt werden soll, zunächst aber entsprechend der vorausgehend beschriebenen Vorgehensweise an Ort und Stelle verbleibt. Der Längenunterschied in Bezug auf die Gewebebedeckung beträgt lediglich k, was in Fig. 30 durch den dicken, dunkelgrauen Doppelpfeil links oben in der Abbildung verdeutlicht wird. Weil außerdem noch alle Lichtapplikatoren 9 sehr weit im Organ 3 stecken, ist dieser Längenunterschied nur von untergeordneter Bedeutung. Folglich ist die Summe der bei der Bewegung des ersten Schablonenteils 71 durch seine Untergruppe der Lichtapplikatoren 9 auf das Organ 3 ausgeübten Haftreibungskräfte betragsmäßig ähnlich groß wie die Summe der in entgegengesetzte Richtung wirkenden Haftreibungskräfte der Untergruppe der unbewegten Lichtapplikatoren 9 des zweiten Schablonenteils 75.

Wenn allerdings dann, ausgehend von der Fig. 31 und beim Übergang zur Fig. 32, der zweite Schablonenteil 75 mit seiner Untergruppe der Lichtapplikatoren 9 nachgezogen wird, dann ist die Untergruppe der Lichtapplikatoren 9, die im ersten Schablonenteil 71 fixiert ist und entsprechend der vorausgehend beschriebenen Vorgehensweise nun an Ort und Stelle verbleibt, um eine Länge 3k weniger mit Gewebe des Organs 3 bedeckt als die Untergruppe der Lichtapplikatoren 9, die im zweiten Schablonenteil 75 fixiert ist und nun in proximale Richtung nachgezogen wird, was in Fig. 31 durch den dicken, dunkelgrauen Doppelpfeil links oben in der Abbildung verdeutlicht wird. Weil nun aber außerdem die Untergruppe der Lichtapplikatoren 9 des ersten Schablonenteils 71 nur noch über eine vergleichsweise geringe Schaftlänge im Organ 3 stecken, ist dieser ohnehin schon größere Längenunterschied 3k von noch höherer Bedeutung. Folglich übt die Untergruppe der in Bewegung gesetzten Lichtapplikatoren 9 des zweiten Schablonenteils 75 in der Summe eine deutlich höhere Haftreibungskraft auf das Organ 3 aus als die Untergruppe der ruhenden Lichtapplikatoren 9 des ersten Schablonenteils 71. Von einer Kompensation der Haftreibungskräfte kann dann, bei der Bewegung bzw. beim Nachziehen des zweiten Schablonenteils 75, nicht mehr die Rede sein. Die resultierende Gesamt-Haftreibungskraft unterscheidet sich also deutlich von Null, was dazu führt, dass das Organ 3 von der Untergruppe der Lichtapplikatoren 9 des zweiten Schablonenteils 75 in proximale Richtung mitgenommen wird, wie bereits schon in Fig. 25b schematisch dargestellt, und zu einer veränderten Lage im Körper 5 führt, wie in Fig. 32 dargestellt. Folglich deckt die Sollpunktgitterstruktur 35 nach dem Verschieben von Schablonenteilen das Organ 3 nicht mehr in der ursprünglich vorgesehenen Art und Weise ab, wie das zu Beginn noch der Fall war und beispielsweise in der Fig. 29 dargestellt ist. Dies führt dazu, dass die Behandlung nicht mehr in der ursprünglich geplanten einfachen, schnellen, definierten und - im Hinblick auf eine lückenlose Bestrahlung - sicheren Weise durchgeführt werden kann.

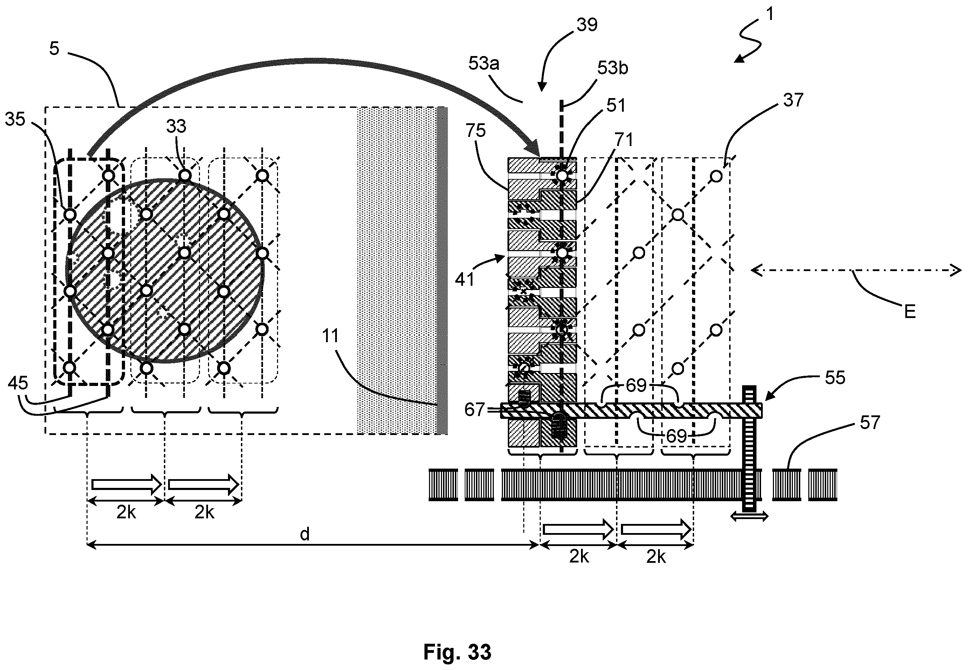

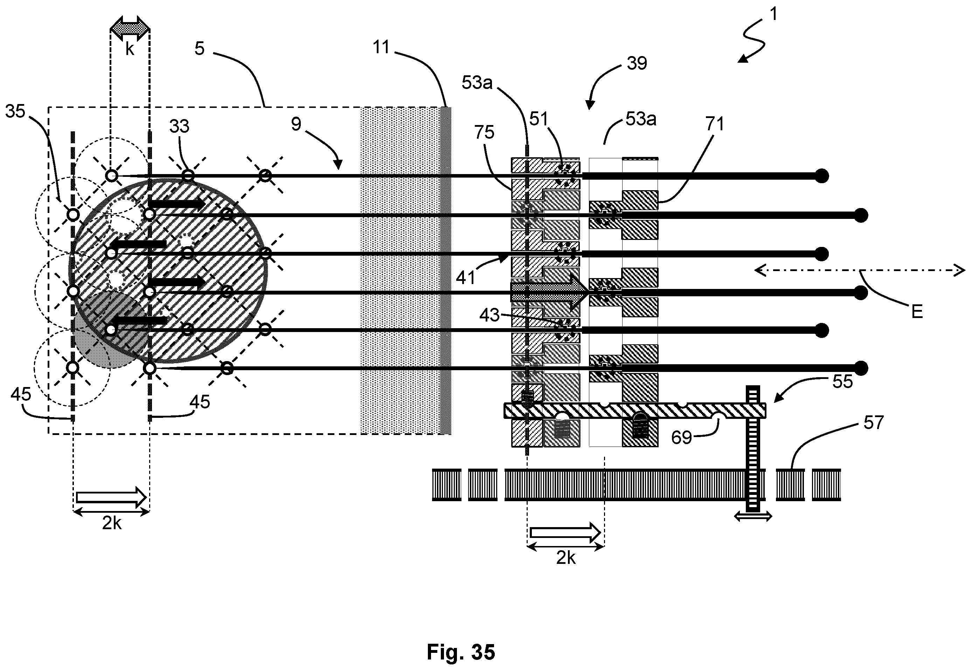

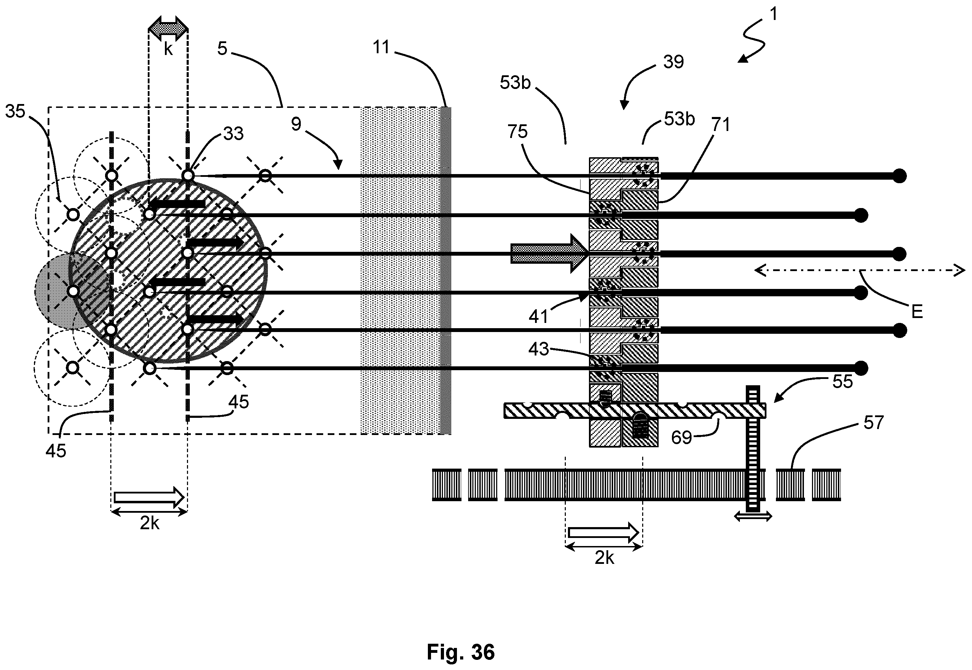

Eine Lösung des vorausgehend geschilderten Problems zeigt die am Ausführungsbeispiel der Fig. 33 bis 36 erläuterte Vorgehensweise. Kennzeichnend für diese Vorgehensweise ist, sofern der zweite Schablonenteil 75 in proximale Richtung relativ zum ersten Schablonenteil 71 nur bis zur Anlage an den ersten Schablonenteil 71 verschiebbar ist, dass bei Anlage des zweiten Schablonenteils 75 am ersten Schablonenteil 71 die vom ersten Schablonenteil 71 definierte erste Untergruppe 73 der Fixierpunktaufnahmen 51 distal von der vom zweiten Schablonenteil 75 definierten zweiten Untergruppe 77 der Fixierpunktaufnahmen 51 liegt. Mit anderen Worten befinden sich die Fixierpunktaufnahmen 51 des zweiten Schablonenteils 75 nicht mehr in der Fixierpunktaufnahmegitterfläche 53a des zweiten Schablonenteils 75, wie dies noch im vorausgehenden Ausführungsbeispiel der Fall war, siehe z.B. Fig. 29, sondern in der Fixierpunktaufnahmegitterfläche 53b des ersten Schablonenteils 71. Umgekehrt befinden sich die Fixierpunktaufnahmen 51 des ersten Schablonenteils 71 nun nicht mehr in der Fixierpunktaufnahmegitterfläche 53b des ersten Schablonenteils 71, sondern in der Fixierpunktaufnahmegitterfläche 53a des zweiten Schablonenteils 75, siehe Fig. 33.

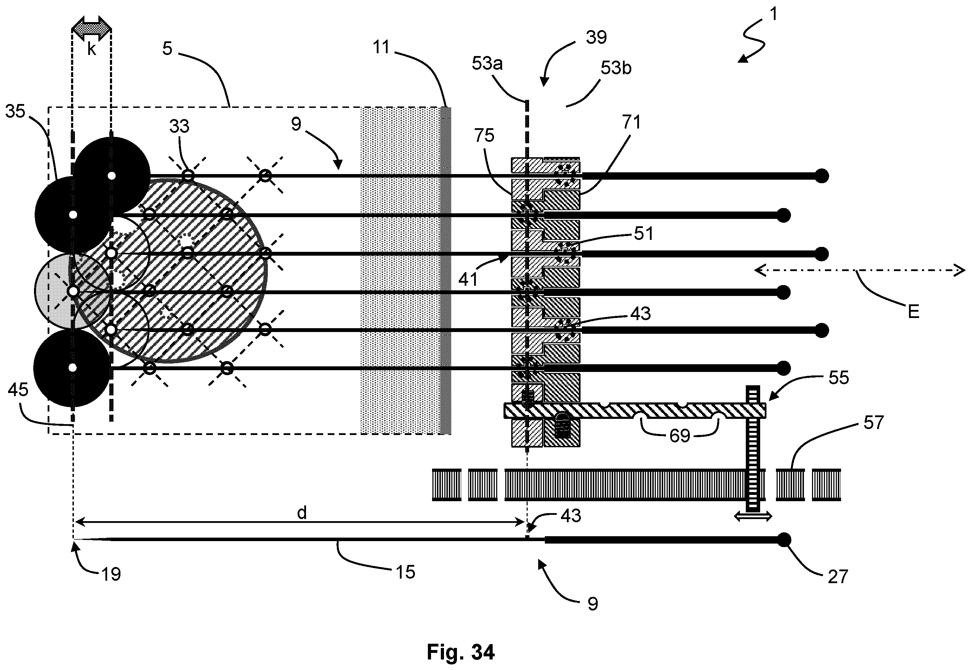

Dies hat zur Folge, dass, unabhängig davon, welche Untergruppe von Lichtapplikatoren 9 bewegt wird, der Unterschied der Schaftlänge, welche mit Gewebe des Organs 3 bedeckt ist, nie größer als die Länge k ist, siehe Fig. 34, 35 und 36, wohingegen im vorherigen Ausführungsbeispiel dieser Unterschied 3k betragen kann, also dreimal so groß sein kann, siehe Fig. 31. Dementsprechend kann der unerwünschte "Aufgabeleffekt" weitgehend vermieden werden und die Behandlung in der geplanten einfachen, schnellen, definierten und - im Hinblick auf eine lückenlose Bestrahlung - sicheren Weise durchgeführt werden.

### Bezugszeichenliste:

- 1: System
- 3: Organ
- 4: umgebendes, mit dem Organ 3 direkt verbundenes Gewebe
- 5: Körper
- 7: pathologischer Gewebebereich
- 9: Lichtapplikator
- 10: erster Lichtapplikator
- 11: Haut
- 13: distales Ende des Einführabschnitts
- 15: Einführabschnitt des Lichtapplikators
- 17: lichtemittierendes Element / LED
- 19: Applikatorspitze
- 21: Lichtkugel
- 23: Versorgungseinheit
- 25: Anschluss
- 27: Griffelement
- 29: Kabel
- 31: Stecker
- 33: Sollpunkt
- 35: Sollpunktgitterstruktur
- 37: Fixierpunktgitterstruktur
- 39: Platzierungsschablone
- 41: Führung
- 43: Fixierpunkt
- 44: Fixierpunkt
- 45: Sollpunktgitterfläche
- 47: Fixierpunktgitterfläche
- 51: Fixierpunktaufnahme
- 53: Fixierpunktaufnahmegitterfläche
- 53a: Fixierpunktaufnahmegitterfläche
- 53b: Fixierpunktaufnahmegitterfläche
- 55: Befestigungsvorrichtung
- 57: Referenzfläche
- 59: Eingriffselement
- 61: Eingriffsaufnahme
- 63: Vorsprung
- 65: Aufnahmesicherung
- 67: Eingriffselement
- 69: Eingriffsaufnahme
- 71: erster Schablonenteil
- 73: Untergruppe von Führungen bzw. Lichtapplikatoren
- 75: zweiter Schablonenteil
- 77: Untergruppe von Führungen bzw. Lichtapplikatoren

## Patentansprüche

1. System (1) zur Durchführung einer transkutanen photodynamischen Therapie, PDT, in einem Organ (3) oder Organsegment eines organischen Körpers (5),
wobei das System (1)
- eine Mehrzahl von individuell manuell platzierbaren Lichtapplikatoren (9),
- eine Versorgungseinheit (23) zum Versorgen der Lichtapplikatoren (9) mit Licht und/oder Strom, und
- eine in einer definierten Position zum organischen Körper (5) platzierbare Platzierungsschablone (39) zum definierten Ausrichten, Positionieren und Fixieren der einzelnen Lichtapplikatoren (9) aufweist,
wobei die Lichtapplikatoren (9) jeweils einen nadelartigen Einführabschnitt (15) zum transkutanen Einstechen entlang einer Einstichachse (E) in das Organ (3) oder Organsegment, am distalen Ende (13) des Einführabschnitts (15) eine lichtemittierende Applikatorspitze (19) und mindestens einen definierten Fixierpunkt (43) in einem proximalen Abstand d von der Applikatorspitze (19) aufweisen, wobei die Platzierungsschablone (39) eine Mehrzahl an Fixierpunktaufnahmen (51) definiert, mittels welcher die Lichtapplikatoren (9) mit ihrem mindestens einen definierten Fixierpunkt (43) fixierbar sind, wobei die Fixierpunktaufnahmen (51) entsprechend einer dreidimensionalen Fixierpunktgitterstruktur (37) angeordnet sind, wobei die Fixierpunktgitterstruktur (37) einer virtuellen organspezifischen Sollpunktgitterstruktur (35) für die lichtemittierenden Applikatorspitzen (19) im Organ (3) oder Organsegment entspricht und um den Abstand d entlang der Einstichachse (E) parallelverschoben zur Sollpunktgitterstruktur (35) ist.

2. System (1) nach Anspruch 1, wobei die Versorgungseinheit (23) dazu eingerichtet ist, die Lichtapplikatoren (9) mit Strom zu versorgen und jeder Lichtapplikator (9) am distalen Ende (13) des Einführabschnitts (15) eine mit dem Strom betreibbare LED (17) aufweist.

3. System (1) nach einem der vorhergehenden Ansprüche, wobei der mindestens eine Fixierpunkt (43) durch einen Anschlag ausgebildet ist und entsprechend der dreidimensionalen Fixierpunktgitterstruktur (37) an der Platzierungsschablone (39) arretierbar ist.

4. System (1) nach einem der vorhergehenden Ansprüche, wobei die virtuelle organspezifische Sollpunktgitterstruktur (35) eine Mehrzahl von räumlich auf Sollpunktgitterflächen (45) verteilt angeordneten Sollpunkten (33) aufweist, wobei die Sollpunktgitterflächen (45) entlang der Einstichachse (E) einen Abstand (k) zueinander haben.

5. System (1) nach Anspruch 4, wobei die Lichtapplikatoren (9) jeweils mindestens zwei Fixierpunkte (43) aufweisen, welche zueinander entlang der Einstichachse (E) den Abstand (k) haben.

6. System (1) nach einem der vorhergehenden Ansprüche, wobei die Platzierungsschablone (39) eine Mehrzahl von räumlich auf Fixierpunktaufnahmegitterflächen (53) verteilt angeordneten Fixierpunktaufnahmen (51) aufweist, wobei die Fixierpunktaufnahmegitterflächen (53) entlang der Einstichachse (E) einen Abstand (k) zueinander haben.

7. System (1) nach einem der vorhergehenden Ansprüche, wobei die Platzierungsschablone (39) mindestens einen ersten Schablonenteil (71) aufweist, der eine erste Untergruppe (73) der Fixierpunktaufnahmen (51) definiert, und mindestens einen zweiten Schablonenteil (75) aufweist, der eine zweite Untergruppe (77) der Fixierpunktaufnahmen (51) definiert,
wobei der erste Schablonenteil (71) relativ zum zweiten Schablonenteil (75) entlang der Einstichachse (E) geführt verschiebbar ist.

8. System (1) nach Anspruch 7, wobei die erste Untergruppe (73) der Fixierpunktaufnahmen (51) ein erstes Fixierpunktaufnahmeteilgitter und die zweite Untergruppe (77) der Fixierpunktaufnahmen (51) ein zweites Fixierpunktaufnahmeteilgitter definiert, wobei das zweite Fixierpunktaufnahmeteilgitter quer zur Einstichachse (E) um einen lateralen Gitterversatz versetzt zum ersten Fixierpunktaufnahmeteilgitter ist.

9. System (1) nach Anspruch 8, wobei der laterale Gitterversatz kleiner ist als eine Seitenlänge einer Gitterelementarzelle des ersten Fixierpunktaufnahmeteilgitters und/oder des zweiten Fixierpunktaufnahmeteilgitters.

10. System (1) nach einem der Ansprüche 7 bis 9, wobei die erste Untergruppe (73) der Fixierpunktaufnahmen (51), die zweite Untergruppe (77) der Fixierpunktaufnahmen (51) sowie die Lichtapplikatoren (9) zusammen eine dreidimensionale Fixierpunktgitterstruktur (37) definieren, wobei die Fixierpunktgitterstruktur (37) einer virtuellen organspezifischen Sollpunktgitterstruktur (35) für die lichtemittierenden Applikatorspitzen (19) im Organ (3) oder Organsegment entspricht und um einen Abstand d entlang der Einstichachse (E) parallelverschoben zur Sollpunktgitterstruktur (35) ist, wobei jeder Lichtapplikator (9) mindestens einen definierten Fixierpunkt (43) im Abstand d von der lichtemittierenden Applikatorspitze (19) aufweist.

11. System (1) nach Anspruch 10, wobei die virtuelle organspezifische Sollpunktgitterstruktur (35) eine Mehrzahl von räumlich auf Sollpunktgitterflächen (45) verteilt angeordneten Sollpunkten (33) aufweist, wobei die Sollpunktgitterflächen (45) entlang der Einstichachse (E) einen Abstand k zueinander haben.

12. System (1) nach Anspruch 10 oder 11, wobei eine mit der ersten Untergruppe (73) der Fixierpunktaufnahmen (51) korrespondierende erste Untergruppe der Sollpunkte (33) ein erstes Sollpunktteilgitter und eine mit der zweiten Untergruppe (77) der Fixierpunktaufnahmen (51) korrespondierende zweite Untergruppe der Sollpunkte (33) ein zweites Sollpunktteilgitter definiert, sodass sich das erste Sollpunktteilgitter und das zweites Sollpunktteilgitter zu einer der Sollpunktgitterflächen (45) ergänzen.

13. System (1) nach einem der Ansprüche 7 bis 12, wobei der zweite Schablonenteil (75) in proximale Richtung relativ zum ersten Schablonenteil (71) nur bis zur Anlage an den ersten Schablonenteil (71) verschiebbar ist, wobei bei Anlage des zweiten Schablonenteils (75) am ersten Schablonenteil (71) die vom ersten Schablonenteil (71) definierte erste Untergruppe (73) der Fixierpunktaufnahmen (51) distal von der vom zweiten Schablonenteil (75) definierten zweiten Untergruppe (77) der Fixierpunktaufnahmen (51) liegt.

14. System (1) nach einem der Ansprüche 7 bis 13, wobei die Fixierpunktaufnahmen (51) auf Fixierpunktaufnahmegitterflächen (53) verteilt angeordnet sind, wobei die Fixierpunktaufnahmegitterflächen (53) entlang der Einstichachse (E) einen Abstand k zueinander haben.

15. System (1) nach Anspruch 14, wobei der erste Schablonenteil (71) eine erste Untergruppe der Fixierpunktaufnahmen (51) einer ersten Fixierpunktaufnahmegitterfläche (53a) und der zweite Schablonenteil (75) eine zweite Untergruppe der Fixierpunktaufnahmen (51) einer zweiten Fixierpunktaufnahmegitterfläche (53b) aufweist.

## Claims

1. A system (1) for carrying out a transcutaneous photodynamic therapy, PDT,
in an organ (3) or organ segment of an organic body (5), wherein the system (1) comprises
- a plurality of individually manually placeable light applicators (9),
- a supply unit (23) for supplying the light applicators (9) with light and/or power, and
- a placement template (39), placeable in a defined position relative to the organic body (5), for defined alignment, positioning and fixing of the individual light applicators (9),
wherein the light applicators (9) each have a needle-like insertion portion (15) for transcutaneous piercing along a piercing axis (E) into the organ (3) or organ segment, at the distal end (13) of the insertion portion (15) a light-emitting applicator tip (19) and at least one defined fixing point (43) at a proximal distance d from the applicator tip (19), wherein the placement template (39) defines a plurality of fixing point receptacles (51), by means of which the light applicators (9) are fixable with their at least one defined fixing point (43), wherein the fixing point receptacles (51) are arranged in accordance with a three-dimensional fixing point grid structure (37), wherein the fixing point grid structure (37) corresponds to a virtual organ-specific target point grid structure (35) for the light-emitting applicator tips (19) in the organ (3) or organ segment and is parallel-displaced relative to the target point grid structure (35) by the distance d along the piercing axis (E).

2. The system (1) according to claim 1, wherein the supply unit (23) is set up to supply power to the light applicators (9) and each light applicator (9) has an LED (17) at the distal end (13) of the insertion portion (15), said LEDs being operable with the power.

3. The system (1) according to one of the preceding claims, wherein the at least one fixing point (43) is formed by a stop and is lockable to the placement template (39) in accordance with the three-dimensional fixing point grid structure (37).

4. The system (1) according to one of the preceding claims, wherein the virtual organ-specific target point grid structure (35) has a plurality of target points (33) distributed spatially on target point grid areas (45), wherein the target point grid areas (45) have a distance (k) from one another along the piercing axis (E).

5. The system (1) according to claim 4, wherein the light applicators (9) each have at least two fixing points (43), which have the distance (k) from one another along the piercing axis (E).

6. The system (1) according to one of the preceding claims, wherein the placement template (39) has a plurality of fixing point receptacles (51) distributed spatially on fixing point receptacle grid areas (53), wherein the fixing point receptacle grid areas (53) have a distance (k) from one another along the piercing axis (E).

7. The system (1) according to one of the preceding claims, wherein the placement template (39) has at least one first template part (71), which defines a first sub-group (73) of the fixing point receptacles (51), and at least one second template part (75), which defines a second sub-group (77) of the fixing point receptacles (51), wherein the first template part (71) is guided displaceably relative to the second template part (75) along the piercing axis (E).

8. The system (1) according to claim 7, wherein the first sub-group (73) of the fixing point receptacles (51) defines a first fixing point receptacle sub-grid and the second sub-group (77) of the fixing point receptacles (51) defines a second fixing point receptacle sub-grid, wherein the second fixing point receptacle sub-grid, transversely to the piercing axis (E), is offset from the first fixing point receptacle sub-grid by a lateral grid offset.

9. The system (1) according to claim 8, wherein the lateral grid offset is smaller than a side length of a grid elementary cell of the first fixing point receptacle sub-grid and/or of the second fixing point receptacle sub-grid.

10. The system (1) according to one of claims 7 to 9, wherein the first sub-group (73) of fixing point receptacles (51), the second sub-group (77) of fixing point receptacles (51) and the light applicators (9) together define a three-dimensional fixing point grid structure (37), wherein the fixing point grid structure (37) corresponds to a virtual organ-specific target point grid structure (35) for the light-emitting applicator tips (19) in the organ (3) or organ segment and is parallel-displaced relative to the target point grid structure (35) by a distance d along the piercing axis (E), wherein each light applicator (9) has at least one defined fixing point (43) at the distance d from the light-emitting applicator tip (19).

11. The system (1) according to claim 10, wherein the virtual organ-specific target point grid structure (35) has a plurality of target points (33) distributed spatially on target point grid areas (45), wherein the target point grid areas (45) have a distance k from one another along the piercing axis (E).

12. The system (1) according to claim 10 or 11, wherein a first sub-group of the target points (33) corresponding to the first sub-group (73) of the fixing point receptacles (51) defines a first target point sub-grid, and a second sub-group of the target points (33) corresponding to the second sub-group (77) of the fixing point receptacles (51) defines a second target point sub-grid, so that the first target point sub-grid and the second target point sub-grid supplement one another to form the target point grid areas (45).

13. The system (1) according to one of claims 7 to 12, wherein the second template part (75) is displaceable in the proximal direction relative to the first template part (71) only until in contact with the first template part (71), wherein, when the second template part (75) is in contact with the first template part (71), the first sub-group (73) of the fixing point receptacles (51) defined by the first template part (71) lies distally of the second sub-group (77) of fixing point receptacles (51) defined by the second template part (75).

14. The system (1) according to one of claims 7 to 13, wherein the fixing point receptacles (51) are distributed on fixing point receptacle grid areas (53), wherein the fixing point receptacle grid areas (53) have a distance k from one another along the piercing axis (E).

15. The system (1) according to claim 14, wherein the first template part (71) has a first sub-group of fixing point receptacles (51) of a first fixing point receptacle grid area (53a) and the second template part (75) has a second sub-group of fixing point receptacles (51) of a second fixing point receptacle grid area (53b)

## Revendications

1. Système (1) pour la réalisation d'un traitement photodynamique transcutané, PDT,
dans un organe (3) ou un segment d'organe d'un corps organique (5),
dans lequel le système (1) présente
- une pluralité d'applicateurs de lumière (9) pouvant être placés manuellement et individuellement,
- une unité d'alimentation (23) pour alimenter les applicateurs de lumière (9) en lumière et/ou en courant électrique, et
- un pochoir de placement (39) pouvant être placé dans une position définie par rapport au corps organique (5) pour l'alignement, le positionnement et la fixation définis des différents applicateurs de lumière (9),
dans lequel les applicateurs de lumière (9) présentent respectivement un tronçon d'introduction (15) en forme d'aiguille pour piquer en transcutané dans l'organe (3) ou le segment d'organe le long d'un axe de piqûre (E), une pointe d'applicateur (19) émettrice de lumière sur l'extrémité (13) distale du tronçon d'introduction (15) et au moins un point de fixation (43) défini dans un écart proximal d par rapport à la pointe d'applicateur (19), dans lequel le pochoir de placement (39) définit une pluralité de réceptions de points de fixation (51) au moyen desquelles les applicateurs de lumière (9) peuvent être fixés par leur au moins un point de fixation défini (43), dans lequel les réceptions de points de fixation (51) sont disposées conformément à une structure de grille de points de fixation (37) tridimensionnelle, dans lequel la structure de grille de points de fixation (37) correspond à une structure de grille de points nominaux (35) virtuelle spécifique à l'organe pour les pointes d'applicateur (19) émettrices de lumière dans l'organe (3) ou le segment d'organe et est décalée en parallèle par rapport à la structure de grille de points nominaux (35), de l'écart d le long de l'axe de piqûre (E).

2. Système (1) selon la revendication 1, dans lequel l'unité d'alimentation (23) est conçue pour alimenter les applicateurs de lumière (9) en courant électrique et chaque applicateur de lumière (9) présente une LED (17) pouvant être alimentée avec le courant électrique à l'extrémité distale (13) du tronçon d'introduction (15).

3. Système (1) selon l'une des revendications précédentes, dans lequel l'au moins un point de fixation (43) est formé par une butée et peut être arrêté contre le pochoir de positionnement (39) conformément à la structure de grille de points de fixation (37) tridimensionnelle.

4. Système (1) selon l'une des revendications précédentes, dans lequel la structure de grille de points nominaux (35) virtuelle spécifique à l'organe présente une pluralité de points nominaux (33) disposés en étant répartis spatialement sur des surfaces de grille de points nominaux (45), dans lequel les surfaces de grille de points nominaux (45) ont un écart (k) entre elles le long de l'axe de piqûre (E).

5. Système (1) selon la revendication 4, dans lequel les applicateurs de lumière (9) présentent respectivement au moins deux points de fixation (43), lesquels ont l'écart (k) entre eux le long de l'axe de piqûre (E).

6. Système (1) selon l'une des revendications précédentes, dans lequel le pochoir de placement (39) présente une pluralité de réceptions de points de fixation (51) disposées en étant réparties spatialement sur des surfaces de grille de réception de points de fixation (53), dans lequel les surfaces de grille de réception de points de fixation (53) ont un écart (k) entre elles le long de l'axe de piqûre (E).

7. Système (1) selon l'une des revendications précédentes, dans lequel le pochoir de placement (39) présente au moins une première partie de pochoir (71) qui définit un premier sous-groupe (73) des réceptions de points de fixation (51), et au moins une seconde partie de pochoir (75) qui définit un second sous-groupe (77) des réceptions de points de fixation (51),
dans lequel la première partie de pochoir (71) est mobile en étant dirigée le long de l'axe de piqûre (E) par rapport à la seconde partie de pochoir (75).

8. Système (1) selon la revendication 7, dans lequel le premier sous-groupe (73) des réceptions de points de fixation (51) définit une première grille partielle de réception de points de fixation et le second sous-groupe (77) des réceptions de points de fixation (51) définit une seconde grille partielle de réception de points de fixation, dans lequel la seconde grille partielle de réception de points de fixation est décalée d'un décalage de grille latéral transversalement à l'axe de piqûre (E) par rapport à la première grille partielle de réception de points de fixation.

9. Système (1) selon la revendication 8, dans lequel le décalage de grille latéral est plus petit qu'une longueur latérale d'une cellule élémentaire de grille de la première grille partielle de réception de points de fixation et/ou de la seconde grille partielle de réception de points de fixation.

10. Système (1) selon l'une des revendications 7 à 9, dans lequel le premier sous-groupe (73) des réceptions de points de fixation (51), le second sous-groupe (77) des réceptions de points de fixation (51) ainsi que les applicateurs de lumière (9) définissent ensemble une structure de grille de points de fixation (37) tridimensionnelle, dans lequel la structure de grille de points de fixation (37) correspond à une structure de grille de points nominaux (35) virtuelle spécifique à l'organe pour les pointes d'applicateur (19) émettrices de lumière dans l'organe (3) ou le segment d'organe et est décalée en parallèle par rapport à la structure de grille de points nominaux (35) d'un écart d le long de l'axe de piqûre (E), dans lequel chaque applicateur de lumière (9) présente au moins un point de fixation (43) défini dans l'écart d par rapport à la pointe d'applicateur (19) émettrice de lumière.

11. Système (1) selon la revendication 10, dans lequel la structure de grille de points nominaux (35) virtuelle spécifique à l'organe présente une pluralité de points nominaux (33) disposés en étant répartis spatialement sur des surfaces de grille de points nominaux (45), dans lequel les surfaces de grille de points nominaux (45) ont un écart k entre elles le long de l'axe de piqûre (E).

12. Système (1) selon la revendication 10 ou 11, dans lequel un premier sous-groupe des points nominaux (33) correspondant au premier sous-groupe (73) des réceptions de points de fixation (51) et un second sous-groupe des points nominaux (33) correspondant au second sous-groupe (77) des réceptions de points de fixation (51) définit une seconde grille partielle de points nominaux, de sorte que la première grille partielle de points nominaux et la seconde grille partielle de points nominaux se complètent en une des surfaces de grille de points nominaux (45).

13. Système (1) selon une des revendications 7 à 12, dans lequel la seconde partie de pochoir (75) peut être décalée dans le sens proximal par rapport à la première partie de pochoir (71) uniquement jusqu'à l'appui contre la première partie de pochoir (71), dans lequel lors de l'appui de la seconde partie de pochoir (75) contre la première partie de pochoir (71), le premier sous-groupe (73) des réceptions de points de fixation (51) défini par la première partie de pochoir (71) repose de manière distale par rapport au second sous-groupe (77) des réceptions de points de fixation (51) défini par la seconde partie de pochoir (75).

14. Système (1) selon une des revendications 7 à 13, dans lequel les réceptions de points de fixation (51) sont disposées en étant réparties sur des surfaces de grille de réception de points de fixation (53), dans lequel les surfaces de grille de réception de points de fixation (53) ont un écart k entre elles le long de l'axe de piqûre (E).

15. Système (1) selon la revendication 14, dans lequel la première partie de pochoir (71) représente un premier sous-groupe des réceptions de points de fixation (51) d'une première surface de grille de réception de points de fixation (53a) et la seconde partie de pochoir (75) présente un second sous-groupe des réceptions de points de fixation (51) d'une seconde surface de grille de réception de points de fixation (53b).
